# EUROPEAN PATENT SPECIFICATION

(11) **EP 3 440 461 B1**
(45) Date of publication and mention of the grant of the patent: **18.11.2026**
(21) Application number: 17778798.3
(22) Date of filing: 06.04.2017
(51) Int. Cl.: G01N 33/50, G01N 33/53, G01N 33/564, C12Q 1/68

(54) **INFILTRATING IMMUNE CELL PROPORTIONS PREDICT ANTI-TNF RESPONSE IN COLON BIOPSIES**
INFILTRIERENDE IMMUNZELLANTEILE ZUR VORHERSAGE EINER ANTI-TNF-ANTWORT IN DICKDARMBIOPSIEN
PRÉDICTION DE LA RÉPONSE ANTI-TNF DANS DES BIOPSIES DE CÔLON AVEC LES PROPORTIONS DE CELLULES IMMUNITAIRES INFILTRANTES

(30) Priority: 06.04.2016 US 201662318971 P
(43) Date of publication of application: 13.02.2019
(73) Proprietor: Technion Research & Development Foundation Limited, 3200004 Haifa (IL); The Board of Trustees of the Leland Stanford Junior University, Stanford, CA 94305-2038 (US)
(72) Inventor: SHEN-ORR, Shai S., 3706254 Karkur (IL); STAROVETSKY, Elina, 2069363 Yokneam Ilit (IL); MAIMON, Naama, 3080800 Doar-Na Hof HaCarmel (IL); KHATRI, Purvesh, Menlo Park, California 94025 (US); GAUJOUX, Renaud, 7925 Cape Town (ZA); VALLANIA, Francesco, San Francisco, California 94107 (US)
(74) Representative: Dennemeyer & Associates S.A.
(86) International application number: PCT/IL2017/050419
(87) International publication number: WO 2017/175228

(56) References cited:
- WO-A2-2013/080050
- BIOLEGEND: "Purified anti-human CD138 (Syndecan-1) Antibody", 6 September 2014 (2014-09-06), pages 1 - 2, XP055626104, Retrieved from the Internet <URL:https://www.biolegend.com/en-us/global-elements/pdf-popup/purified-anti-human-cd138-syndecan-1-antibody-7312?filename=Purified%20anti-human%20CD138%20Syndecan-1%20Antibody.pdf&pdfgen=true> [retrieved on 20190925]
- FRANCO SCALDAFERRI: "Mucosal biomarkers in inflammatory bowel disease: Key pathogenic players or disease predictors?", WORLD JOURNAL OF GASTROENTEROLOGY, vol. 16, no. 21, 1 January 2010 (2010-01-01), pages 2616, XP055090265, ISSN: 1007-9327, DOI: 10.3748/wjg.v16.i21.2616
- K LEIPER ET AL: "Rituximab in active ulcerative colitis", 1 January 2012 (2012-01-01), pages 174 - 176, XP055626054, Retrieved from the Internet <URL:https://www.gastrojournal.org/article/S0016-5085(11)01570-8/pdf> [retrieved on 20190925]
- LENKA FROLOVA ET AL: "Expression of Toll-like Receptor 2 (TLR2), TLR4, and CD14 in Biopsy Samples of Patients With Inflammatory Bowel Diseases: Upregulated Expression of TLR2 in Terminal Ileum of Patients With Ulcerative Colitis", JOURNAL OF HISTOCHEMISTRY AND CYTOCHEMISTRY, vol. 56, no. 3, 12 November 2007 (2007-11-12), US, pages 267 - 274, XP055625654, ISSN: 0022-1554, DOI: 10.1369/jhc.7A7303.2007
- RENAUD GAUJOUX ET AL: "Conclusions", GUT, vol. 68, no. 4, 4 April 2018 (2018-04-04), UK, pages 604 - 614, XP055625670, ISSN: 0017-5749, DOI: 10.1136/gutjnl-2017-315494
- MAIMON NAAMA: "High-Dimensional Characterization of Inflammatory Bowel Disease Tissue and its Reflection in the Periphery Yields New approaches for Disease Management", 1 August 2020, Haifa, XP093239856
- WLODARCZYK, MARCIN K. ET AL.: "Neutrophil-lymphocyte ratio in Crohn's disease patients predicts sustained response to infliximab 52-week therapy", JOURNAL OF GASTROINTESTINAL AND LIVER DISEASES: JGLD, vol. 24, no. 1, 31 March 2015 (2015-03-31), pages 127 - 128, XP009514048, ISSN: 1842-1121
- BULLENS, D. ET AL.: "P917 Circulating b cell subsets correlate with clinical response to anti-TNF therapy in IBD", UEG WEEK 2013 POSTER PRESENTATIONS UNITED EUROPEAN GASTROENTEROLOGY JOURNAL, vol. 1, no. 1, 14 October 2013 (2013-10-14), pages A378 - A379, XP055428538
- ARIJS, INGRID ET AL.: "Predictive value of epithelial gene expression profiles for response to infliximab in Crohn's disease", INFLAMMATORY BOWEL DISEASES, vol. 16, no. 12, 28 June 2010 (2010-06-28), pages 2090 - 2098, XP055428545

## Description

The present disclosure, in some aspects thereof, relates to methods for predicting responsiveness of a subject having an inflammatory bowel disease (IBD) to treatment with a tumor necrosis factor (TNF)-alpha inhibitor, more particularly, but not exclusively, to methods of selecting a treatment for a subject diagnosed with the inflammatory bowel disease (IBD) and also to a method of selecting treatment to inflammatory bowel disease (IBD) in a subject in need thereof,.
Inflammatory Bowel Diseases (IBDs) comprises primarily from ulcerative colitis (UC) and Crohn's disease (CD) disease conditions, for which treatment with anti-TNF monoclonal antibodies such as Infliximab have shown an ability to treat inflammation and achieve mucosal healing *(1, 2).* However, response to such treatments is very heterogeneous, with overall only 60% of the patients showing clear primary phenotypic improvement *(3).* The remainder of patients do not respond at all, or lose response after a short period. Because of the high cost of the anti-TNF biologics combined with their systemic side effects and the uncertainty of response, these drugs are generally not used as a first line treatment. If anti-TNF is eventually prescribed, the patient's endoscopic and histologic state is monitored over 8 and 14 weeks to assess response. During this "trial" period, side effects such as infections, anaphylaxis-like reactions, induction of auto-antibodies, skin eruptions and injection site reactions have been reported or are common (1-5), which, for non-responder patients, adds up to the burden of their unresolved IBD condition.

Predictive gene signatures for response in IBD have previously been proposed based on microarray gene expression experiments. Two studies identified sets of genes that discriminate responders from non-responders in UC and CD colon biopsies respectively *(4, 5).* A core set of 5 genes (TNFRSF11B, STC1, PTGS2, IL13RA2 and IL11) defined from the UC cohort data could perfectly classify the independent CD samples, supporting a common mechanism of (non)-response to treatment in both conditions. These genes encode for proteins involved in signaling in the adaptive immune response, pathogenesis of inflammation and TNF pathways *(6-8).* Moreover, PTGS2, STC1 and IL13RA2, are also implicated in intestinal homeostasis and pathology *(9-12).* Yet, their forming role in the molecular mechanisms of infliximab is not well understood. Biomarkers from blood gene expression *(13),* and genetic susceptibility loci for disease or non-response to anti-TNF have also been proposed *(14, 15),* and, very recently, association with microbiome composition has been investigated *(16).* However, research has not yet translated into a clinical test that can predict response to anti-TNF prior to onset of treatment. Hence, finding a robust, clinically feasible predictive assay of response is of high value as it would provide a personalized patient care, and improve the benefit-cost ratio of anti-TNF therapies by enabling the early-on treatment of predicted responders while limiting the risk of failure to response.

Inflammation in IBD is driven by an exacerbated immune response, where infiltrating immune cells in colon tissue are key actors of the disease's etiology and progression, notably through the interface with intestinal commensal microbes *(17).* For example, the presence of macrophage-formed granulomas is a common flag for CD diagnostic, and plasmacytic or neutrophil infiltrates are common clinical indicators of tissue inflammation. Moreover, biological function analysis of gene-level differences associated with response displayed a clear enrichment in immune-related functional categories *(4, 5).* However, the link between response to anti-TNF response and the characteristics of the endothelial immune compartment has not yet been investigated.

US 20110059445 A1 to Paul Rutgeerts and Frans Schuit (Mucosal gene signatures) discloses *in vitro* methods of determining if a subject suffering from an inflammatory condition of the large intestine and/or small intestine will respond to anti-TNFα therapy, using the IL-13R(alpha)2 (in UC patients) and the IL-13R(alpha)2, TNFRSF11B, STC1, PTGS2 and IL-11 (in IBD patients).

US 20110045490 A1 to Zoltán Konthur, et al. discloses biomarkers such as RAB11B, PPP2R1A, KPNB1, COG4, FDFT1, PECI, CTNND2, NSMCE1, KTELC1, HS6ST1, ARMC6, TH1L, PSME1, GPC1, EDC4, PRC1, NAT6, EEF1AL3, NP_ 612480.1, PLXNA2, ELMO2 and NDUFS2 for the prediction of responsiveness to an anti-tumour necrosis factor alpha (tnf) treatment.

U.S. 20100069256 to Frederic Baribaud et al. discloses a method of predicting the suitability of treatment with a target therapy for a gastrointestinal-related disorder with anti-TNFα antibody by assaying nucleic acids from a specimen obtained from the subject.

WO 2013080050 is directed to methods and compositions useful for predicting the efficacy of a TNFa inhibitor for treating an inflammatory bowel disease (IBD). It determines the level of expression of TNFa by delivering a labeled anti-TNFa antibody on to the cells of the intestinal mucosa of a subject having IBD, whereby the TNFa level of expression can be used to predict whether the subject will be responsive or not to the antibody therapy. Levels of TNFa may be determined in vivo or ex vivo. It further provides methods of locally administering a TNFa antibody, e.g., topically to the intestinal mucosa, for the treatment of IBD.

### SUMMARY OF THE DISCLOSURE

The present invention is defined by the appended claims.

Any subject-matter falling outside the scope of the claims is provided for information purposes only. The potential references to methods of treatment in this description are to be interpreted as references to the compounds, pharmaceutical compositions and medicaments of the present disclosure for use in a method for treatment of the human (or animal) body by therapy (or for diagnosis).

According to an aspect of some aspects of the present disclosure there is provided a method of predicting responsiveness of a subject having an inflammatory bowel disease (IBD) to treatment with a tumor necrosis factor (TNF)-alpha inhibitor, comprising:
analyzing a frequency of at least one subpopulation of immune cells in a tissue biopsy of the subject,
wherein a frequency above a predetermined threshold of immune cells of a subpopulation selected from the group consisting of activated monocytes M1 macrophages, memory B cells, and neutrophils is indicative of the subject being non-responder to the TNF-alpha inhibitor, and/or
wherein a frequency below a predetermined threshold of immune cells of a subpopulation selected from the group consisting of activated monocytes M2 macrophages and CD8+ T cells is indicative of the subject being non-responder to the TNF-alpha inhibitor,
thereby predicting the responsiveness of the subject having the inflammatory bowel disease (IBD) to the TNF-alpha inhibitor.

According to an aspect of some aspects of the present disclosure there is provided a method of selecting treatment to inflammatory bowel disease (IBD) in a subject in need thereof, the method comprising:
(a) determining responsiveness to a TNF-alpha inhibitor according to the method of some aspects of the disclosure; and
(b) selecting treatment based on the responsiveness.

According to an aspect of some aspects of the present disclosure there is provided a method of treating inflammatory bowel disease (IBD) in a subject in need thereof, the method comprising:
(a) determining responsiveness to a TNF-alpha inhibitor according to the method of some aspects of the disclosure; and
(b) treating the subject based on the responsiveness.

According to an aspect of some aspects of the present disclosure there is provided a kit for predicting responsiveness of a subject to a tumor necrosis factor (TNF)-alpha inhibitor comprising an agent capable of analyzing a frequency of at least one subpopulation of immune cells in a tissue biopsy of the subject, and a reference expression data of the frequency of at least one subpopulation of immune cells of a tissue biopsy obtained from at least one TNF-alpha inhibitor responder subject and/or at least one TNF-alpha inhibitor non-responder subject, wherein the immune cells are of a subpopulation selected from the group consisting of: activated monocytes M1 macrophages, memory B cells, neutrophils, activated monocytes M2 macrophages and CD8+ T cells.

According to some aspects of the disclosure, the tissue biopsy of the subject comprises an inflamed tissue.

According to some aspects of the disclosure, the IBD comprises ulcerative colitis (UC).

According to some aspects of the disclosure, the IBD comprises Crohn's disease (CD).

According to some aspects of the disclosure, the tissue biopsy comprises a colon tissue.

According to some aspects of the disclosure, the tissue biopsy comprises an ileum tissue.

According to some aspects of the disclosure, the activated monocytes M1 macrophages are characterized by CD68+ expression signature.

According to some aspects of the disclosure, the activated monocytes M1 macrophages are characterized by CD68+/CCR7+/CD86+/CD80+ expression signature.

According to some aspects of the disclosure, the activated monocytes M1 macrophages are characterized by CD68+/CCR7+/CD86+/CD80+/CD11b+/CCR2+ expression signature.

According to some aspects of the disclosure, the activated monocytes M2 macrophages are characterized by CD68+ expression signature.

According to some aspects of the disclosure, the activated monocytes M2 macrophages are characterized by CD68+/CD163+/CD206+ expression signature.

According to some aspects of the disclosure, the memory B cells are plasma cells, and wherein the plasma cells are characterized by positive expression of CD138.

According to some aspects of the disclosure, the memory B cells are plasma cells, and wherein the plasma cells are characterized by CD138+/ CD45+/BCMA+/CD38+/IgM+/IgG+/IgA+/IgE+.

According to some aspects of the disclosure, the memory B cells are non-plasma cells, and wherein the non-plasma cells are characterized by CD20+/ CD19+/CD45RA+ expression signature.

According to some aspects of the disclosure, the memory B cells are non-plasma cells, and wherein the non-plasma cells are characterized by CD20+/ CD19+/CD45RA+/CD45+/MHC-Class II+/IgG+/IgA+/IgE+/IgD+ expression signature.

According to some aspects of the disclosure, the neutrophils are characterized by CD45+, CD66b+ and/or CD16+ expression signature.

According to some aspects of the disclosure, the CD8+ T cells are characterized by CD8+ expression signature.

According to some aspects of the disclosure, the CD8+ T cells are characterized by CD8+/CD69+ expression signature.

According to some aspects of the disclosure, the CD8+ T cells are characterized by CD8+/CD69+/CD3+/CD45+/CD45RA+ expression signature.

According to some aspects of the disclosure, the subject is a naive subject who hasn't been treated with the TNF-alpha inhibitor.

According to some aspects of the disclosure, the cells of the tissue biopsy are intact cells.

According to some aspects of the disclosure, the analyzing the frequency of the at least one subpopulation of immune cells is performed by a morphometric analysis.

According to some aspects of the disclosure, the analyzing the frequency of the at least one subpopulation of immune cells is performed using at least one histological stain.

According to some aspects of the disclosure, the analyzing the frequency of the at least one subpopulation of immune cells is performed using at least one antibody.

According to some aspects of the disclosure, the antibody is used in an immuno-histochemistry (IHC) or immuno-fluorescence method.

According to some aspects of the disclosure, the antibody is used in flow cytometry or Fluorescence-activated cell sorting (FACS) analysis.

According to some aspects of the disclosure, the analyzing the frequency of the at least one subpopulation of immune cells is performed by an RNA in-situ hybridization assay.

According to some aspects of the disclosure, the analyzing the frequency of the at least one subpopulation of immune cells is performed by a single cell RNA sequencing (RNA SEQ) analysis.

According to some aspects of the disclosure, the analyzing the frequency of the at least one subpopulation of immune cells is performed by exome sequencing.

According to some aspects of the disclosure, the analyzing the frequency of the at least one subpopulation of immune cells is performed by RNA SEQ followed by deconvolution.

According to some aspects of the disclosure, the analyzing the frequency of the at least one subpopulation of immune cells is performed by reverse-transcriptase polymerase chain reaction (RT-PCR) followed by deconvolution.

According to some aspects of the disclosure, the analyzing the frequency of the at least one subpopulation of immune cells is performed by micro array followed by deconvolution.

Unless otherwise defined, all technical and/or scientific terms used herein have the same meaning as commonly understood by one of ordinary skill in the art to which the disclosure pertains. Although methods and materials similar or equivalent to those described herein can be used in the practice or testing of aspects of the disclosure, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some aspects of the disclosure are herein described, by way of example only, with reference to the accompanying drawings. With specific reference now to the drawings in detail, it is stressed that the particulars shown are by way of example and for purposes of illustrative discussion of aspects of the disclosure. In this regard, the description taken with the drawings makes apparent to those skilled in the art how aspects of the disclosure may be practiced.

In the drawings:

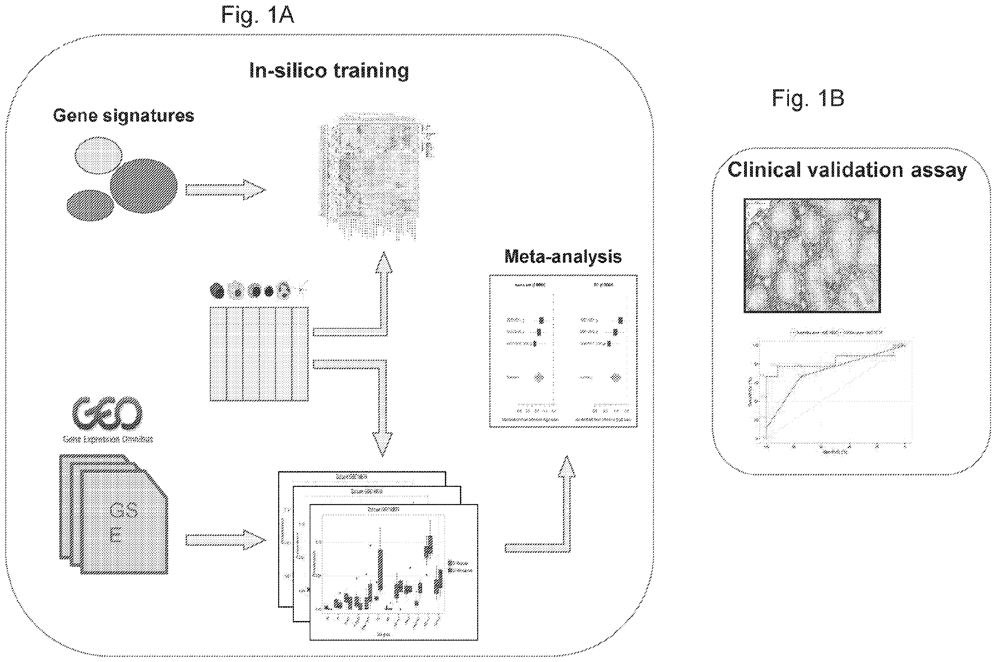

Figs. 1A-B schematically illustrate the cell centered meta-analysis pipeline. Schematic view of the meta-analysis pipeline used to identify baseline cellular signature of response to anti-TNF. Figure 1A - in-silico training. Figure 1B - clinical validation assay.

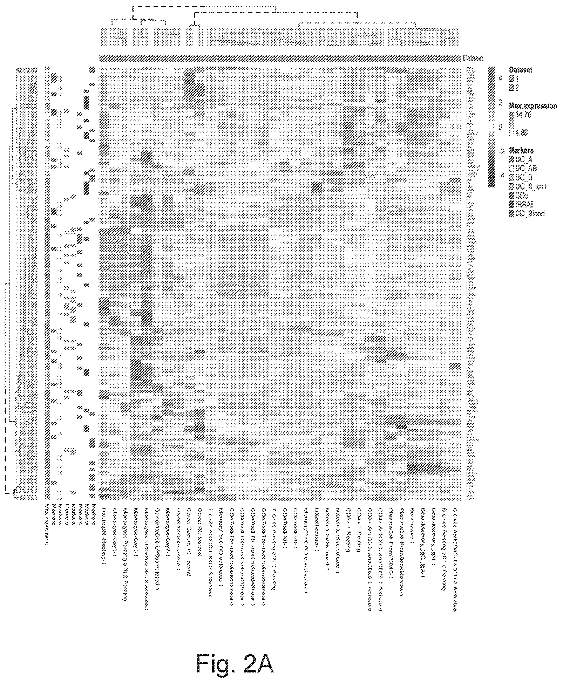

Figs. 2A-B depict the cell type expression analysis of previously reported gene signature. Figure 2A - Expression of gene signatures in immune cell subsets and colon tissue. Each row was standardized (z-score) separately to highlight gene expression cell type-specificity. Color scale range from blue/low to red/high, with white/zero representing average expression across all samples. Rows and columns were clustered using euclidean distance with average linkage. Row annotations indicates the absolute maximum log2 expression of each gene (shades of green), and the membership(s) of each gene to a signature. Row dendrogram clades (8) are coloured to highlight genes that the present inventors associated with specific -- group of -- cell types. Column annotation indicates the GEO dataset from which each sample was obtained. Column dendrogram clades (7) are coloured to highlight cell types that belong to a common lineage. (B) Scores and p-values from single sample enrichment analysis using GSVA.

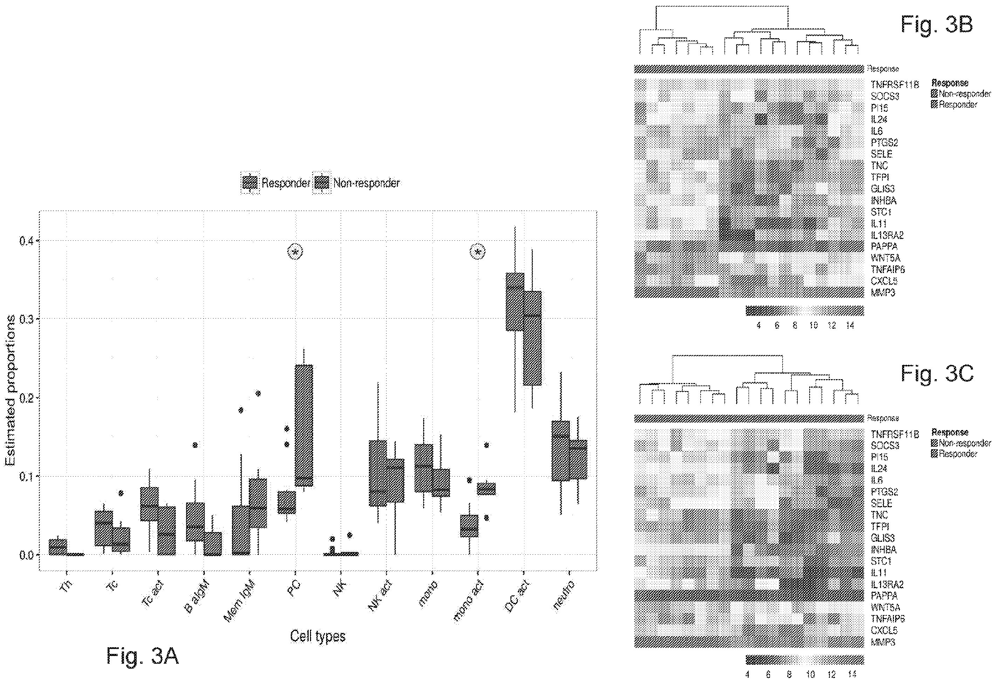

Figs. 3A-C depict computational deconvolution of gene expression data. Figure 3A - Boxplot of estimated proportions in cohort GSE16789 (baseline CD colon samples). Only cell types with non-zero proportions in more than 75% of the samples are shown. Group differences are highlighted by separate boxplots for responders (blue) and non-responders (red). Significant differences are indicated with circled stars (nominal p-value <= 0.05, Wilcoxon rank sum test). The y-axis represents the estimated proportion of each cell type in each sample. "mono act" = M1 Macrophage. Figures 3B-C - Expression of the top 20-genes signature previously identified in UC patients, and shown to be able to predict response in CD patients *(4)* (Figure 3B). After correction for estimated proportions of activated monocytes and plasma cells, the predictive power of this signature drops (Figure 3C). The heatmap shows the log2 expression of each gene. For better comparison, rows in the top panel were clustered using the same metric and linkage method as the columns (dendrogram not shown), and the resulting ordering was applied to the rows in the bottom panel.

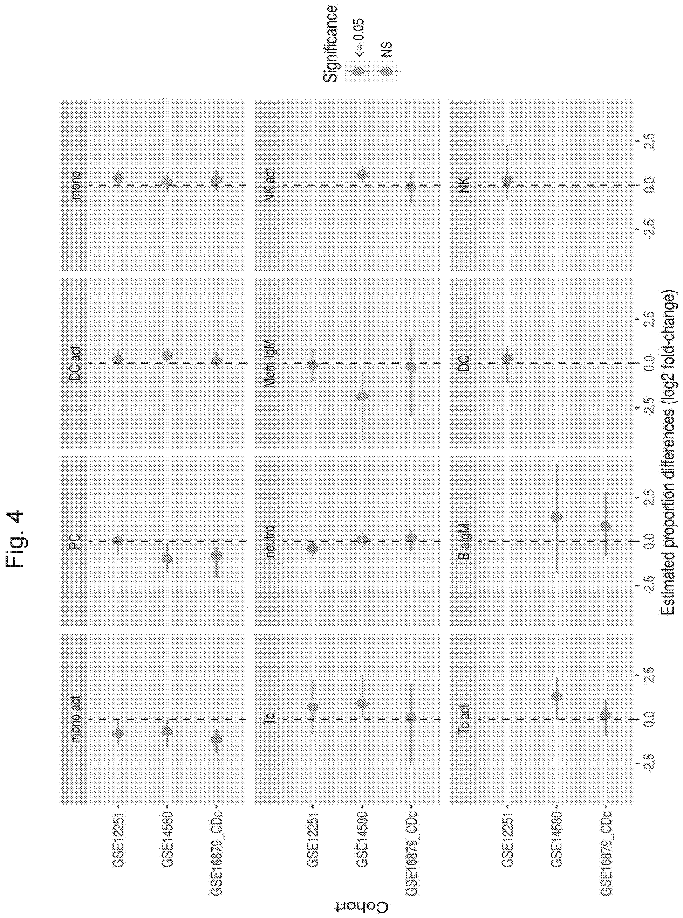

Fig. 4 depicts meta-analysis of cell subset proportion identified consistent immune cell subset different between responders and non-responder to infliximab. Each panel shows estimated group proportion differences (pseudo median) and 95% confidence interval for a given cell subset, across all discovery cohorts. Missing data comes from cell type/cohort pairs not included in the meta-analysis because of too many zero estimated proportions. The x-axis represents the log2 proportion fold change (i.e. log2(Responders/Non-Responders)). The y-axis indicates the discovery cohorts. Statistical significance was calculated using Wilcoxon rank sum test (nominal p-value <= 0.05), and is shown in red (significant) and blue (non-significant). "mono act" = M1 Macrophage.

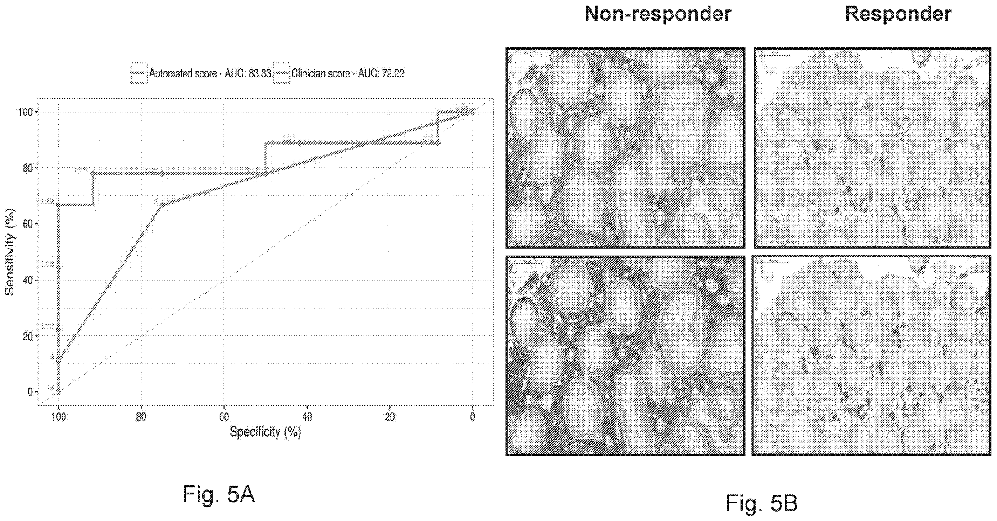

Figs. 5A-B depict validation by staining of plasma cells in independent IBD biopsies. Figure 5A - ROC curve showing the predictive power of plasma cell proportions from staining as quantified by two scoring methods: a clinician categorical score (blue) and automated pixel quantitation (red). The respective Area Under the Curve (AUC) achieved by each scoring method are indicated in the legend. Figure 5B - Staining slides showing visual differences between responders and non-responders. CD138+ plasma cells are colored in brown, showing an increased staining in non-responsive patients. The blue staining indicates the brown regions detected by automated quantitation with ImagePro Plus software.

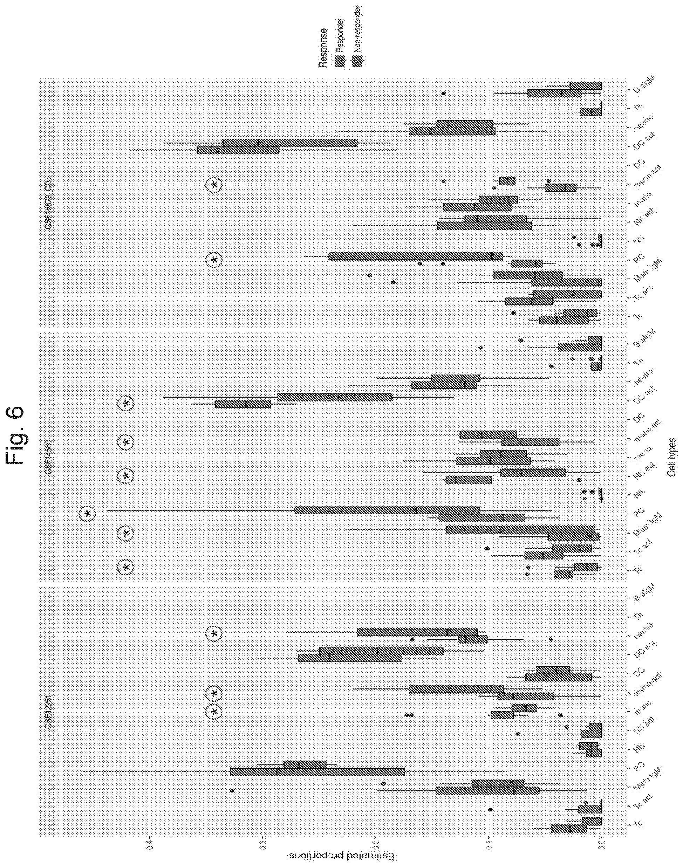

Fig. 6 depicts estimated cell type proportions in all discovery cohorts. Proportions were estimated in each sample separately and compared within each cohort between responders and non-responders. Only cell types with non-zero proportions in more than 75% of the samples are shown. Group differences are highlighted by separate boxplots for responders (blue) and non-responders (red). Significant differences are indicated with circled stars (nominal p-value <= 0.05, wilcoxon rank sum test). "mono act" = M1 Macrophage.

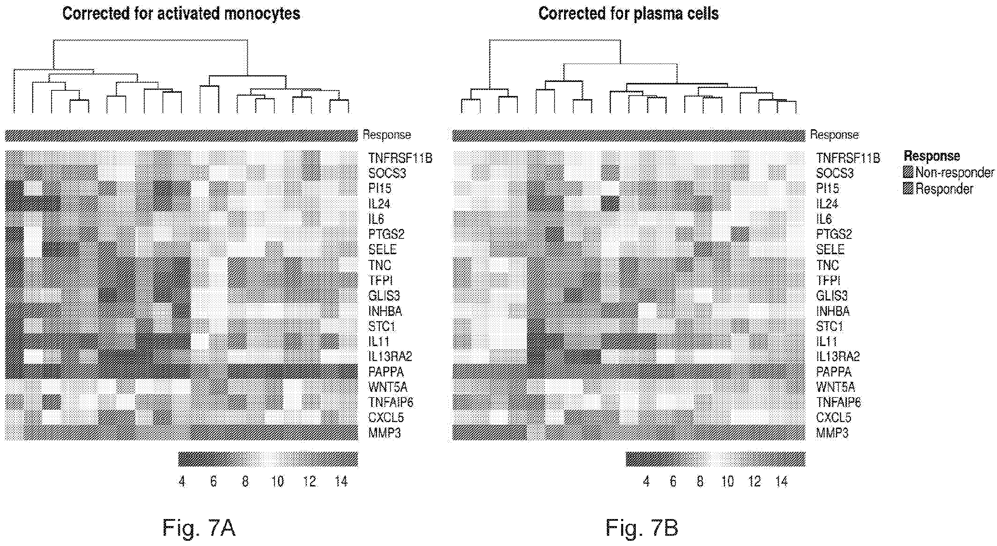

Figs. 7A-B the predictive power of a 20-genes signatures after correction for cell type proportions. Expression of the 20-UC genes predictive signature in CDc samples, after correction for estimated proportions of activated monocytes (Figure 7A) and plasma cells (Figure 7B). After correction, the predictive power of this signature drops. The heatmap shows the log2 expression of each gene. For better comparison, rows in both panels were ordered according to the clustering order in the original data (unadjusted for proportions) shown in Figure 3B.

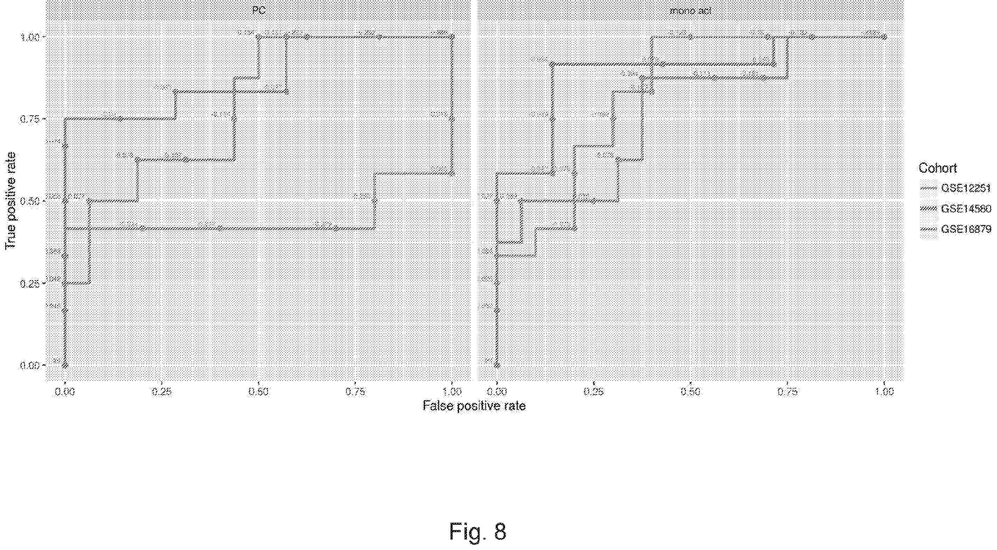

Fig. 8 depicts ROC curve analysis for the cell types selected in each of the discovery cohorts. Each panel shows the ROC curve computed from the estimated proportions of a given cell type [plasma cells (left) and activated monocytes (right)] in each discovery cohort: GSE12251 (red), GSE14580 (green) and GSE16879 (blue). The x and y axis represent the false positive rate (1-specificity) and true positive rate (sensitivity) respectively. "mono act" = M1 Macrophage.

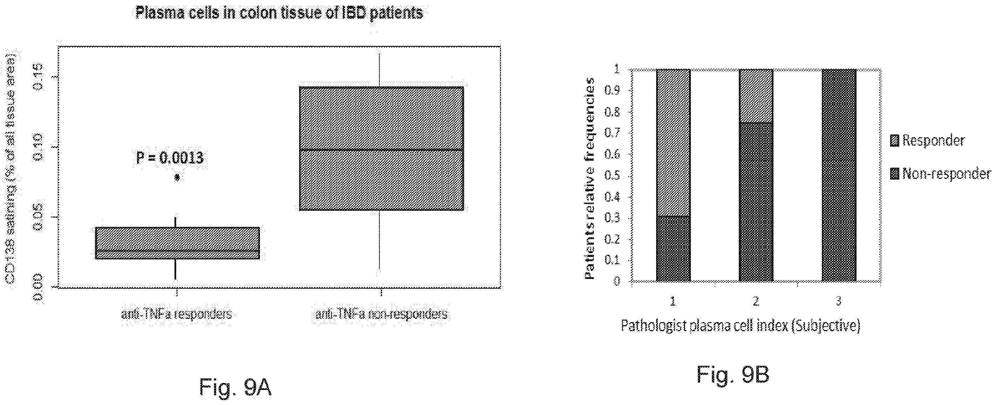

Figs. 9A-B depict results from staining of plasma cells in the validation IBD biopsy samples. Figure 9A - Automated quantitation. Figure 9B - Pathologist blind score. The Y-axis gives the proportion of assessed biopsies achieving a given score (x-axis).

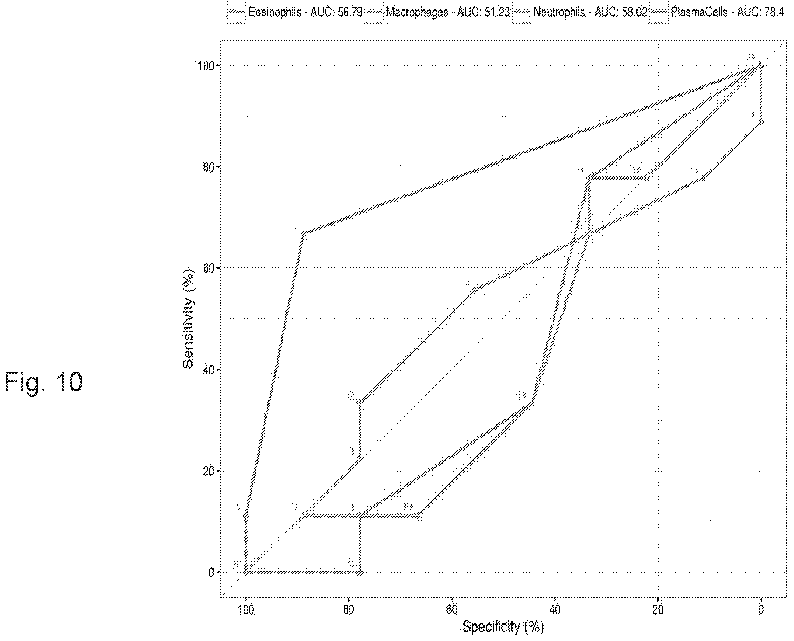

Fig. 10 depicts ROC curve analysis of pathologist validation of cell-types signatures highlights plasma cell differences between anti-TNF responders versus non-responders.

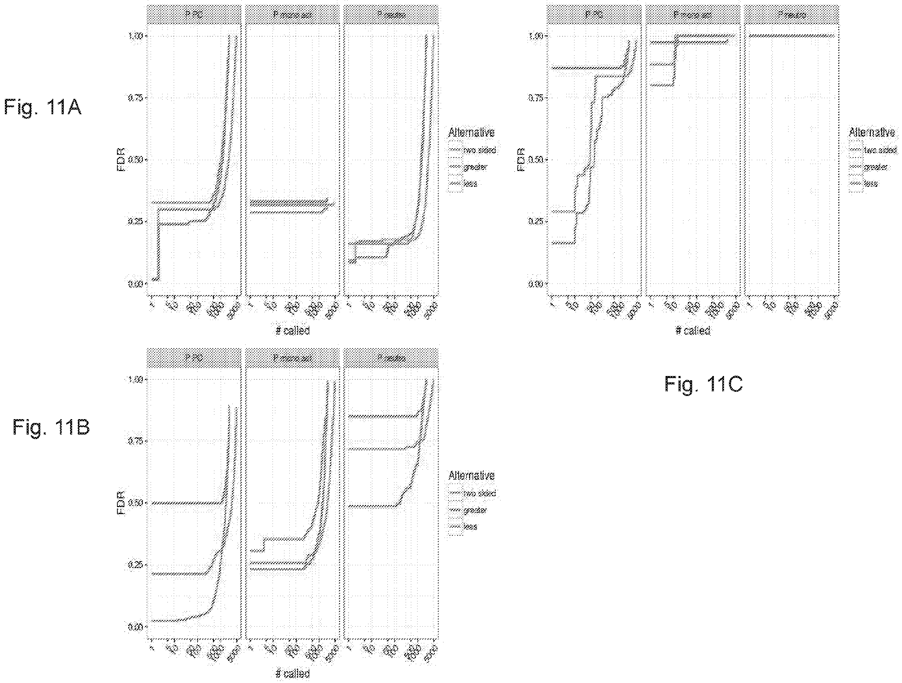

Fig. 11A-C depict cell type specific differential expression in all discovery cohorts. csSAM runs on the 3 discovery cohorts including plasma cells, activated monocytes and neutrophils identifies differentially expressed genes in plasma cells. "mono act" = M1 Macrophage.

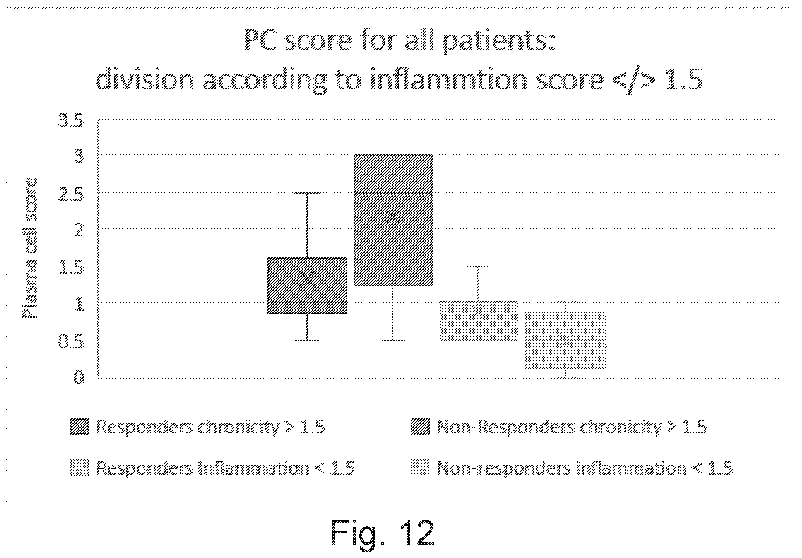

Fig. 12 is a histogram demonstrating that plasma cell proportions in inflamed colon tissue can predict response to infliximab (IFX) prior to treatment initiation. Formalin-fixed slides of paraffin-embedded colon tissues were immunostained with H&E to show the basic tissue morphology. All biopsies were collected prior to IFX therapy initiation. Slides were then coded and interpreted by a specialist pathologist. A specific cell abundance categorical index between 0 and 3 was determined by the pathologist for plasma cells proportion and for inflammation level. Chronic inflammation score was defined as a combined score that reflects tissue distortion and plasmacytosis. Minimal amount of cells or inflammation was scored as "0", whereas the highest cell abundance or inflammation stage detected across all slides was scored as "3". The tissues were scored one by one in a blinded manner. Nine non-responders and twenty responders were included in this 2nd cohort. Inflamed tissue sites (inflammation score > 1.5) were scored from 7 responders and 5 non-responders.

### DESCRIPTION OF SPECIFIC ASPECTS OF THE DISCLOSURE

The present disclosure, in some aspects thereof, relates to methods and kits for predicting responsiveness of a subject having an inflammatory bowel disease (IBD) to treatment with a tumor necrosis factor (TNF)-alpha inhibitor, more particularly, but not exclusively, to methods of selecting a treatment for a subject diagnosed with the IBD. The kits are not part of the invention, since they are not claimed.

Before explaining at least one aspect of the disclosure in detail, it is to be understood that the disclosure is not necessarily limited in its application to the details set forth in the following description or exemplified by the Examples. The disclosure is capable of other aspects or of being practiced or carried out in various ways.

IBD conditions can be treated with a TNF-alpha inhibitor to treat inflammation and achieve mucosal healing. However, response to such treatments is very heterogeneous, with overall only 60% of the patients showing clear primary phenotypic improvement. The remainder of patients do not respond at all, or lose response after a short period. Because of the high cost of the anti-TNF biologics combined with their systemic side effects and the uncertainty of response, these drugs are generally not used as a first line treatment.

The present inventors have hypothesized that the relative proportions of the various immune cell subsets infiltrating the affected tissue does not only reflect disease state, but may also be predictive of a patient's potential to respond to anti-TNF treatment. Thus, as shown in the Examples section which follows, the present inventors analyzed public gene expression data using recent bioinformatics methodology developments that enable the computational deconvolution of mixture data such as blood or bulk tissue, i.e. the estimation of the proportions of constituting cell types directly from heterogeneous samples *(18).* By means of a meta-analysis framework, the present inventors integrated estimated immune cell subset proportions from multiple IBD cohorts, and identified consistent proportion differences between responders and non-responders in immune cells such as macrophages and plasma cells. The implication of plasma cells was further supported by a cell type-specific differential analysis. The present inventors validated these results on an independent set of samples, where plasma cells proportions assessed in immunostained biopsies could predict response to anti-TNF with high accuracy [Area Under the Curve (AUC) 80%]. Overall, these results propose a novel clinically feasible and efficient mean of predicting response to anti-TNF treatment in naive patients, which can be used to improve patient care through maximizing response rate. These results also provide novel insights on the immune target of TNF blockade in IBD.

Thus, according to an aspect of some aspects of the disclosure there is provided a method of predicting responsiveness of a subject having an inflammatory bowel disease (IBD) to treatment with a tumor necrosis factor (TNF)-alpha inhibitor, the method comprising:
analyzing a frequency of at least one subpopulation of immune cells in a tissue biopsy of the subject,
wherein a frequency above a predetermined threshold of immune cells of a subpopulation selected from the group consisting of activated monocytes M1 macrophages, memory B cells, and neutrophils is indicative of the subject being non-responder to the TNF-alpha inhibitor, and/or
wherein a frequency below a predetermined threshold of immune cells of a subpopulation selected from the group consisting of activated monocytes M2 macrophages and CD8+ T cells is indicative of the subject being non-responder to the TNF-alpha inhibitor,
thereby predicting the responsiveness of the subject having the inflammatory bowel disease (IBD) to the treatment with the TNF-alpha inhibitor.

According to some aspects of the disclosure, the tissue biopsy of the subject comprises an inflamed tissue.

As used herein the term "inflammatory bowel disease (IBD)" refers to a pathology characterized by an inflammatory condition of the colon and the small intestine. Crohn's disease (CD) and ulcerative colitis (UC) are the principal types of inflammatory bowel disease.

According to some aspects of the disclosure, the IBD comprises ulcerative colitis (UC).

Ulcerative colitis (UC) is a long-term condition that results in inflammation and ulcers of the colon and rectum. The primary symptom of active disease is abdominal pain and diarrhea mixed with blood. Other common symptoms include, weight loss, fever, anemia, which can be ranged from mild to severe. Symptoms typically occur intermittently with periods of no symptoms between flares; and complications may include megacolon, inflammation of the eye, joints, or liver, and colon cancer.

According to some aspects of the disclosure, the IBD comprises Crohn's disease (CD).

Crohn's disease (CD) is a type of inflammatory bowel disease (IBD) that may affect any part of the gastrointestinal tract from mouth to anus. Signs and symptoms often include abdominal pain, diarrhea (which may be bloody if inflammation is severe), fever, and weight loss. Other complications may include anemia, skin rashes, arthritis, inflammation of the eye, and feeling tired.

As used herein, the term "subject" includes mammals, preferably human beings at any age which suffer from the pathology.

According to some aspects of the disclosure, the subject is a naive subject who hasn't been treated with the TNF-alpha inhibitor.

According to some aspects of the disclosure, the subject is refractory to corticosteroids and/or immunosuppression treatment. For example, the subject has been subjected to corticosteroids and/or immunosuppression treatment, yet without sufficient, or any therapeutic effect.

As used herein the phrase "TNF-alpha" or "tumor necrosis factor alpha", which is interchangeably used herein, refers to a multifunctional pro-inflammatory cytokine [also known as DIF; TNFA; TNFSF2; TNLG1F;] that belongs to the tumor necrosis factor (TNF) superfamily. TNF-alpha is mainly secreted by macrophages. It can bind to, and thus functions through its receptors TNFRSF1A/TNFR1 and TNFRSF1B/TNFBR. This cytokine is involved in the regulation of a wide spectrum of biological processes including cell proliferation, differentiation, apoptosis, lipid metabolism, and coagulation, and is being implicated in a variety of diseases, including autoimmune diseases, insulin resistance, and cancer.

It should be noted that the "responsiveness" of a subject to a TNF-alpha inhibitor refers to the success or failure of treatment of the subject with the TNF-alpha inhibitor.

A positive response to TNF-alpha inhibitor refers to an improvement following treatment with the TNF-alpha inhibitor in at least one relevant clinical parameter as compared to an untreated subject diagnosed with the same pathology (e.g., the same type, stage, degree and/or classification of the pathology), or as compared to the clinical parameters of the same subject prior to treatment with the TNF-alpha inhibitor. Hence, improvement of clinical symptom(s) following treatment implicates that the subject is a "responder" to the treatment.

On the other hand, a negative response to the treatment with the TNF-alpha inhibitor means that the subject has no sufficient improvement in clinical symptoms, or has a complete lack of improvement of clinical symptoms, or has a worsening of clinical symptoms characterizing the pathology (the IBD condition), with or without appearance of antibodies (e.g., antibody against infliximab) which neutralize the TNF-alpha inhibitor. Such a subject is a "non-responder" to the treatment.

According to some aspects of the disclosure, a subject diagnosed with and/or suffering from Crohn's disease or ulcerative colitis is considered to be a responder to the treatment with the TNF-alpha inhibitor if his follow-up clinical data (a year after biopsy) point to remission by Physicians Global Assessment (PGA), laboratory parameters [haemoglobin (Hb), erythrocyte sedimentation rate (ESR), C-reactive protein (CRP), albumin] and used medicines (e.g., steroids, 5-ASA, thiopurines, methotrexate, biologics).

According to some aspects of the disclosure, a subject diagnosed with and/or suffering from Crohn's disease or ulcerative colitis is considered to be a non-responder to the treatment with the TNF-alpha inhibitor if his follow-up clinical data (a year after biopsy) point to continuous flare/chronic disease by Physicians Global Assessment (PGA), laboratory parameters (Hb, ESR, CRP, albumin) and used medicines (e.g., steroids, 5-ASA, thiopurines, methotrexate, biologics).

For example, a positive response to treatment with TNF-alpha inhibitor in a subject having an IBD such as ulcerative colitis (UC) or Crohn's disease (CD) disease is a mucosal healing.

Additional and/or alternative parameters which indicate a positive response to the treatment with the TNF-alpha inhibitor (thus indicating that the subject is responder to treatment) include, for example, reduction in the number of liquid or very soft stools; reduction in the abdominal pain; reduction in symptoms or findings presumed related to Crohn's disease: arthritis or arthralgia, iritis or uveitis, erythema nodosum, pyoderma gangrenosum, aphthous stomatitis, anal fissure, fistula or perirectal abscess, other bowel-related fistula, febrile (fever), episode over 100 degrees during past week; and/or reduction in abdominal mass.

The response (i.e., positive or negative) for treatment with the TNF-alpha inhibitor of some aspects of the disclosure can be evaluated using known and accepted medical indexes and/or calculators.

For example, the Crohn's Disease Activity Index (CDAI) calculator gauges the progress or lack of progress for people with Crohn's disease. It is accepted that CDAI scores below 150 indicate a better prognosis than higher scores.

The CDAI calculator takes into consideration the following parameters:
(1). Number of liquid or very soft stools in one week;
(2). Sum of seven daily abdominal pain ratings: (0=none, 1=mild, 2=moderate, 3=severe);
(3). Sum of seven daily ratings of general well-being: (0=well, 1=slightly below par, 2=poor, 3=very poor, 4=terrible);
(4). Symptoms or findings presumed related to Crohn's disease: arthritis or arthralgia, iritis or uveitis, erythema nodosum, pyoderma gangrenosum, aphthous stomatitis, anal fissure, fistula or perirectal abscess, other bowel-related fistula, febrile (fever) episode over 100 degrees during past week;
(5). Taking Lomotil or opiates for diarrhea;
(6). Abnormal mass: 0=none; 0.4=questionable; 1=present
(7). Hematocrit [ (Typical - Current) x 6 ] Normal average: For Male = 47 For Female = 42;
(8). 100 x [(standard weight-actual body weight) / standard weight]

Additionally or alternatively, the clinical status of patients with CD following treatment with the TNF-alpha inhibitor can be evaluated using the Harvey-Bradshaw index (HBI) which was devised in 1980 as a simpler version of the Crohn's disease activity index (CDAI) for data collection purposes. It consists of only clinical parameters.

Following is a non-limiting an exemplary calculator for score using the HBI index.

### Table 1

**Table 1: Harvey-Bradshaw index (HBI).**

| **Parameter** | **Scoring** |
|---|---|
| General well-being | very well +0 |
| | slightly below par +1 |
| | poor +2 |
| | very poor +3 |
| | terrible +4 |
| Abdominal pain | none +0 |
| | mild +1 |
| | moderate +2 |
| | severe +3 |

| Number of liquid stools per day | |
|---|---|
| Abdominal mass | none +0 |
| | dubious +1 |
| | definite +2 |
| | definite and tender +3 |
| Complications | none +0 |
| | arthralgia +1 |
| | uveitis +1 |
| | erythema nodosum +1 |
| | aphthous ulcers +1 |
| | pyoderma gangrenosum +1 |
| | anal fissure +1 |
| | new fistula +1 |

| **Parameter** | **Scoring** |
|---|---|
| | abscess +1 |

Patients with Crohn's disease who scored 3 or less on the HBI are very likely to be in remission according to the CDAI. Patients with a score of 8 to 9 or higher are considered to have severe disease.

According to some aspects of the disclosure, a subject diagnosed with and/or suffering from Crohn's disease is considered to be a responder to treatment with the TNF-alpha inhibitor if the Crohn's Disease Activity Index (CDAI) score is 150 or less.

According to some aspects of the disclosure, a subject diagnosed with and/or suffering from Crohn's disease is considered to be a responder to treatment if following treatment with the TNF-alpha inhibitor the Crohn's Disease Activity Index (CDAI) score is reduced in at least 70 points as compared to the CDAI score prior to the treatment.

According to some aspects of the disclosure, a subject diagnosed with and/or suffering from Crohn's disease is considered to be a responder to treatment if following treatment with the TNF-alpha inhibitor the Crohn's Disease Activity Index (CDAI) score is reduced in at least 70 points, e.g., by at least 71, 72, 73, 74, 75, 76, 77, 78, 79, 80, 81, 82, 83, 84, 85, 86, 87, 88, 89, 90, 95, 100, 105, 110, 115, 120, 125, 130, 135, 140, 145, or 150 points as compared to the CDAI score prior to the treatment.

According to some aspects of the disclosure, a subject diagnosed with and/or suffering from Crohn's disease is considered to be a non-responder to the treatment with the TNF-alpha inhibitor if the Crohn's Disease Activity Index (CDAI) score is higher than 220.

According to some aspects of the disclosure, a subject diagnosed with and/or suffering from Crohn's disease is considered to be a non-responder to the treatment if following treatment with the TNF-alpha inhibitor the Crohn's Disease Activity Index (CDAI) score remains the same or even increased as compared to the CDAI score prior to the treatment.

According to some aspects of the disclosure, a subject diagnosed with and/or suffering from Crohn's disease is considered to be a non-responder to the treatment if following treatment with the TNF-alpha inhibitor the Crohn's Disease Activity Index (CDAI) score was reduced in a value lower than 69 as compared to the CDAI score prior to the treatment.

According to some aspects of the disclosure, a subject diagnosed with and/or suffering from Crohn's disease is considered to be a non-responder to the treatment if following treatment with the TNF-alpha inhibitor the Crohn's Disease Activity Index (CDAI) score was reduced in a value lower than 69 points, e.g., the CDAI is lower than 68, 67, 66, 65, 64, 63, 62, 61, 60, 59, 58, 57, 56, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2 or 1 point(s) as compared to the CDAI score prior to the treatment.

For patients having ulcerative colitis (UC) the Mayo Clinic scoring system (Rutgeerts P, Sandborn WJ, Feagan BG, Reinisch W, et al. Infliximab for induction and maintenance therapy for ulcerative colitis. N Engl J Med. 2005 Dec 8;353(23):2462-76) can be used for assessments of UC activity before or following treatment with the TNF-alpha inhibitor. The Mayo score ranges from 0 to 12, with higher scores indicating more severe disease. This score can be used for both initial evaluation and monitoring treatment response.

Table 2 provides an exemplary calculator according to the Mayo Clinic scoring system.

### Table 2

**Table 2.**

| **Stool Frequency** |
|---|
| Normal number of stools for patient |
| 1 to 2 stools per day more than normal |
| 3 to 4 stools more than normal |
| >= 5 stools more than normal |

| **Rectal Bleeding** |
|---|
| No blood seen. |
| Streaks of blood with stool less than half the time. |
| Obvious blood with stool most of the time. |
| Blood alone passes. |

| **Endoscopic findings** |
|---|
| Normal or inactive disease. |
| Mild Disease. |
| Moderate Disease. |
| Severe Disease. |

| **Physician's Global Assessment** |
|---|
| Normal |
| Mild disease |
| Moderate disease |
| Severe disease |

According to some aspects of the disclosure, a subject diagnosed with and/or suffering from ulcerative colitis is considered to be a responder to treatment with the TNF-alpha inhibitor if the Mayo Clinic score is 2 or less.

According to some aspects of the disclosure, a subject diagnosed with and/or suffering from ulcerative colitis is considered to be a responder to treatment if following treatment with the TNF-alpha inhibitor the Mayo Clinic score is reduced in at least 2 points as compared to the Mayo Clinic score prior to the treatment.

According to some aspects of the disclosure, a subject diagnosed with and/or suffering from ulcerative colitis is considered to be a non-responder to the treatment if following treatment with the TNF-alpha inhibitor the Mayo Clinic score remains the same or even increased as compared to the Mayo Clinic score prior to the treatment.

Following is a non-limiting description of determining responsiveness of a subject to the anti-TNF treatment.

### Clinical evaluation of patients:

The clinical state of the patients can be evaluated using the Harvey Bradshaw Index (HBI) at each visit in the Doctor's clinic. Clinical state was defined as either remission, mild disease, moderate disease, or severe disease based on the HBI score definition. Subjects can be defined as clinical responders if clinical state improved or remained at remission during all visits.

***Biomarker response** -* evaluated biomarkers include, but are not limited to serum C-reactive protein (CRP) and fecal calprotectin. The determination of responders or non-responders can be performed using the following guidelines:
(1) Subjects having at least 2 fecal calprotectin samples taken at least 1 week apart are considered responders when at least a 50% reduction in levels is demonstrated in the second sample retrieved from the feces of the subject.
(2) Subjects who stably remain at normal levels of fecal calprotectin (≤ 50 mg/gram of feces) at all visits, regardless of serum CRP are considered responders.
(3) Subjects with less than 2 samples of fecal calprotectin are considered responders when demonstrated at least a 50% reduction in serum CRP levels in a second blood sample taken at least a week after the first blood sample.
(4) Subjects who exhibit normal levels of CRP (:S 5 mg/dl) at all visits are considered responders.

***Steroid dependence** -* The persistent need of concurrent steroid therapy is a valuable marker of disease state and of response to therapy. Subjects, who are receiving steroid therapy at the clinic visit at the 14^{th} week of treatment (" 14-week") are considered non-responders.

***Immunogenic status*** - Subjects having measurable serum antibodies to Infliximab at their week 14-week visit are considered non-responders.

Study response algorithm - The present inventors have formulated a decision algorithm to conclude whether a subject is responsive or not to therapy. The algorithm is mainly based on the primary gastroenterologist following the subject. For each subject on the 14-week visit the physician, after reviewing the subjects' records, decides whether the subject responded to therapy, failed or if it is still indeterminate. For the latter (indeterminate), a decision tree is performed with the following steps: a definition of failure is set when steroid treatment is given at 14-week visit. If no steroids are given the next step is to test the biomarker dynamics. A substantial reduction in fecal calprotectin is defined as response. If fecal calprotectin is not available, a reduction in serum CRP (as defined previously) is considered a response to treatment. For subjects who are not steroid-dependent and show no substantial biomarker dynamics, a physician decision on week 26 is made to determine the response status.

As used herein the phrase a "TNF-alpha inhibitor" refers to an agent capable of inhibiting (e.g., downregulating) the expression level and/or activity of tumor necrosis factor alpha (TNFα) and/or capable of competing and/or antagonizing the TNFα activity.

For example, the anti TNFα inhibitor can inhibit the binding to TNFα to its TNFRSF1A/TNFR1 and/or TNFRSF1B/TNFBR receptors.

According to some aspects of the disclosure, the TNF-alpha inhibitor is an antibody.

Non-limiting examples of anti-TNFα antibodies include, Infliximab, adalimumab, and certolizumab pegol.

Infliximab (e.g., marketed as REMICADE^{™} REMSIMA^{™}, INFLECTRA^{™}) is a chimeric IgG1κ monoclonal antibody (composed of human constant and murine variable regions) used as a biologic drug against tumor necrosis factor alpha (TNF-α) that is a key part of the autoimmune reaction. Infliximab neutralizes the biological activity of TNFα by binding with high affinity to the soluble and transmembrane forms of TNFα and inhibits binding of RNFα with its receptors. Infliximab has a molecular weight of approximately 149.1 kilodaltons, and is produced by a recombinant cell line cultured by continuous perfusion and is purified by a series of steps that includes measures to inactivate and remove viruses. Infliximab is used to treat autoimmune diseases such as Crohn's disease, ulcerative colitis, psoriasis, psoriatic arthritis, ankylosing spondylitis, and rheumatoid arthritis.

For example, treatment with Infliximab (IFX) can include, for example, intravenous infusion of 5 mg IFX per kg body weight. If additional treatment is needed, subsequent doses of IFX can be administered, e.g., after 2 and 6 weeks of the first dose of administration of IFX.

Adalimumab (e.g., marketed as HUMIRA^{™} and EXEMPTIA) is a medication used for rheumatoid arthritis, psoriatic arthritis, ankylosing spondylitis, Crohn's disease, ulcerative colitis, moderate to severe chronic psoriasis, moderate to severe hidradenitis suppurativa, and juvenile idiopathic arthritis. In rheumatoid arthritis, adalimumab has a response rate similar to methotrexate, and in combination nearly doubles the response rate of methotrexate alone. Like Infliximab, Adalimumab binds to TNFα and prevents it from activating TNF receptors.

Certolizumab pegol (e.g., CDP870, marketed as CIMZIA^{™}) is a therapeutic monoclonal antibody to tumor necrosis factor alpha (TNF-α), for the treatment of Crohn's disease and rheumatoid arthritis.

Antibodies and methods of generating, isolating and/or using same are further described hereinunder.

According to some aspects of the disclosure, the TNF-alpha inhibitor is an antagonist of TNFα such as a soluble TNF receptor.

Non-limiting examples of soluble TNF receptors which can be used according to some aspects of the disclosure include ENBREL^{™} (Etanercept). Like Infliximab, Etanercept binds to TNFα, preventing it from activating TNF receptors.

Etanercept is a fusion protein produced by recombinant DNA. It fuses the TNF receptor to the constant end of the IgG1 antibody.

As described hereinabove, the method of some aspects of the disclosure comprises analyzing a frequency of at least one subpopulation of immune cells in a tissue biopsy of the subject.

The tissue biopsy used by the method of some aspects comprises a colon tissue.

The tissue biopsy used by the method of some aspects comprises an ileum.

According to some aspects of the disclosure, the cells of the tissue biopsy are intact cells.

According to some aspects of the disclosure, a frequency above a predetermined threshold of immune cells of a subpopulation selected from the group consisting of activated monocytes M1 macrophages, memory B cells, and neutrophils is indicative of the subject being non-responder to the TNF-alpha inhibitor.

As used herein the phrase "frequency above a predetermined threshold" refers to a frequency of the subpopulation of immune cells which is at least 0.01%, 0.02%, 0.03%. 0.04%, 0.05%, 0.06%, 0.07%, 0.08%, 0.09%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40% or higher than a predetermined threshold.

The predetermined threshold can be determined by the frequency of the subpopulation of immune cells in the same tissue biopsy of a subject with a known outcome of TNF-alpha inhibitor treatment (i.e., responder or non-responder), yet, wherein the tissue biopsy of the subject is obtained prior to the first administration of the TNF-alpha inhibitor to the subject (i.e., when the subject is naive to the TNF-alpha treatment). Such a subject can be considered a reference subject. The reference subject can be a TNF-alpha responder or a TNF-alpha non-responder.

Non-limiting exemplary ranges of the subpopulations of immune cells in responders and non-responders patients can be found in Table 11 of the Examples section which follows.

According to some aspects of the disclosure, a frequency of activated monocytes M1 macrophages which is above 10% (e.g., above 11%) indicates that the subject is predicted to be a non-responder to the treatment with the TNF-alpha inhibitor.

According to some aspects of the disclosure, a frequency of plasma cells which is above 14% indicates that the subject is predicted to be a non-responder to the treatment with the TNF-alpha inhibitor.

According to some aspects of the disclosure, a frequency of neutrophils which is above 13% (e.g., above 14%) indicates that the subject is predicted to be a non-responder to the treatment with the TNF-alpha inhibitor.

According to some aspects of the disclosure, a ratio of M1/M2 macrophages which is higher than 1, e.g., higher than 1.1 is indicative of the subject being non-responder to treatment with the TNF-alpha inhibitor.

According to some aspects of the disclosure, the method of predicting responsiveness of a subject having an inflammatory bowel disease (IBD) to a TNF-alpha inhibitor can be performed by:
(a) analyzing a frequency of at least one subpopulation of immune cells in a tissue biopsy of the subject,
   and;
(b) comparing the frequency of the at least one subpopulation of immune cells in the tissue biopsy of the subject to an expression data of the at least one subpopulation of immune cells in a corresponding tissue biopsy obtained from at least one TNF-alpha inhibitor responder subject and/or at least one TNF-alpha inhibitor non-responder subject, thereby predicting the responsiveness of the subject to the TNF-alpha inhibitor treatment.

As mentioned, the tissue biopsy can be from an inflamed region as determined by tissue distortion and plasmacytosis.

According to some aspects of the disclosure, when the tissue biopsy comprises both an inflamed tissue and a non-inflamed tissue the method can sufficiently determine the responsiveness of the subject to (TNF)-alpha inhibitor therapy based on frequencies of macrophages or plasma cells.

According to some aspects of the disclosure, when the tissue biopsy comprises mainly an inflamed tissue the method can sufficiently determine the responsiveness of the subject to (TNF)-alpha inhibitor therapy based on frequencies of plasma cells or macrophages

According to some aspects of the disclosure, the activated monocytes M1 macrophages are characterized by CD68+ and one of CCR7+, CD86+ or CD80+ or a combination of CCR7+, CD86+ and CD80+ as core expression signature.

It should be noted that the sign "+" as used herein refers to a positive expression *(i.e.,* the cell expresses the indicated marker); and the sign "-" as used herein refers to a negative expression *(i.e.,* the cell does not express the indicated marker).

According to some aspects of the disclosure, the activated monocytes M1 macrophages are characterized by CD68+/ CCR7+ expression signature.

According to some aspects of the disclosure, the activated monocytes M1 macrophages are characterized by CD68+/ CD86+ expression signature.

According to some aspects of the disclosure, the activated monocytes M1 macrophages are characterized by CD68+/ CD80+ expression signature.

According to some aspects of the disclosure, the activated monocytes M1 macrophages are characterized by CD68+/ CCR7+/ CD86+ expression signature.

According to some aspects of the disclosure, the activated monocytes M1 macrophages are characterized by CD68+/ CCR7+/ CD80+ expression signature.

According to some aspects of the disclosure, the activated monocytes M1 macrophages are characterized by CD68+/ CD86+/ CD80+ expression signature.

According to some aspects of the disclosure, the activated monocytes M1 macrophages are characterized by CD68+/ CCR7+/ CD86+/ CD80+ expression signature.

Additionally or alternatively, the activated monocytes M1 macrophages are further characterized by CD11b+ and/or CCR2+ expression markers.

According to some aspects of the disclosure, the activated monocytes M1 macrophages are characterized by CD68+/ CCR7+/ CD11b+/ CCR2+ expression signature.

According to some aspects of the disclosure, the activated monocytes M1 macrophages are characterized by CD68+/ CCR7+/ CD86+/ CD80+/ CD11b+ expression signature.

According to some aspects of the disclosure, the activated monocytes M1 macrophages are characterized by CD68+/ CCR7+/ CD86+/ CD80+/ CCR2+ expression signature.

According to some aspects of the disclosure, the activated monocytes M1 macrophages are characterized by CD68+/ CCR7+/ CD86+/ CD80+/ CD11b+/ CCR2+ expression signature.

According to some aspects of the disclosure, the memory B cells are plasma cells, and wherein the plasma cells are characterized by positive expression of a marker selected from the group consisting of: CD138 as a core signature, and optionally one or more of the markers selected from the group consisting of: CD45, BCMA, CD38, IgM, IgG, IgA and/or IgE.

According to some aspects of the disclosure, the plasma cells are characterized by CD138+ expression signature.

According to some aspects of the disclosure, the plasma cells are further characterized by a positive expression of one or more markers of the CD45, BCMA, CD38, IgM, IgG, IgA and/or IgE markers.

According to some aspects of the disclosure, the plasma cells are characterized by CD138+/ CD45+ expression signature.

According to some aspects of the disclosure, the plasma cells are characterized by CD138+/ BCMA+ expression signature.

According to some aspects of the disclosure, the plasma cells are characterized by CD138+/ CD38+ expression signature.

According to some aspects of the disclosure, the plasma cells are characterized by CD138+/ IgM+ expression signature.

According to some aspects of the disclosure, the plasma cells are characterized by CD138+/IgG+ expression signature.

According to some aspects of the disclosure, the plasma cells are characterized by CD138+/IgE+ expression signature.

According to some aspects of the disclosure, the plasma cells are characterized by CD138+/CD45+/BCMA+/CD38+/IgM+/IgG+/IgA+/IgE+ expression signature.

According to some aspects of the disclosure, the memory B cells are non-plasma cells, and wherein the non-plasma cells are characterized by positive expression of CD20, CD19, and CD45RA as a core signature.

According to some aspects of the disclosure, the non-plasma cells are further characterized by an expression of at least one marker or a combination of markers selected from the group of CD45, MHC-Class II, IgG, IgA, IgE and/or IgD markers.

According to some aspects of the disclosure, the neutrophils are characterized by CD45+, CD66b+ and/or CD16+ expression signature.

According to some aspects of the disclosure, a frequency below a predetermined threshold of immune cells of a subpopulation selected from the group consisting of activated monocytes M2 macrophages and CD8+ T cells is indicative of the subject being non-responder to the TNF-alpha inhibitor,

As used herein the phrase "frequency below a predetermined threshold" refers to a frequency of the subpopulation of immune cells which is lower than 50%, 45%, 44%, 43%, 42%, 41%, 40%, 39%, 38%, 37%, 36%, 35%, 34%, 33%, 32%, 31%, 30%, 29%, 28%, 27%, 26%, 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, 0.9%, 0.08%, 0.07%, 0.06%, 0.05%, 0.04%, 0.03%, 0.02%, 0.01% of a predetermined threshold.

According to some aspects of the disclosure, a frequency of CD8+ T cells which is lower than 2% indicates that the subject is predicted to be a non-responder to the treatment with the TNF-alpha inhibitor.

According to some aspects of the disclosure, the activated monocytes M2 macrophages are characterized by CD68+ expression signature.

According to some aspects of the disclosure, the activated monocytes M2 macrophages are further characterized by expression of one or more markers selected from the group consisting of CD163+ and CD206+.

According to some aspects of the disclosure, the activated monocytes M2 macrophages are characterized by CD68+/ CD163+ expression signature.

According to some aspects of the disclosure, the activated monocytes M2 macrophages are characterized by CD68+/ CD206+ expression signature.

According to some aspects of the disclosure, the activated monocytes M2 macrophages are characterized by CD68+/ CD163+/ CD206+ expression signature.

According to some aspects of the disclosure, the CD8+ T cells are characterized by CD8+ expression signature.

According to some aspects of the disclosure, the CD8+ T cells are characterized by CD8+ and CD69+ expression signature.

According to some aspects of the disclosure, the CD8+ T cells are characterized by CD8+/CD3+ expression signature.

According to some aspects of the disclosure, the CD8+ T cells are characterized by CD8+/ CD45+ expression signature.

According to some aspects of the disclosure, the CD8+ T cells are characterized by CD8+/ CD45RA+ expression signature.

According to some aspects of the disclosure, the CD8+ T cells are characterized by CD8+/CD69+/CD3+ expression signature.

According to some aspects of the disclosure, the CD8+ T cells are characterized by CD8+/CD69+/CD45+ expression signature.

According to some aspects of the disclosure, the CD8+ T cells are characterized by CD8+/CD69+/ CD45RA+ expression signature.

According to some aspects of the disclosure, the CD8+ T cells are characterized by CD8+/CD69+/CD3+/CD45+/CD45RA+ expression signature.

Analysis of the frequency of at least one subpopulation of immune cells can be performed by determining the presence of the subpopulation of immune cells in the sample and calculating the frequencies thereof out of the total immune cells present in the sample. Methods of determining which subpopulations of immune cells are present in a sample include, for example, identification of cell types from the cells in the sample and calculating the frequencies of each subpopulation of immune cells.

According to some aspects of the disclosure, the analyzing the frequency of the at least one subpopulation of immune cells is performed by a morphometric analysis.

According to some aspects of the disclosure, the analyzing the frequency of the at least one subpopulation of immune cells is performed by using at least one histological stain.

According to some aspects of the disclosure, the analyzing the frequency of the at least one subpopulation of immune cells is performed by using at least one antibody.

According to some aspects of the disclosure, the antibody is used in an immuno-histochemistry (IHC) or immuno-fluorescence method.

According to some aspects of the disclosure, the antibody is used in a flow cytometry or Fluorescence-activated cell sorting (FACS) analysis.

According to some aspects of the disclosure, the analyzing the frequency of the at least one subpopulation of immune cells is performed by mass-cytometry.

According to some aspects of the disclosure, the mass-cytometry is CyTOF (e.g., FLUIDIGM^{R}).

According to some aspects of the disclosure, the analyzing the frequency of the at least one subpopulation of immune cells is performed by an RNA in-situ hybridization assay.

According to some aspects of the disclosure, the analyzing the frequency of the at least one subpopulation of immune cells is performed by a single cell RNA sequencing (RNA SEQ) analysis.

According to some aspects of the disclosure, the analyzing the frequency of the at least one subpopulation of immune cells is performed by exome sequencing followed by computational deconvolution.

According to some aspects of the disclosure, the analyzing the frequency of the at least one subpopulation of immune cells is performed by RNA SEQ followed by computational deconvolution.

According to some aspects of the disclosure, the analyzing the frequency of the at least one subpopulation of immune cells is performed by reverse-transcriptase polymerase chain reaction (RT-PCR) followed by computational deconvolution.

According to some aspects of the disclosure, the analyzing the frequency of the at least one subpopulation of immune cells is performed by microarray followed by computational deconvolution.

According to an aspect of some aspects of the disclosure, there is provided a method of selecting treatment to inflammatory bowel disease (IBD) in a subject in need thereof, the method comprising:
(a) determining responsiveness to a treatment with a TNF-alpha inhibitor according to the method of some aspects of the disclosure (e.g., any of the aspects described hereinabove); and
(b) selecting treatment based on the responsiveness.

According to an aspect of some aspects of the disclosure, there is provided a method of treating to inflammatory bowel disease (IBD) in a subject in need thereof, the method comprising:
(a) determining responsiveness to a TNF-alpha inhibitor according to the method of some aspects of the disclosure; and
(b) treating the subject based on the responsiveness.

The term "treating" refers to inhibiting, preventing or arresting the development of a pathology (disease, disorder or condition) and/or causing the reduction, remission, or regression of a pathology. Those of skill in the art will understand that various methodologies and assays can be used to assess the development of a pathology, and similarly, various methodologies and assays may be used to assess the reduction, remission or regression of a pathology.

The treatment of the subject, e.g., the treatment plan or regimen, depends on the predicted responsiveness of the subject to the TNF-alpha inhibitor. For example, if the subject is predicted to response to the TNF-alpha inhibitor (a TNF-alpha inhibitor responder subject), then the treatment selected for treating such a responder subject can include administration of the TNF-alpha inhibitor. On the other hand, if the subject is predicted to not respond to the TNF-alpha inhibitor (a TNF-alpha non-responder subject), then the treatment selected for treating such as non-responder subject will not include the TNF-alpha inhibitor.

The agents of some aspects of the disclosure which are described herein for predicting responsiveness of a subject to treatments with a tumor necrosis factor (TNF)-alpha inhibitor may be included in a diagnostic kit/article of manufacture preferably along with appropriate instructions for use and labels indicating FDA approval for use in diagnosing and/or assessing the prediction of responsiveness of a subject to treatment with a tumor necrosis factor (TNF)-alpha inhibitor.

Such a kit can include, for example, at least one container including at least one of the herein described diagnostic agents (e.g., an antibody which can specifically bind to a cell marker characteristic of the immune cell subpopulation; or a probe which can specifically hybridize to and/or elongate a nucleic acid sequence, e.g., an RNA sequence, characteristic of the immune cell subpopulation) and an imaging reagent packed in another container (e.g., enzymes, secondary antibodies, buffers, chromogenic substrates, fluorogenic material). The kit may also include appropriate buffers and preservatives for improving the shelf-life of the kit.

According to an aspect of some aspects of the disclosure, there is provided a kit for predicting responsiveness of a subject to treatment with a tumor necrosis factor (TNF)-alpha inhibitor comprising an agent capable of analyzing a frequency of at least one subpopulation of immune cells in a tissue biopsy of the subject, and a reference expression data of the frequency of at least one subpopulation of immune cells of a tissue biopsy obtained from at least one TNF-alpha inhibitor responder subject and/or at least one TNF-alpha inhibitor non-responder subject, wherein the immune cells are of a subpopulation selected from the group consisting of: activated monocytes M1 macrophages, memory B cells, neutrophils, activated monocytes M2 macrophages and CD8+ T cells.

Table 3 hereinbelow, provides a non-limiting description of suitable agents (e.g., antibodies) for identifying subpopulations of immune cells from the tissue biopsy. It should be noted that the antibodies can be directly (e.g., by conjugation to a label) or indirectly labeled (e.g., by conjugation to an identifiable moiety) for visualization and further detection.

### Table 3

**Table 3.**

| ***Population*** | ***Antibody*** | ***Catalogue number*** | ***Company*** |
|---|---|---|---|
| Plasma cells | Mouse anti human CD138 | MCA2459GA | AbD Serotec |
| Activated monocytes (M1) | Mouse anti Human CD68 | MCA5709 | AbD Serotec |
| | Mouse anti human CD80 | 305202 | BioLegend |
| | Goat anti Human CD86 | AF-141-NA | R&D Systems |
| | Mouse anti human CCR7 | MAB197 | R&D Systems |
| Activated monocytes (M2) | Mouse anti Human CD68 | MCA5709 | AbD Serotec |
| | Mouse anti human CD163 | MCA1853 | AbD Serotec |
| | Mouse anti human CD206 | MCA5552Z | AbD Serotec |
| CD8+ T cells | mouse anti human CD8 | MCA1817T | AbD Serotec |
| | mouse anti human CD69 | NBP2-25236 | Novus |
| Neutrophils | rabbit anti human CD16 | BS-6028R-A488 | BioSS |
| | mouse anti human C66b | NB100-77808 | Novus |
| Memory B cells | mouse anti human CD20 | MCA1915T | AbD Serotec |
| | mouse anti human CD19 | MCA2454 | AbD Serotec |
| | mouse anti human CD45RA | MCA88 | AbD Serotec |

Table 4 provides a non-limiting sequence information for the antigens (markers) which can be used to identify the various immune cells (e.g., subpopulation of cells) according to some aspects of the disclosure. The Table provides the GenBank Accession numbers (and the respective sequence identifiers) for the polypeptides of the antigens (cell markers) and the polynucleotide encoding same. It should be noted that the polypeptides can be identified using various protein detection methods such as those described hereinunder; and that the polynucleotides can be identified using various RNA detection methods such as those described hereinunder.

### Table 4

**Table 4.**

| ***Marker (presence* "+"; *absence "-")*** | ***Polypeptide GenBank Accession No.*** | ***Polypeptide SEQ ID NO:*** | ***Polynucleotide GenBank Accession No.*** | ***Polynucleotide SEQ ID NO:*** |
|---|---|---|---|---|
| CD68+ | NP_001035148 | 5 | NM_001040059.1 | 37 |
| CD68+ | NP_001242.2 | 6 | NM_001251.2 | 38 |
| CD86+ | NP_001193853.1 | 7 | NM_001206924.1 | 39 |
| CD86+ | NP_001193854.1 | 8 | NM_001206925.1 | 40 |
| CD86+ | NP_008820.3 | 9 | NM_006889.4 | 41 |
| CD86+ | NP_787058.4 | 10 | NM_175862.4 | 42 |
| CD86+ | NP_795711.1 | 11 | NM_176892.1 | 43 |
| CD64+ | NP_000557.1 | 12 | NM_000566.3 | 44 |
| CD20+ | NP_061883.1 | 13 | NM_019010.2 | 45 |
| CD19+ | NP_001761.3 | 14 | NM_001770.5 | 46 |
| CD19+ | NP_001171569.1 | 15 | NM_001178098.1 | 47 |
| IgD+ | - | | NG_001019.5 (977531..984804) | 48 |
| IgA+ | NP_067612.1 | 17 | NM_021601.3 | 49 |
| IgA+ | NP_001774.1 | 18 | NM_001783.3 | 50 |
| CD138+ | NP_001006947.1 | 19 | NM_001006946.1 | 51 |
| CD138+ | NP_002988.3 | 20 | NM_002997.4 | 52 |
| CD45+ | NP_001254727.1 | 21 | NM_001267798.1 | 53 |
| CD45+ | NP_002829.3 | 22 | NM_002838.4 | 54 |
| CD45+ | NP_563578.2 | 23 | NM_080921.3 | 55 |
| CD66b+ | NP_001807.2 | 24 | NM_001816.3 | 56 |
| CD16+ | NP_000560.5 | 25 | NM_000569.6 | 57 |
| CD16+ | NP_001121064.1 | 26 | NM_001127592.1 | 58 |
| CD16+ | NP_001121065.1 | 27 | NM_001127593.1 | 59 |
| CD16+ | NP_001121067.1 | 28 | NM_001127595.1 | 60 |
| CD16+ | NP_001121068.1 | 29 | NM_001127596.1 | 61 |
| CD 163+ | NP_004235.4 | 30 | NM_004244.5 | 62 |
| CD 163+ | NP_981961.2 | 31 | NM_203416.3 | 63 |
| CD206+ | NP_002429.1 | 32 | NM_002438.3 | 64 |
| CD8+ | NP_001139345.1 | 33 | NM_001145873.1 | 65 |
| CD8+ | NP_001759.3 | 34 | NM_001768.6 | 66 |
| CD8+ | NP_741969.1 | 35 | NM_171827.3 | 67 |
| CD69+ | NP_001772.1 | 36 | NM_001781.2 | 68 |

Following is a non-limiting description of methods of detecting RNA and/or protein sequences within cells of the tissue biopsy of some aspects of the disclosure.

### Methods of detecting the expression level of RNA

The expression level of the RNA in the cells of some aspects of the disclosure can be determined using methods known in the arts.

***RT-PCR analysis:*** This method uses PCR amplification of relatively rare RNAs molecules. First, RNA molecules are purified from the cells and converted into complementary DNA (cDNA) using a reverse transcriptase enzyme (such as an MMLV-RT) and primers such as, oligo dT, random hexamers or gene specific primers. Then by applying gene specific primers and Taq DNA polymerase, a PCR amplification reaction is carried out in a PCR machine. Those of skills in the art are capable of selecting the length and sequence of the gene specific primers and the PCR conditions *(i.e.,* annealing temperatures, number of cycles and the like) which are suitable for detecting specific RNA molecules. It will be appreciated that a semi-quantitative RT-PCR reaction can be employed by adjusting the number of PCR cycles and comparing the amplification product to known controls.

***RNA in situ hybridization stain:*** In this method DNA or RNA probes are attached to the RNA molecules present in the cells. Generally, the cells are first fixed to microscopic slides to preserve the cellular structure and to prevent the RNA molecules from being degraded and then are subjected to hybridization buffer containing the labeled probe. The hybridization buffer includes reagents such as formamide and salts (e.g., sodium chloride and sodium citrate) which enable specific hybridization of the DNA or RNA probes with their target mRNA molecules *in situ* while avoiding non-specific binding of probe. Those of skills in the art are capable of adjusting the hybridization conditions (*i.e.,* temperature, concentration of salts and formamide and the like) to specific probes and types of cells. Following hybridization, any unbound probe is washed off and the bound probe is detected using known methods.

For example, if a radio-labeled probe is used, then the slide is subjected to a photographic emulsion which reveals signals generated using radio-labeled probes; if the probe was labeled with an enzyme then the enzyme-specific substrate is added for the formation of a colorimetric reaction; if the probe is labeled using a fluorescent label, then the bound probe is revealed using a fluorescent microscope; if the probe is labeled using a tag (e.g., digoxigenin, biotin, and the like) then the bound probe can be detected following interaction with a tag-specific antibody which can be detected using known methods.

***In situ RT-PCR stain:*** This method is described in Nuovo GJ, et al. [Intracellular localization of polymerase chain reaction (PCR)-amplified hepatitis C cDNA. Am J Surg Pathol. 1993, 17: 683-90] and Komminoth P, et al. [Evaluation of methods for hepatitis C virus detection in archival liver biopsies. Comparison of histology, immunohistochemistry, in situ hybridization, reverse transcriptase polymerase chain reaction (RT-PCR) and in situ RT-PCR. Pathol Res Pract. 1994, 190: 1017-25]. Briefly, the RT-PCR reaction is performed on fixed cells by incorporating labeled nucleotides to the PCR reaction. The reaction is carried on using a specific *in situ* RT-PCR apparatus such as the laser-capture microdissection PixCell I LCM system available from Arcturus Engineering (Mountainview, CA).

***Oligonucleotide microarray*** - In this method oligonucleotide probes capable of specifically hybridizing with the polynucleotides of some aspects of the disclosure are attached to a solid surface (e.g., a glass wafer). Each oligonucleotide probe is of approximately 20-25 nucleic acids in length. To detect the expression pattern of the polynucleotides of some aspects of the disclosure in a specific cell sample (e.g., blood cells), RNA is extracted from the cell sample using methods known in the art (using e.g., a TRIZOL solution, Gibco BRL, USA). Hybridization can take place using either labeled oligonucleotide probes (e.g., 5'-biotinylated probes) or labeled fragments of complementary DNA (cDNA) or RNA (cRNA).

Briefly, double stranded cDNA is prepared from the RNA using reverse transcriptase (RT) (e.g., Superscript II RT), DNA ligase and DNA polymerase I, all according to manufacturer's instructions (Invitrogen Life Technologies, Frederick, MD, USA). To prepare labeled cRNA, the double stranded cDNA is subjected to an *in vitro* transcription reaction in the presence of biotinylated nucleotides using e.g., the BioArray High Yield RNA Transcript Labeling Kit (Enzo, Diagnostics, Affymetix Santa Clara CA). For efficient hybridization the labeled cRNA can be fragmented by incubating the RNA in 40 mM Tris Acetate (pH 8.1), 100 mM potassium acetate and 30 mM magnesium acetate for 35 minutes at 94 °C. Following hybridization, the microarray is washed and the hybridization signal is scanned using a confocal laser fluorescence scanner which measures fluorescence intensity emitted by the labeled cRNA bound to the probe arrays.

For example, in the Affymetrix microarray (Affymetrix^{®}, Santa Clara, CA) each gene on the array is represented by a series of different oligonucleotide probes, of which, each probe pair consists of a perfect match oligonucleotide and a mismatch oligonucleotide. While the perfect match probe has a sequence exactly complimentary to the particular gene, thus enabling the measurement of the level of expression of the particular gene, the mismatch probe differs from the perfect match probe by a single base substitution at the center base position. The hybridization signal is scanned using the Agilent scanner, and the Microarray Suite software subtracts the non-specific signal resulting from the mismatch probe from the signal resulting from the perfect match probe.

***Exome sequencing*** (also known as Whole Exome Sequencing, WES or WXS) is a targeted sequencing approach that is restricted to the protein-coding regions of genomes (exome). The exome is estimated to encompass approximately 1% of the genome, yet contains approximately 85% of disease-causing mutations. In the initial step, the subset of DNA encoding proteins (exons) are selected, followed by sequencing of the exons using a high throughput DNA sequencing technology. The exome sequencing enables a rapid, cost-effective identification of common single nucleotide variants (SNVs), copy number variations (CNVs), and small insertions or deletions (indels), as well as rare de novo mutations that may explain the heritability of Mendelian and complex disorders. Exome sequencing can be performed using, e.g., the Ion Torrent^{™} Next-Generation Sequencing (Available from ThermoFisher Scientific).

***Strand Specific RNA-Sequencing Library Construction -*** The following is a representative protocol for the preparation of sequencing libraries from purified RNAs. This protocol is optimized for very low amounts of input RNA, and uses an adapter-ligation strategy in order to map locations of crosslinks (e.g., for the AMT protocol). This RNA-sequencing protocol also includes several steps that remove contaminating ssDNA probes.

RNA can be extracted using the miRNeasy kit (Qiagen, 217004) and poly(A) RNA is further isolated using, for example, Oligo d (T25) beads (NEB, E7490L). The Poly(A) fraction is then fragmented (Invitrogen, AM8740), and fragments smaller than 200 bps are preferably eliminated (Zymo, R1016) and the remaining fraction is treated with FastAP Thermosensitive Alkaline Phosphatase (Thermo Scientific, EF0652) and T4 Polynucleotide Kinase (NEB, M0201L). RNA is then ligated to a RNA adaptor essentially as described in Engreitz, J. M. et al. Science 341: 1237973, (2013), using T4 RNA Ligase 1 (NEB, M0204L), which is then used to facilitate cDNA synthesis using Affinity Script Multiple Temperature Reverse Transcriptase (Agilent, 600105). More specifically, the following adaptors reported in Engreitz, J. M. *et al. 2013* can be used:
RNA sequencing - RiL-19 3' RNA adaptor:
   /Phosphate/rArGrArUrCrGrGrArArGrArGrCrGrUrCrGrUrG/ddC (SEQ ID NO: 1);
   RNA sequencing - AR17 RT primer: ACACGACGCTCTTCCGA (SEQ ID NO: 2);
RNA sequencing - 3Tr3 5' DNA adaptor:
   /Phosphate/AGATCGGAAGAGCACACGTCTG/ddC (SEQ ID NO: 3);
RNA sequencing - PCR enrichment:

AATGATACGGCGACCACCGAGATCTACACTCTTTCCCTACACGACGCTCTTC
CGATCTCAAGCAGAAGACGGCATACGAGATNNNNNNNNGTGACTGGAGTTC
AGACGTGTGCTCTTCCGATCT (SEQ ID NO: 4).

RNA is then degraded and the cDNA is ligated to a DNA adaptor using T4 RNA Ligase 1 as described in Engreitz, J. M. *et al. 2013.* Final library amplification is completed using NEB Next High Fidelity 2X PCT Master Mix (M054L). To clean up the final PCR and removed adapter dimers, two subsequent 1X and 8X SPRI reactions ire completed to prepare the final library for sequencing.

### Methods of detecting the expression level of protein

Non-limiting examples of protein detection methods include, flow cytometry (e.g., intra or extra-cellular flow cytometry), FACS, ELISA, Western Blot, RIA, immunohistochemistry, protein activity assays and Mass cytometry (e.g., CyTOF (FLUIDIGM^{R})).

***Mass cytometry:*** Mass-cytometry uniquely combines time-of-flight mass spectrometry with Maxpar metal-labeling technology to enable breakthrough discovery and comprehensive functional profiling applications. Cellular targets are labeled with metal-tagged antibodies and detected and quantified by time-of-flight mass spectrometry. The high purity and choice of metal isotopes ensure minimal background noise from signal overlap or endogenous cellular components. For example, CyTOF (Fludigm) is a recently introduced mass-cytometer capable of detecting up to 40 markers conjugated to heavy metals simultaneously on single cells.

***Enzyme linked immunosorbent assay (ELISA):*** This method involves fixation of a sample (e.g., fixed cells or a proteinaceous solution) containing a protein substrate to a surface such as a well of a microtiter plate. A substrate specific antibody coupled to an enzyme is applied and allowed to bind to the substrate. Presence of the antibody is then detected and quantitated by a colorimetric reaction employing the enzyme coupled to the antibody. Enzymes commonly employed in this method include horseradish peroxidase and alkaline phosphatase. If well calibrated and within the linear range of response, the amount of substrate present in the sample is proportional to the amount of color produced. A substrate standard is generally employed to improve quantitative accuracy.

***Western blot:*** This method involves separation of a substrate from other protein by means of an acrylamide gel followed by transfer of the substrate to a membrane (e.g., nylon or PVDF). Presence of the substrate is then detected by antibodies specific to the substrate, which are in turn detected by antibody binding reagents. Antibody binding reagents may be, for example, protein A, or other antibodies. Antibody binding reagents may be radiolabeled or enzyme linked as described hereinabove. Detection may be by autoradiography, colorimetric reaction or chemiluminescence. This method allows both quantitation of an amount of substrate and determination of its identity by a relative position on the membrane which is indicative of a migration distance in the acrylamide gel during electrophoresis.

***Radio-immunoassay (RIA):*** In one version, this method involves precipitation of the desired protein (*i.e.,* the substrate) with a specific antibody and radiolabeled antibody binding protein (e.g., protein A labeled with I¹²⁵) immobilized on a precipitable carrier such as agarose beads. The number of counts in the precipitated pellet is proportional to the amount of substrate.

In an alternate version of the RIA, a labeled substrate and an unlabeled antibody binding protein are employed. A sample containing an unknown amount of substrate is added in varying amounts. The decrease in precipitated counts from the labeled substrate is proportional to the amount of substrate in the added sample.

***Fluorescence activated cell sorting (FACS):*** This method involves detection of a substrate *in situ* in cells by substrate specific antibodies. The substrate specific antibodies are linked to fluorophores. Detection is by means of a cell sorting machine which reads the wavelength of light emitted from each cell as it passes through a light beam. This method may employ two or more antibodies simultaneously.

***Immunohistochemical analysis:*** This method involves detection of a substrate *in situ* in fixed cells by substrate specific antibodies. The substrate specific antibodies may be enzyme linked or linked to fluorophores. Detection is by microscopy and subjective or automatic evaluation. If enzyme linked antibodies are employed, a colorimetric reaction may be required. It will be appreciated that immunohistochemistry is often followed by counterstaining of the cell nuclei using for example Hematoxyline or Giemsa stain.

***In situ activity assay:*** According to this method, a chromogenic substrate is applied on the cells containing an active enzyme and the enzyme catalyzes a reaction in which the substrate is decomposed to produce a chromogenic product visible by a light or a fluorescent microscope.

***In vitro activity assays:*** In these methods the activity of a particular enzyme is measured in a protein mixture extracted from the cells. The activity can be measured in a spectrophotometer well using colorimetric methods or can be measured in a non-denaturing acrylamide gel (*i.e.,* activity gel). Following electrophoresis, the gel is soaked in a solution containing a substrate and colorimetric reagents. The resulting stained band corresponds to the enzymatic activity of the protein of interest. If well calibrated and within the linear range of response, the amount of enzyme present in the sample is proportional to the amount of color produced. An enzyme standard is generally employed to improve quantitative accuracy.

As mentioned above, the analysis of the subpopulations of immune cells can employ a method of detecting total RNA (e.g., RT-PCR, microarray, RNA SEQ, exome sequencing) or protein (e.g., ELISA, immunofluorescence, immuno-histochemistry) or DNA methylation (e.g., Methylation microarray) in a biological sample, followed by a computational deconvolution.

***Computational deconvolution:*** This method involves using computational algorithms to estimate the composition/proportion of constituting cell subpopulation in bulk samples assayed on a given technology. Often, but not necessarily, this makes use of prior knowledge in the form of cell subset markers or profiles from the same assay.

Deconvolution algorithms have been proposed for a variety of assays, including but not only, gene expression measured by microarray or RNA-seq, and methylation arrays essentially as described elsewhere *(18).*

As used herein, the term "antibody" refers to a substantially intact antibody molecule.

As used herein, the phrase "antibody fragment" refers to a functional fragment of an antibody (such as Fab, F(ab')2, Fv or single domain molecules such as VH and VL) that is capable of binding to an epitope of an antigen.

Suitable Antibody fragments for practicing some aspects of the disclosure include a complementarity-determining region (CDR) of an immunoglobulin light chain (referred to herein as "light chain"), a complementarity-determining region of an immunoglobulin heavy chain (referred to herein as "heavy chain"), a variable region of a light chain, a variable region of a heavy chain, a light chain, a heavy chain, an Fd fragment, and antibody fragments comprising essentially whole variable regions of both light and heavy chains such as an Fv, a single chain Fv, an Fab, an Fab', and an F(ab')2.

Functional antibody fragments comprising whole or essentially whole variable regions of both light and heavy chains are defined as follows:
(i) Fv, defined as a genetically engineered fragment consisting of the variable region of the light chain and the variable region of the heavy chain expressed as two chains;
(ii) single chain Fv ("scFv"), a genetically engineered single chain molecule including the variable region of the light chain and the variable region of the heavy chain, linked by a suitable polypeptide linker as a genetically fused single chain molecule.
(iii) Fab, a fragment of an antibody molecule containing a monovalent antigen-binding portion of an antibody molecule which can be obtained by treating whole antibody with the enzyme papain to yield the intact light chain and the Fd fragment of the heavy chain which consists of the variable and CH1 domains thereof;
(iv) Fab', a fragment of an antibody molecule containing a monovalent antigen-binding portion of an antibody molecule which can be obtained by treating whole antibody with the enzyme pepsin, followed by reduction (two Fab' fragments are obtained per antibody molecule);
(v) F(ab')2, a fragment of an antibody molecule containing a monovalent antigen-binding portion of an antibody molecule which can be obtained by treating whole antibody with the enzyme pepsin (i.e., a dimer of Fab' fragments held together by two disulfide bonds); and
(vi) Single domain antibodies are composed of a single VH or VL domains which exhibit sufficient affinity to the antigen.

Methods of generating antibodies (*i.e.,* monoclonal and polyclonal) are well known in the art. Antibodies may be generated via any one of several methods known in the art, which methods can employ induction of in-vivo production of antibody molecules, screening of immunoglobulin libraries (Orlandi D.R. et al., 1989. Proc. Natl. Acad. Sci. U. S. A. 86:3833-3837; Winter G. et al., 1991. Nature 349:293-299) or generation of monoclonal antibody molecules by continuous cell lines in culture. These include, but are not limited to, the hybridoma technique, the human B-cell hybridoma technique, and the Epstein-Barr virus (EBV)-hybridoma technique (Kohler G. et al., 1975. Nature 256:495-497; Kozbor D. et al., 1985. J. Immunol. Methods 81:31-42; Cote RJ. et al., 1983. Proc. Natl. Acad. Sci. U. S. A. 80:2026-2030; Cole SP. et al., 1984. Mol. Cell. Biol. 62:109-120).

In cases where target antigens are too small to elicit an adequate immunogenic response when generating antibodies in-vivo, such antigens (haptens) can be coupled to antigenically neutral carriers such as keyhole limpet hemocyanin (KLH) or serum albumin [e.g., bovine serum albumine (BSA)] carriers (see, for example, US. Pat. Nos. 5,189,178 and 5,239,078]. Coupling a hapten to a carrier can be effected using methods well known in the art. For example, direct coupling to amino groups can be effected and optionally followed by reduction of the imino linkage formed.

Alternatively, the carrier can be coupled using condensing agents such as dicyclohexyl carbodiimide or other carbodiimide dehydrating agents. Linker compounds can also be used to effect the coupling; both homobifunctional and heterobifunctional linkers are available from Pierce Chemical Company, Rockford, Ill.

The resulting immunogenic complex can then be injected into suitable mammalian subjects such as mice, rabbits, and the like. Suitable protocols involve repeated injection of the immunogen in the presence of adjuvants according to a schedule which boosts production of antibodies in the serum. The titers of the immune serum can readily be measured using immunoassay procedures which are well known in the art.

The antisera obtained can be used directly or monoclonal antibodies may be obtained as described hereinabove.

Antibody fragments can be obtained using methods well known in the art. [(see, for example, Harlow and Lane, "Antibodies: A Laboratory Manual", Cold Spring Harbor Laboratory, New York, (1988)]. For example, antibody fragments according to some aspects of the disclosure can be prepared by proteolytic hydrolysis of the antibody or by expression in E. coli or mammalian cells (e.g., Chinese hamster ovary cell culture or other protein expression systems) of DNA encoding the fragment.

Alternatively, antibody fragments can be obtained by pepsin or papain digestion of whole antibodies by conventional methods. As described hereinabove, an (Fab')2 antibody fragments can be produced by enzymatic cleavage of antibodies with pepsin to provide a 5S fragment. This fragment can be further cleaved using a thiol reducing agent, and optionally a blocking group for the sulfhydryl groups resulting from cleavage of disulfide linkages to produce 3.5S Fab' monovalent fragments. Alternatively, enzymatic cleavage using pepsin produces two monovalent Fab' fragments and an Fc fragment directly. Ample guidance for practicing such methods is provided in the literature of the art (for example, refer to: Goldenberg, U.S. Pat. Nos. 4,036,945 and 4,331,647; Porter, RR., 1959. Biochem. J. 73:119-126). Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

As described hereinabove, an Fv is composed of paired heavy chain variable and light chain variable domains. This association may be noncovalent (see, for example, Inbar et al., 1972. Proc. Natl. Acad. Sci. USA. 69:2659-62). Alternatively, as described hereinabove the variable domains can be linked to generate a single chain Fv by an intermolecular disulfide bond, or alternately, such chains may be cross-linked by chemicals such as glutaraldehyde.

Preferably, the Fv is a single chain Fv.

Single chain Fv's are prepared by constructing a structural gene comprising DNA sequences encoding the heavy chain variable and light chain variable domains connected by an oligonucleotide encoding a peptide linker. The structural gene is inserted into an expression vector, which is subsequently introduced into a host cell such as E. coli. The recombinant host cells synthesize a single polypeptide chain with a linker peptide bridging the two variable domains. Ample guidance for producing single chain Fv's is provided in the literature of the art (for example, refer to: Whitlow and Filpula, 1991. Methods 2:97-105; Bird et al., 1988. Science 242:423-426; Pack et al., 1993. Bio/Technology 11:1271-77; and Ladner et al., U.S. Pat. No. 4,946,778).

Isolated complementarity determining region peptides can be obtained by constructing genes encoding the complementarity determining region of an antibody of interest. Such genes may be prepared, for example, by RT-PCR of mRNA of an antibody-producing cell. Ample guidance for practicing such methods is provided in the literature of the art (for example, refer to Larrick and Fry, 1991. Methods 2:106-10).

It will be appreciated that for human therapy or diagnostics, humanized antibodies are preferably used. Humanized forms of non human (e.g., murine) antibodies are genetically engineered chimeric antibodies or antibody fragments having-preferably minimal-portions derived from non human antibodies. Humanized antibodies include antibodies in which complementary determining regions of a human antibody (recipient antibody) are replaced by residues from a complementarity determining region of a non human species (donor antibody) such as mouse, rat or rabbit having the desired functionality. In some instances, Fv framework residues of the human antibody are replaced by corresponding non human residues.

Humanized antibodies may also comprise residues which are found neither in the recipient antibody nor in the imported complementarity determining region or framework sequences.

In general, the humanized antibody will comprise substantially all of at least one, and typically two, variable domains, in which all or substantially all of the complementarity determining regions correspond to those of a non human antibody and all, or substantially all, of the framework regions correspond to those of a relevant human consensus sequence.

Humanized antibodies optimally also include at least a portion of an antibody constant region, such as an Fc region, typically derived from a human antibody (see, for example, Jones et al., 1986. Nature 321:522-525; Riechmann et al., 1988. Nature 332:323-329; and Presta, 1992. Curr. Op. Struct. Biol. 2:593-596).

Methods for humanizing non human antibodies are well known in the art. Generally, a humanized antibody has one or more amino acid residues introduced into it from a source which is non human. These non human amino acid residues are often referred to as imported residues which are typically taken from an imported variable domain. Humanization can be essentially performed as described (see, for example: Jones et al., 1986. Nature 321:522-525; Riechmann et al., 1988. Nature 332:323-327; Verhoeyen et al., 1988. Science 239:1534-1536; U.S. Pat. No. 4,816,567) by substituting human complementarity determining regions with corresponding rodent complementarity determining regions.

Accordingly, such humanized antibodies are chimeric antibodies, wherein substantially less than an intact human variable domain has been substituted by the corresponding sequence from a non human species. In practice, humanized antibodies may be typically human antibodies in which some complementarity determining region residues and possibly some framework residues are substituted by residues from analogous sites in rodent antibodies.

Human antibodies can also be produced using various techniques known in the art, including phage display libraries [see, for example, Hoogenboom and Winter, 1991. J. Mol. Biol. 227:381; Marks et al., 1991. J. Mol. Biol. 222:581; Cole et al., "Monoclonal Antibodies and Cancer Therapy", Alan R. Liss, pp. 77 (1985); Boerner et al., 1991. J. Immunol. 147:86-95). Humanized antibodies can also be made by introducing sequences encoding human immunoglobulin loci into transgenic animals, e.g., into mice in which the endogenous immunoglobulin genes have been partially or completely inactivated. Upon antigenic challenge, human antibody production is observed in such animals which closely resembles that seen in humans in all respects, including gene rearrangement, chain assembly, and antibody repertoire. Ample guidance for practicing such an approach is provided in the literature of the art (for example, refer to: U.S. Pat. Nos. 5,545,807, 5,545,806, 5,569,825, 5,625,126, 5,633,425, and 5,661,016; Marks et al., 1992. Bio/Technology 10:779-783; Lonberg et al., 1994. Nature 368:856-859; Morrison, 1994. Nature 368:812-13; Fishwild et al., 1996. Nature Biotechnology 14:845-51; Neuberger, 1996. Nature Biotechnology 14:826; Lonberg and Huszar, 1995. Intern. Rev. Immunol. 13:65-93).

It will be appreciated that targeting of particular compartment within the cell can be achieved using intracellular antibodies (also known as "intrabodies"). These are essentially SCA to which intracellular localization signals have been added (e.g., ER, mitochondrial, nuclear, cytoplasmic). This technology has been successfully applied in the art (for review, see Richardson and Marasco, 1995, TIBTECH vol. 13). Intrabodies have been shown to virtually eliminate the expression of otherwise abundant cell surface receptors and to inhibit a protein function within a cell (See, for example, Richardson et al., 1995, Proc. Natl. Acad. Sci. USA 92: 3137-3141; Deshane et al., 1994, Gene Ther. 1: 332-337; Marasco et al., 1998 Human Gene Ther 9: 1627-42; Shaheen et al., 1996 J. Virol. 70: 3392-400; Werge, T. M. et al., 1990, FEBS Letters 274:193-198; Carlson, J.R. 1993 Proc. Natl. Acad. Sci. USA 90:7427-7428; Biocca, S. et al., 1994, Bio/Technology 12: 396-399; Chen, S-Y. et al., 1994, Human Gene Therapy 5:595-601; Duan, L et al., 1994, Proc. Natl. Acad. Sci. USA 91:5075-5079; Chen, S-Y. et al., 1994, Proc. Natl. Acad. Sci. USA 91:5932-5936; Beerli, R.R. et al., 1994, J. Biol. Chem. 269:23931-23936; Mhashilkar, A.M. et al., 1995, EMBO J. 14:1542-1551; PCT Publication No. WO 94/02610 by Marasco et al.; and PCT Publication No. WO 95/03832 by Duan et al.).

To prepare an intracellular antibody expression vector, the cDNA encoding the antibody light and heavy chains specific for the target protein of interest are isolated, typically from a hybridoma that secretes a monoclonal antibody specific for the marker. Hybridomas secreting anti-marker monoclonal antibodies, or recombinant monoclonal antibodies, can be prepared using methods known in the art. Once a monoclonal antibody specific for the marker protein is identified (e.g., either a hybridoma-derived monoclonal antibody or a recombinant antibody from a combinatorial library), DNAs encoding the light and heavy chains of the monoclonal antibody are isolated by standard molecular biology techniques. For hybridoma derived antibodies, light and heavy chain cDNAs can be obtained, for example, by PCR amplification or cDNA library screening. For recombinant antibodies, such as from a phage display library, cDNA encoding the light and heavy chains can be recovered from the display package (e.g., phage) isolated during the library screening process and the nucleotide sequences of antibody light and heavy chain genes are determined. For example, many such sequences are disclosed in Kabat, E. A., et al. (1991) Sequences of Proteins of Immunological Interest, Fifth Edition, U.S. Department of Health and Human Services, NIH Publication No. 91-3242 and in the "Vbase" human germline sequence database. Once obtained, the antibody light and heavy chain sequences are cloned into a recombinant expression vector using standard methods.

For cytoplasmic expression of the light and heavy chains, the nucleotide sequences encoding the hydrophobic leaders of the light and heavy chains are removed. An intracellular antibody expression vector can encode an intracellular antibody in one of several different forms. For example, in one aspect, the vector encodes full-length antibody light and heavy chains such that a full-length antibody is expressed intracellularly. In another aspect, the vector encodes a full-length light chain but only the VH/CH1 region of the heavy chain such that a Fab fragment is expressed intracellularly. In another aspect, the vector encodes a single chain antibody (scFv) wherein the variable regions of the light and heavy chains are linked by a flexible peptide linker [e.g., (Gly₄Ser)₃ and expressed as a single chain molecule. To inhibit marker activity in a cell, the expression vector encoding the intracellular antibody is introduced into the cell by standard transfection methods, as discussed hereinbefore.

Once antibodies are obtained, they may be tested for activity, for example via ELISA.

The antibody of some aspects of the disclosure is used for therapeutic purposes, e.g., the antibody which is used as a TNF-alpha inhibitor.

Additionally or alternatively, several detection methods (e.g., protein detection methods) which are encompassed by some aspects of the disclosure employ the use of antibodies (e.g., antibodies for diagnostic, identification and/or classification purposes).

According some aspects of the disclosure, the antibody is conjugated to a functional moiety (also referred to as an "immunoconjugate") such as a detectable or a therapeutic moiety. The immunoconjugate molecule can be an isolated molecule such as a soluble and/or a synthetic molecule.

Various types of detectable or reporter moieties may be conjugated to the antibody of the disclosure. These include, but not are limited to, a radioactive isotope (such as ^{[125]}iodine), a phosphorescent chemical, a chemiluminescent chemical, a fluorescent chemical (fluorophore), an enzyme, a fluorescent polypeptide, an affinity tag, and molecules (contrast agents) detectable by Positron Emission Tomography (PET) or Magnetic Resonance Imaging (MRI).

Examples of suitable fluorophores include, but are not limited to, phycoerythrin (PE), fluorescein isothiocyanate (FITC), Cy-chrome, rhodamine, green fluorescent protein (GFP), blue fluorescent protein (BFP), Texas red, PE-Cy5, and the like. For additional guidance regarding fluorophore selection, methods of linking fluorophores to various types of molecules see Richard P. Haugland, "Molecular Probes: Handbook of Fluorescent Probes and Research Chemicals 1992-1994", 5th ed., Molecular Probes, Inc. (1994); U.S. Pat. No. 6,037,137 to Oncoimmunin Inc.; Hermanson, "Bioconjugate Techniques", Academic Press New York, N.Y. (1995); Kay M. et al., 1995. Biochemistry 34:293; Stubbs et al., 1996. Biochemistry 35:937; Gakamsky D. et al., "Evaluating Receptor Stoichiometry by Fluorescence Resonance Energy Transfer," in "Receptors: A Practical Approach," 2nd ed., Stanford C. and Horton R. (eds.), Oxford University Press, UK. (2001); U.S. Pat. No. 6,350,466 to Targesome, Inc.]. Fluorescence detection methods which can be used to detect the antibody when conjugated to a fluorescent detectable moiety include, for example, fluorescence activated flow cytometry (FACS), immunofluorescence confocal microscopy, fluorescence *in-situ* hybridization (FISH) and fluorescence resonance energy transfer (FRET).

Numerous types of enzymes may be attached to the antibody of the disclosure [e.g., horseradish peroxidase (HPR), beta-galactosidase, and alkaline phosphatase (AP)] and detection of enzyme-conjugated antibodies can be performed using ELISA (e.g., in solution), enzyme-linked immunohistochemical assay (e.g., in a fixed tissue), enzyme-linked chemiluminescence assay (e.g., in an electrophoretically separated protein mixture) or other methods known in the art [see e.g., Khatkhatay MI. and Desai M., 1999. J Immunoassay 20:151-83; Wisdom GB., 1994. Methods Mol Biol. 32:433-40; Ishikawa E. et al., 1983. J Immunoassay 4:209-327; Oellerich M., 1980. J Clin Chem Clin Biochem. 18:197-208; Schuurs AH. and van Weemen BK., 1980. J Immunoassay 1:229-49).

The affinity tag (or a member of a binding pair) can be an antigen identifiable by a corresponding antibody [e.g., digoxigenin (DIG) which is identified by an anti-DIG antibody) or a molecule having a high affinity towards the tag [e.g., streptavidin and biotin]. The antibody or the molecule which binds the affinity tag can be fluorescently labeled or conjugated to enzyme as described above.

Various methods, widely practiced in the art, may be employed to attach a streptavidin or biotin molecule to the antibody of the disclosure. For example, a biotin molecule may be attached to the antibody of the disclosure via the recognition sequence of a biotin protein ligase (e.g., BirA) as described in the Examples section which follows and in Denkberg, G. et al., 2000. Eur. J. Immunol. 30:3522-3532.

Alternatively, a streptavidin molecule may be attached to an antibody fragment, such as a single chain Fv, essentially as described in Cloutier SM. et al., 2000. Molecular Immunology 37:1067-1077; Dubel S. et al., 1995. J Immunol Methods 178:201; Huston JS. et al., 1991. Methods in Enzymology 203:46; Kipriyanov SM. et al., 1995. Hum Antibodies Hybridomas 6:93; Kipriyanov SM. et al., 1996. Protein Engineering 9:203; Pearce LA. et al., 1997. Biochem Molec Biol Intl 42:1179-1188).

Functional moieties, such as fluorophores, conjugated to streptavidin are commercially available from essentially all major suppliers of immunofluorescence flow cytometry reagents (for example, Pharmingen or Becton-Dickinson).

According to some aspects of the disclosure, biotin conjugated antibodies are bound to a streptavidin molecule to form a multivalent composition (e.g., a dimmer or tetramer form of the antibody).

Table 5 provides non-limiting examples of identifiable moieties which can be conjugated to the antibody of the disclosure.

### Table 5

**Table 5.**

| ***Identifiable Moiety*** | ***Amino Acid sequence (GenBank Accession No.)*** | ***SEQ ID NO:*** | ***Nucleic Acid sequence (GenBank Accession No.)*** | ***SEQ ID NO:*** |
|---|---|---|---|---|
| Green Fluorescent protein | AAL33912 | 69 | AF435427 | 78 |
| Alkaline phosphatase | AAK73766 | 70 | AY042185 | 79 |
| Peroxidase | CAA00083 | 71 | A00740 | 80 |
| Histidine tag | Amino acids 264-269 of GenBank Accession No. AAK09208 | 72 | Nucleotides 790-807 of GenBank Accession No. AF329457 | 81 |
| Myc tag | Amino acids 273-283 of GenBank Accession No. AAK09208 | 73 | Nucleotides 817-849 of GenBank Accession No. AF329457 | 82 |
| Biotin lygase tag | LHHILDAQ**K**M VWNHR / | 74 | - | - |
| orange fluorescent protein | AAL33917 | 75 | AF435432 | 83 |
| Beta galactosidase | ACH42114 | 76 | EU626139 | 84 |
| Streptavidin | AAM49066 | 77 | AF283893 | 16 |

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various aspects of this disclosure may be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

When reference is made to particular sequence listings, such reference is to be understood to also encompass sequences that substantially correspond to its complementary sequence as including minor sequence variations, resulting from, e.g., sequencing errors, cloning errors, or other alterations resulting in base substitution, base deletion or base addition, provided that the frequency of such variations is less than 1 in 50 nucleotides, alternatively, less than 1 in 100 nucleotides, alternatively, less than 1 in 200 nucleotides, alternatively, less than 1 in 500 nucleotides, alternatively, less than 1 in 1000 nucleotides, alternatively, less than 1 in 5,000 nucleotides, alternatively, less than 1 in 10,000 nucleotides.

It is understood that any Sequence Identification Number (SEQ ID NO) disclosed in the instant application can refer to either a DNA sequence or a RNA sequence, depending on the context where that SEQ ID NO is mentioned, even if that SEQ ID NO is expressed only in a DNA sequence format or a RNA sequence format. For example, SEQ ID NO: 37 is expressed in a DNA sequence format (e.g., reciting T for thymine), but it can refer to either a DNA sequence that corresponds to a CD68 nucleic acid sequence, or the RNA sequence of an RNA molecule nucleic acid sequence. Similarly, though some sequences are expressed in a RNA sequence format (*e.g.,* reciting U for uracil), depending on the actual type of molecule being described, it can refer to either the sequence of a RNA molecule comprising a dsRNA, or the sequence of a DNA molecule that corresponds to the RNA sequence shown. In any event, both DNA and RNA molecules having the sequences disclosed with any substitutes are envisioned.

It is appreciated that certain features of the disclosure, which are, for clarity, described in the context of separate aspects, may also be provided in combination in a single aspect. Conversely, various features of the disclosure, which are, for brevity, described in the context of a single aspect, may also be provided separately or in any suitable subcombination or as suitable in any other described aspect of the disclosure. Certain features described in the context of various aspects are not to be considered essential features of those aspects, unless the aspect is inoperative without those elements.

Various aspects and aspects of the present disclosure as delineated hereinabove and as claimed in the claims section below find experimental support in the following examples.

### EXAMPLES

Reference is now made to the following examples, which together with the above descriptions illustrate some aspects of the disclosure in a non limiting fashion.

Generally, the nomenclature used herein and the laboratory procedures utilized in the present disclosure include molecular, biochemical, microbiological and recombinant DNA techniques. Such techniques are thoroughly explained in the literature. See, for example, "Molecular Cloning: A laboratory Manual" Sambrook et al., (1989); "Current Protocols in Molecular Biology" Volumes I-III Ausubel, R. M., ed. (1994); Ausubel et al., "Current Protocols in Molecular Biology", John Wiley and Sons, Baltimore, Maryland (1989); Perbal, "A Practical Guide to Molecular Cloning", John Wiley & Sons, New York (1988); Watson et al., "Recombinant DNA", Scientific American Books, New York; Birren et al. (eds) "Genome Analysis: A Laboratory Manual Series", Vols. 1-4, Cold Spring Harbor Laboratory Press, New York (1998); methodologies as set forth in U.S. Pat. Nos. 4,666,828; 4,683,202; 4,801,531; 5,192,659 and 5,272,057; "Cell Biology: A Laboratory Handbook", Volumes I-III Cellis, J. E., ed. (1994); "Current Protocols in Immunology" Volumes I-III Coligan J. E., ed. (1994); Stites et al. (eds), "Basic and Clinical Immunology" (8th Edition), Appleton & Lange, Norwalk, CT (1994); Mishell and Shiigi (eds), "Selected Methods in Cellular Immunology", W. H. Freeman and Co., New York (1980); available immunoassays are extensively described in the patent and scientific literature, see, for example, U.S. Pat. Nos. 3,791,932; 3,839,153; 3,850,752; 3,850,578; 3,853,987; 3,867,517; 3,879,262; 3,901,654; 3,935,074; 3,984,533; 3,996,345; 4,034,074; 4,098,876; 4,879,219; 5,011,771 and 5,281,521; "Oligonucleotide Synthesis" Gait, M. J., ed. (1984); "Nucleic Acid Hybridization" Hames, B. D., and Higgins S. J., eds. (1985); "Transcription and Translation" Hames, B. D., and Higgins S. J., Eds. (1984); "Animal Cell Culture" Freshney, R. I., ed. (1986); "Immobilized Cells and Enzymes" IRL Press, (1986); "A Practical Guide to Molecular Cloning" Perbal, B., (1984) and "Methods in Enzymology" Vol. 1-317, Academic Press; "PCR Protocols: A Guide To Methods And Applications", Academic Press, San Diego, CA (1990); Marshak et al., "Strategies for Protein Purification and Characterization - A Laboratory Course Manual" CSHL Press (1 996). Other general references are provided throughout this document. The procedures therein are believed to be well known in the art and are provided for the convenience of the reader.

### GENERAL MATERIALS AND EXPERIMENTAL METHODS

All analyses were performed in the R statistical software (www(dot)r-project(dot)org), using additional packages available from the Bioconductor project (www(dot)bioconductor(dot)org).

*Cell type expression pattern of predictive gene signatures -* CEL files of sorted cell type samples from IRIS (GSE22886, (34)) and the Human body index (GSE7307) were downloaded from GEO, and normalized separately using frma. In GSE7307, the present inventors then extracted the profiles from all immune cells (10 profiles from monocyte, T cell and B cell lineages) and colon tissues (2 profiles). The present inventors then created a combined gene expression matrix, correcting for batch (dataset) effect using Combat. Previously reported gene signatures were collected as lists of gene symbols from their associated original publications or patent application as detailed in Table 6. Symbols were mapped to the genes assayed on platform HGU133A using Bioconductor symbol and alias mappings available in the hgu133plus2.db annotation package.

*Gene expression datasets* - Normalized gene expression data for datasets GSE12251, GSE14580 and GSE16879 were downloaded from GEO using the GEOquery package. In each dataset the present inventors selected the relevant subset of baseline samples as described in Table 7, each forming a separate discovery cohort.

*Deconvolution analysis -* Given the total gene expression profile of a sample, gene expression deconvolution methods use prior knowledge obtained from sorted cells, e.g., as basis expression profiles or marker gene lists, to estimate the respective contribution of distinct cell types (18). In this work the present inventors used the basis signature and method developed in (26), which returns estimates for 17 immune cell types. The present inventors used the implementation available from the CellMix package (35).

***Meta-analysis of cell type proportions*** - Cell type proportion differences estimated in multiple cohorts were integrated in a meta-analysis. First cell type proportions were log2-transformed and compared between responders and non-responders within each cohort using Wilcoxon rank sum test. Then nominal p-values were combined using Fisher combined probability test, which was corrected using Benjamini and Hochberg FDR correction. Cell types having nominal p-values <= 0.05 in at least 2 cohorts and a combined FDR <= 0.01 were selected for further analysis.

***Patients in the validation cohort** -* Archival slides from 23 patients with an established diagnosis of IBD (13 Crohn's Disease, 7 Ulcerative Colitis, 3 IBDU) from the gastroenterology department of Rambam Health Care Campus were included in this analysis. Responsiveness to anti-TNF treatment was assessed based on parameters such as: abdominal pain, bowel consistency and frequency, blood in stool, nausea/vomiting, constitutional symptoms, extracolonic manifestations, presence of abdominal mass, blood inflammatory markers, and colonic biopsy results. Patients were classified retrospectively as anti-TNF responders when they experienced clinical and/or mucosal improvement within 8 weeks after treatment initiation. Other data collected includes age, gender, disease state when biopsy was taken, disease-related surgery, co-morbidities and medications. A summary of these data is shown in Table 6.

***Biopsy collection in the validation cohort** -* Colonic biopsies were collected from the patients during flexible sigmoidoscopy or full colonoscopy before their first anti-TNF treatment. Biopsies were taken from inflamed and/or uninflamed areas of the intestine ascending/transverse/descending colon and placed into formalin.

***Immuno-histochemistry Quantification*** - Formalin-fixed slides of paraffin-embedded colon tissues, sectioned at 4 µm, were immunostained for the expression of plasma cells (CD138+). The slides were deparaffinized in Xylene (twice, 3 minutes each time) and rehydrated in gradually decreasing concentrations of EtOH (100% EtOH x2, 95%, 85%, 70% and running water). 0.01 M sodium citrate buffer pH 6.0 was used to heat-induced epitope retrieval before incubation with antibody. Slides were immersed in the buffer and heated in a microwave for 20 minutes. The slides were rinsed in cool running water, washed in PBS with 0.1% Tween solution, and blocked in 10% goat serum. Then, they were incubated with CD138 primary monoclonal antibody in 4°C overnight (obtained from Serotec, clone B-A38, dilution 1:250). For detection, the Polink-1 HRP Broad Spectrum DAB Detection Kit (GBI labs) was used.

***Staining data analysis*** - Two scoring methods were used for the analysis of stain biopsies: Slides were coded and interpreted blindly by a specialist pathologist. A "plasma cell abundance" subjective score between 0-3 was determined by the pathologist (minimal amount of plasma cells was scored as "0", the highest abundance which have seen within all slides was scored as "3"), and the tissues were scored one by one.

Slides were scanned in automatic digital slide scanner, and evaluated in Image-Pro Plus 6.0 software. 4 high stained fields were chosen randomly in each patient slide, and Brown color of DAB CD138+ cells was tested. Each field CD138+ staining value was divided by the same field whole tissue staining. Average of those 4 fields is presented.

### Clinical evaluation of patients:

The clinical state of the patients was evaluated using the Harvey Bradshaw Index (HBI) at each visit. Clinical state was defined as either remission, mild disease, moderate disease, or severe disease based on the HBI score definition. Subjects were defined as clinical responders if clinical state improved or remained at remission during all visits.

***Biomarker response*** - evaluated biomarkers were serum C-reactive protein (CRP) and fecal calprotectin. Previous studies have shown that fecal calprotectin levels are highly correlated with disease severity. Due to low subject compliance with handling fecal material, the fecal calprotectin could not be obtained on all visits from all subjects. The determination of responders or non-responders was performed using the following guidelines:
(1) Subjects who had at least 2 fecal calprotectin samples taken at least 1 week apart were considered responders when at least a 50% reduction in levels was demonstrated in the second sample retrieved from the feces of the subject.
(2) Subjects who stably remained at normal levels of fecal calprotectin (≤ 50 mg/gram of feces) at all visits, regardless of serum CRP were considered responders.
(3) Subjects with less than 2 samples of fecal calprotectin were considered responders when demonstrated at least a 50% reduction in serum CRP levels in a second blood sample taken at least a week after the first blood sample.
(4) Subjects who exhibited normal levels of CRP (≤ 5 mg/dl) at all visits were considered responders.

**Steroid dependence** - The persistent need of concurrent steroid therapy is a valuable marker of disease state and of response to therapy. Subjects, who were receiving steroid therapy at the clinic visit at the 14^{th} week of treatment ("14-week") were considered non-responders.

Immunogenic status - Subjects who had measurable serum antibodies to Infliximab at their week 14-week visit were considered non-responders.

Study response algorithm - The present inventors have formulated a decision algorithm to conclude whether a subject is responsive or not to therapy. The algorithm is mainly based on the primary gastroenterologist following the subject. For each subject on the 14-week visit the physician, after reviewing the subjects' records, decides whether the subject responded to therapy, failed or if it is still indeterminate. For the latter (indeterminate), a decision tree is performed with the following steps: a definition of failure is set when steroid treatment is given at 14-week visit. If no steroids are given the next step is to test the biomarker dynamics. A substantial reduction in fecal calprotectin is defined as response. If fecal calprotectin is not available, a reduction in serum CRP (as defined previously) is considered a response to treatment. For subjects who are not steroid-dependent and show no substantial biomarker dynamics, a physician decision on week 26 is made to determine the response status.

### EXAMPLE 1

### PREVIOUSLY REPORTED GENE SIGNATURES INDICATE IMMUNE-DRIVEN SIGNAL

The present inventors have hypothesized that there is a baseline immune cellular signature of response to anti-TNF therapy, and accordingly, expected that at least part of previously predictive gene signatures detected by previous studies was indeed capturing an immune-driven signal, through genes that are more highly expressed by some immune cell subsets. To test this hypothesis, genes belonging to 7 gene signatures (*i.e.* gene sets) that were identified in studies of baseline response to anti-TNF in biopsies (6) or blood (1) (Table 6 below) have been considered. Most biopsy signatures (UC_A, UC _B, UC_AB, CDc and UC_B_knn) were originally defined based on the comparison of gene expression profiles between responders and non-responders to Infliximab treatment in UC and CD cohorts generated by two studies *(4, 5)*. Signatures UC_A and UC_B were on two independent cohorts of UC patients (cohort A and B) from the top 20 differentially expressed genes; UC_AB was defined as the overlap between all differentially expressed genes in these two studies (53 genes) *(4)*; Signature UC_B_knn was derived from UC cohort B using a different methodology based on k-nearest-neighbor classifier *(20)*; The IRRAT signature (Injury-Repair Response Associated Transcripts) was defined in a kidney transplant study *(21)*, but was subsequently found to correlate well with anti-TNF response at baseline in one of the above UC cohort *(22)*; Signatures CDc and CD_blood were identified in CD patients from colon biopsies *(5)* and blood samples (PBMCs) respectively, the later using an iterative multivariate classification algorithm *(13)*.

### Table 6

**Table 6: Previously proposed gene signatures of baseline response to anti-TNF. N: number of genes in each signature.**

| | ***N*** | ***Datasets*** | ***Disease* / *Tissue*** | ***Reference*** |
|---|---|---|---|---|
| UC_A | 20 | GSE14580 | UC / Colon | Arijs et al. (2009) Gut 2009;58(12):1612-9. |
| | | | | Pubmed: 19700435 |
| UC_B | 20 | GSE12251 | UC / Colon | Arijs et al. (2009) Gut 2009;58(12):1612-9. |
| | | | | Pubmed: 19700435 |
| UC_B_knn | 19 | GSE12251 | UC / Colon | www(dot)faqs(dot)org/patents/app/2010 0069256 |
| UC_AB | 53 | GSE12251,GSE1458 0 | UC / Colon | Arijs et al. (2009) Gut 2009;58(12):1612-9. |
| | | | | Pubmed: 19700435 |
| CDc | 20 | GSE16879 | CD / Colon | Arijs et al. Inflamm Bowe Dis.2010;16(12):2090-8. |
| | | | | Pubmed: 20848504 |
| IRRAT | 29 | GSE14580 | UC / Colon | Halloran et al. Inflamm Bowel Dis 2014;20(12):2353-63 |
| | | | | Pubmed: 25397893 |
| CD_Blood | 23 | IBD | Blood | Mesko et al. Genome Med. 2013;5(6):59 Pubmed: 23809696 |

The present inventors looked at the expression of all signature genes across a variety of sorted immune cell subsets and bulk colon tissue samples obtained from two public datasets of sorted cell expression profiles (Figure 2A, see Methods). Cell types from common hematopoietic lineage clustered together, mainly within B cells, T cells and Monocytes, while genes clustered in distinct blocks according to these lineages.

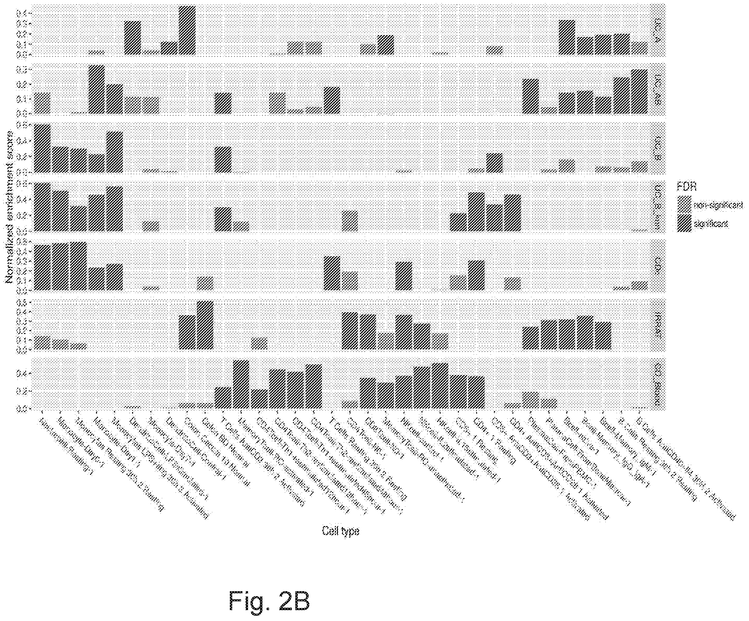

The association between each signature and each cell type was analyzed using a single sample enrichment analysis with GSVA *(23)* (Figure 2B). Genes in the IRRAT signature were associated to subsets from the B cell lineage and neutrophils, while genes from CD_blood were associated to T cells. All other signatures were associated with monocytes. Hence overall most genes were more highly expressed by some immune cell subsets, rather than by colon tissues.

Very few genes were more highly expressed in colon tissues, and of those, most were also highly expressed in some other immune cell subset, mainly from the B cell lineage and neutrophils. This could indicate the presence of resident or infiltrating leukocyte populations within these tissues.

### EXAMPLE 2

### META-ANALYSIS IDENTIFIES CONSISTENT CELL TYPE PROPORTION DIFFERENCES

Meta-analysis of gene expression datasets has shown its ability to extract robust disease gene-based signatures by leveraging the biological and technical heterogeneity in data obtained from multiple sources to select genes that consistent and reproducible differences between two conditions *(19, 24)*. This approach essentially consists of two steps: (1) a discovery phase that identifies features that are consistently different between two conditions in a set of discovery cohorts; and (2) a validation phase that assesses the ability of the selected features in classifying samples from an independent dataset. Here this methodology was applied in a novel way, by combining it with computational deconvolution techniques, to find robust cellular signatures that are predictive of anti-TNF response pre-treatment.

First the present inventors looked in the GEO database *(25)* for datasets of biopsies from IBD patients that were naive to anti-TNF therapy, and selected those for which both pre-treatment expression profiles and response status were available (GSE12251, GSE14580 and GSE16879).

Table 7 summarizes each dataset experimental design and relevant associated clinical data.

### Table 7

**Table 7. Summary of the datasets and biopsy samples used in the meta-analysis. * Responders (R) / Non-responders (NR).**

| Discovery cohorts | | |
|---|---|---|
| Dataset | Cohort | Samples* |
| GSE14580 | UC cohort A, colon biopsies, pre-treatment | 8R/16NR |
| GSE12251 | UC cohort B, colon biopsies, pre-treatment | 12 R / 10 NR |
| GSE16879 | CD colon biopsies, pre-treatment | 12R/7NR |

| Validation | | |
|---|---|---|
| Rambam Hospital | UC/CD/IBDU colon and ileum biopsies | 9 R / 11 NR |

These datasets contain biopsy gene expression profiles generated from 2 cohorts of UC patients (Cohort UC-A and UC-B in GSE14580 and GSE12251 respectively), and 1 cohort of CD (CD-C) patients (GSE16879). They were designed for the discovery of genes that can predict, at baseline, if a patient is likely to respond to an anti-TNF treatment (Infliximab), and, indeed, resulted in most of the previously proposed gene signatures analyzed hereinabove *(4, 5)*. As a matter of fact, dataset GSE16879 contains profiles from other samples such as ileum CD biopsies, for which the response criterion was not as stringent as for the other samples, and consequently did not lead to any signature of response in the original study *(5)*; it also includes pre-treatment UC samples that are part of dataset GSE14580, which were used therein, as well as post-treatment profiles from the same CD and UC patients (8 weeks after therapy initiation). In the baseline analysis, however, only the pre-treatment CD colon samples were used, all other samples were not used. Hence in the following, each cohort is referred using its corresponding GEO id.

Computational gene expression deconvolution methods can estimate the proportions of constituting cell types directly from heterogeneous samples *(18)*. This is typically achieved using either sets of marker genes that are known to be expressed in a cell type-specific manner, or within a linear regression framework that jointly estimates all cell subset proportions -- on each sample separately -- from a reference compendium of sorted cell gene expression profiles *(18)*. Using such a regression-based method *(26)*, the present inventors estimated the proportion of 17 immune cell types in each sample, including most major cell subsets such as neutrophils, monocytes, B cell or T cell subpopulations in resting or activated state. Then, the estimated proportions of each cell type was compared between responders and non-responders, to identify candidate immune driver(s) of response. For robustness, the non-parametric Wilcoxon rank sum test was used, which is free of distributional assumption, and only cell types for which at least 75% of the samples had non-zero estimated proportions were considered. This analysis was performed initially in the CDc cohort (GSE16879), which revealed significant differences in activated monocytes and plasma cells, both showing higher proportions in non-responders (Figure 2A). This same cohort was previously used to show that a predictive signature of 20 genes derived from UC patients was also able to perfectly discriminate responders and non-responders CD patients (5) (Figure 3B). Having the estimated proportions of the two cell types that are the most associated with response enabled the present inventors to perform a second analysis to support the hypothesis of an immune based biomarkers of response. The total gene expression data was corrected for variation in activated monocytes and plasma cells, and the effect on the predictive power of the 20-genes signature was monitored. After correction, the classification accuracy dropped, suggesting that the gene signature indeed reflected, at least partially, a predictive variation in the proportions of these cell types (Figure 3C). Notably, correcting for each cell type individually also lowered the signature's predictive power but not as much as when correcting for both (Figures 7A and 7B). Next, to strengthen the cell - based biomarker prediction, the present inventors repeated the analysis within each discovery cohort (Figure 6). Significant differences were detected in activated monocytes which were lower in responders in all cohorts; plasma cells were lower in responders in 2 out 3 cohorts, including both UC and CD samples; finally, in either one of the cohorts, proportions of monocytes, activated dendritic cells, activated NK cells and CD8 T cells were higher in responders, while proportions of memory IgM B cells and neutrophils were higher in non-responders. Then, these differences were integrated across all cohorts in a meta-analysis, by combining p-values and selecting cell types that showed significant differences in at least 2 out of the 3 discovery cohorts (nominal p-value <= 0.05) and a combined FDR ≤ 0.01. This resulted in the selection of two cell subsets, activated monocytes and plasma cells, with responders having in both cases significantly lower proportions than non-responders (Figure 4). In term of training set prediction power, separate ROC analysis within each cohort resulted in high mean accuracies of 90.3% and 77.8% Area Under the Curve (AUC) for activated monocytes and plasma cell proportions respectively (Figure 8).

### Validation of cell signatures by staining in an independent set of biopsies

In order to validate these findings, the present inventors looked at an independent set of 20 IBD patients (11 responders, 9 non-responders to anti-TNF) for which paraffin embedded biopsies had been stored prior anti-TNF treatment initiation, as part of common standard patient monitoring protocol in IBD. The present inventors defined cell type abundance scores from the examination of immunostained slides, and assessed how their proportion could predict response to treatment via ROC curve and Area Under the Curve (AUC). Since macrophages and plasma cells were the present inventors' top hits, the present inventors set out to define a macrophage and plasma cells morphological abundance score (low/medium/high) based on visual identification by a pathologist. For macrophages, this did not discriminate well responders from non-responders (Figure 10), but plasma cells gave a clearly distinguishable differences. To test these findings, the present inventors stained for plasma cells (CD138+), and used two scoring strategies: first, a pathologist was asked to score the staining for low/medium/high abundance, while blind to the response status. Second, the present inventors used the proportions obtained by automated pixel quantitation averaged over multiple randomly chosen regions (see Methods). The pathologist and automated quantitation scores achieved 72.2% and 83.3% accuracy respectively (Figure 5A). Visually, non-responsive patients showed very clear increased staining for plasma cells compared to non-responsive patients (Figure 5B).

Tables 8A-B hereinbelow (Deconvolution basis signature), discloses raw data of the deconvolution estimation basis matrix. Table 9 herein below summarizes the results from the meta-analysis of the raw data.

### Table 8A

**Table 8A.**

| ***Deconvolution basis signature*** | | | | | | | | | | | |
|---|---|---|---|---|---|---|---|---|---|---|---|
| ***Adaptive immune cell subsets*** | | | | | | | | | | | |
| ***ENTREZ ID*** | ***Symbol*** | ***T CD4*** | ***T CD4 activated*** | ***T CD8*** | ***T CD8 activated*** | ***B cells*** | ***B activated*** | ***B aIgM*** | ***B Mem IgG*** | ***B Mem IgM*** | ***Plasma cells*** |
| | | 5292.9 | 104.6 | 191.29 | 41.553 | 671.33 | 125.9 | 78.485 | 1355.6 | 2167.6 | 575.7 |
| 83481 | EPPK1 | 605.1 | 57.129 | 19.282 | 23.457 | 19.076 | 22.296 | 18.152 | 51.534 | 45.244 | 24.699 |
| 678 | ZFP36L2 | 5213.9 | 625.69 | 860.42 | 267.09 | 1040.9 | 563.83 | 248.18 | 422.51 | 430.73 | 170.09 |
| | | 1710.6 | 222.72 | 147.83 | 79.293 | 319.04 | 236.36 | 156.19 | 134.89 | 262.19 | 128.64 |
| 4929 | NR4A2 | 1407.8 | 247.46 | 19.61 | 143.09 | 50.766 | 112.38 | 322.4 | 202.34 | 365.71 | 42.58 |
| | | 1079.4 | 104.49 | 189.22 | 154.5 | 57.445 | 81.581 | 162.01 | 48.368 | 116.49 | 31.034 |
| 26289 | AK5 | 1379.6 | 330.29 | 203.27 | 52.567 | 50.005 | 48.406 | 38.379 | 69.45 | 65.663 | 55.256 |
| 3707 | ITPKB | 9321.4 | 1863.7 | 1799 | 613.54 | 1615.5 | 2073.6 | 2786.8 | 231.11 | 151.04 | 294.67 |
| | | 6951 | 389.67 | 369.9 | 408.86 | 1149.1 | 388.4 | 630.63 | 1875.9 | 3071.5 | 448.05 |
| 9241 | NOG | 1205.8 | 216.29 | 175.73 | 61.715 | 163.99 | 62.575 | 71.163 | 153.18 | 210.55 | 226.19 |
| 3337 | DNAJB1 | 4361.3 | 980.92 | 842.13 | 919.73 | 580.86 | 779.84 | 629.17 | 342.02 | 318.99 | 888.9 |
| 2935 | GSPT1 | 3343.6 | 1173.4 | 661.92 | 751.8 | 737.37 | 768.24 | 920.58 | 513.34 | 503.68 | 565.05 |
| | | 14624 | 2674.3 | 1387.4 | 3255.3 | 5202 | 1429.3 | 2599.7 | 3060.4 | 3890.8 | 1513.1 |
| 4929 | NR4A2 | 1142.3 | 259.03 | 75.925 | 204.75 | 125.36 | 209.68 | 312.83 | 502.61 | 820.81 | 97.28 |
| 90139 | TSPAN18 | 1948.2 | 400.21 | 234 | 129.54 | 353.61 | 210.34 | 149.64 | 323.63 | 239.4 | 712.5 |
| 146330 | FBXL16 | 3811.3 | 1576.5 | 1403.5 | 697.89 | 732.34 | 672.19 | 494.3 | 833.77 | 550.58 | 1161 |
| 678 | ZFP36L2 | 3793.8 | 620.67 | 771.55 | 219.34 | 1023.5 | 486.97 | 268.09 | 366.91 | 415.21 | 172.63 |
| 112744 | IL17F | 75.661 | 9648.6 | 96.514 | 126.73 | 146.75 | 99.437 | 125.95 | 181.58 | 145.47 | 169.89 |
| 3605 | IL17A | 7.194 | 809.34 | 3.396 | 10.259 | 7.24 | 8.1 | 3.219 | 24.895 | 36.767 | 5.585 |
| 1493 | CTLA4 | 1152.6 | 5171.8 | 644.65 | 1132.7 | 400.95 | 331.91 | 319.33 | 599.21 | 486.96 | 540.88 |
| 1493 | CTLA4 | 456.17 | 1826.6 | 170.64 | 539.95 | 77.447 | 57.268 | 68.635 | 58.954 | 76.762 | 89.278 |
| 940 | CD28 | 484.12 | 1102.4 | 350.46 | 347.35 | 148.4 | 101.36 | 39.628 | 175.46 | 291.21 | 134.16 |
| 51339 | DACT1 | 276.97 | 664.71 | 99.844 | 95.204 | 207.63 | 228.89 | 116.55 | 212.18 | 274.44 | 115.61 |
| 50616 | IL22 | 195.27 | 1621.9 | 140.2 | 272.41 | 182.77 | 177 | 131.76 | 305.89 | 259.44 | 170.05 |
| 143686 | SESN3 | 848.53 | 1665.6 | 388.3 | 162.9 | 305.6 | 519.98 | 590.79 | 135.39 | 151.2 | 120.16 |
| 128553 | TSHZ2 | 669.63 | 1663.3 | 288.41 | 312.36 | 171.51 | 286.96 | 117.25 | 355.25 | 272.75 | 201.94 |
| 145864 | HAPLN3 | 1946.6 | 8728.1 | 1101.6 | 2305.8 | 594.52 | 1180.4 | 388.18 | 558.27 | 536.28 | 992.28 |
| 30812 | SOX8 | 298.04 | 1025 | 19.781 | 21.533 | 30.567 | 26.109 | 23.557 | 28.412 | 27.116 | 41.972 |
| 940 | CD28 | 424.1 | 927.04 | 284.99 | 249.87 | 32.017 | 33.209 | 17.453 | 61.339 | 66.092 | 45.075 |
| 1493 | CTLA4 | 829.63 | 2074.9 | 360.49 | 772.02 | 172.99 | 225.83 | 82.15 | 277.45 | 353.17 | 308.49 |
| 10320 | IKZF1 | 585.01 | 1251.5 | 289.46 | 343.31 | 289.61 | 355.95 | 186.75 | 251.73 | 257.34 | 202.66 |
| 29968 | PSAT1 | 346.27 | 2214.3 | 394.62 | 805.9 | 320.85 | 337.81 | 736.12 | 429.65 | 309.66 | 1025.6 |
| 3578 | IL9 | 97.274 | 1343.5 | 49.426 | 410.4 | 83.163 | 57.007 | 22.289 | 145.32 | 101.28 | 54.427 |
| 128553 | TSHZ2 | 305.87 | 591.41 | 112.12 | 137.34 | 154.73 | 110.94 | 63.719 | 214.32 | 219.95 | 234.12 |
| 926 | CD8B | 41.855 | 32.983 | 1489.1 | 122.68 | 37.742 | 27.152 | 8.683 | 93.873 | 74.936 | 50.758 |
| 54674 | LRRN3 | 735.62 | 503.37 | 6792.8 | 1198.6 | 62.117 | 40.136 | 28.776 | 157.23 | 118.26 | 33.943 |
| 925 | CD8A | 310.06 | 405.99 | 9957.8 | 3315.8 | 611.63 | 460.91 | 106.98 | 363.04 | 243.73 | 125.54 |
| 926 | CD8B | 138.63 | 177.5 | 5811.1 | 2622.3 | 115.71 | 130.58 | 54.383 | 281.08 | 335.21 | 118.48 |
| 54674 | LRRN3 | 706.71 | 581.81 | 8644.5 | 2115.9 | 159.72 | 143.34 | 90.669 | 332.35 | 344.48 | 173.99 |
| 51676 | ASB2 | 79.337 | 112.51 | 2639.9 | 927.9 | 38.727 | 46.681 | 31.775 | 53.498 | 43.509 | 219.16 |
| 9666 | DZIP3 | 63.449 | 15.003 | 194.1 | 12.941 | 59.71 | 26.541 | 25.563 | 37.349 | 36.696 | 17.2 |
| 10730 | YME1L1 | 246.02 | 176.44 | 613.76 | 234.81 | 24.22 | 30.823 | 24.564 | 39.521 | 42.166 | 31.209 |
| 85315 | PAQR8 | 786.88 | 1044.9 | 2261.4 | 883.48 | 460.26 | 520.17 | 258.38 | 424.19 | 344.92 | 557.11 |
| 9402 | GRAP2 | 102.7 | 186.89 | 569.46 | 181.94 | 24.59 | 16.219 | 12.373 | 19.32 | 20.428 | 13.39 |
| 2833 | CXCR3 | 232.2 | 123.87 | 1592.3 | 282.94 | 73.401 | 52.68 | 25.821 | 101.19 | 79.587 | 242.77 |
| 3820 | KLRB1 | 237.41 | 181.43 | 750.95 | 276.46 | 270.68 | 110.13 | 115.56 | 162.12 | 76.456 | 206.92 |
| 4676 | NAP1L4 | 1095.9 | 1054.6 | 1961.3 | 1146 | 851.07 | 858.5 | 642.55 | 569.35 | 587.16 | 796.58 |
| 1731 | 1-Sep | 1853.2 | 1078 | 3456.8 | 1540.5 | 1236.1 | 1119.3 | 944.65 | 371.66 | 442.15 | 1079.7 |
| 814 | CAMK4 | 1278.2 | 978.5 | 2274.6 | 1265.9 | 238.29 | 296.08 | 68.922 | 188 | 231.35 | 301.28 |
| 57124 | CD248 | 366.65 | 216.83 | 1028.2 | 288.42 | 329.16 | 282.39 | 195.08 | 324.75 | 255.77 | 252.92 |
| | | 116.38 | 108.86 | 526.68 | 257.03 | 74.15 | 61.25 | 39.666 | 104.62 | 117.43 | 122.23 |
| 283869 | NPW | 5.177 | 5.275 | 4.551 | 539.25 | 6.061 | 5.692 | 446.58 | 11.427 | 16.884 | 12.242 |
| 199953 | TMEM201 | 192.86 | 235.07 | 220.53 | 3249.7 | 173.54 | 170.17 | 95.56 | 141.98 | 147.22 | 290.42 |
| 399694 | SHC4 | 59.433 | 60.769 | 40.265 | 570.24 | 33.133 | 62.257 | 34.752 | 92.555 | 87.079 | 47.668 |
| 3976 | LIF | 31.201 | 1084.5 | 36.214 | 5823.8 | 25.66 | 21.031 | 19.177 | 49.072 | 46.371 | 25.724 |
| 23176 | 8-Sep | 11.013 | 20.212 | 16.933 | 126.6 | 10.758 | 11.885 | 13.505 | 25.628 | 24.617 | 18.216 |
| 990 | CDC6 | 93.088 | 194.93 | 220.38 | 1223.1 | 107.62 | 112.04 | 83.857 | 174.97 | 175.32 | 310.89 |
| 51010 | EXOSC3 | 118.67 | 789.6 | 738.75 | 2638.3 | 174.02 | 567.47 | 822.37 | 48.067 | 79.427 | 299.82 |
| 1021 | CDK6 | 3018.2 | 4621.4 | 3289.6 | 19509 | 2481.6 | 2276.6 | 1490.8 | 493.55 | 258.8 | 3295 |
| 51293 | CD320 | 144.21 | 273.65 | 232.18 | 1229.6 | 68.268 | 144.65 | 283.68 | 59.468 | 53.224 | 315.2 |
| 1503 | CTPS1 | 233.48 | 828.46 | 608.83 | 2722.2 | 268.05 | 532.91 | 994.27 | 373.66 | 397.97 | 386.96 |
| 84319 | CMSS1 | 844.34 | 1873.3 | 1306.2 | 6709.1 | 935.86 | 1139.8 | 3038.4 | 411.69 | 346.84 | 1622.2 |
| 10622 | POLR3G | 111.28 | 129.25 | 80.188 | 499.74 | 110.15 | 90.279 | 191.06 | 105.25 | 95.599 | 122.2 |
| 199953 | TMEM201 | 169.99 | 261.11 | 249.09 | 1122.7 | 140.65 | 160.81 | 171.54 | 123.62 | 129.9 | 114.47 |
| 1021 | CDK6 | 1750.7 | 2549.3 | 1791.3 | 9645 | 1502.4 | 1408.9 | 897.3 | 752.27 | 632.6 | 2131.3 |
| 1841 | DTYMK | 108.09 | 195.01 | 278.64 | 1007.2 | 163.8 | 160.07 | 273.64 | 133.27 | 153.84 | 292.14 |
| 23464 | GCAT | 220.973 | 14.22 | 28.833 | 191.85 | 16.681 | 11.739 | 14.28 | 22.541 | 21.448 | 16.567 |
| 3336 | HSPE1 | 844.06 | 2476.6 | 1665.5 | 7532.5 | 669.89 | 1505.3 | 2903.3 | 846.93 | 783.39 | 1572.6 |
| | | 59.386 | 43.757 | 62.947 | 48.205 | 1755 | 162.4 | 482.29 | 413.11 | 121.42 | 128.14 |
| 971 | CD72 | 58.037 | 36.506 | 120.52 | 81.1 | 2131.4 | 429.63 | 938.19 | 245.96 | 573.81 | 17.596 |
| 933 | CD22 | 15.819 | 9.314 | 10.67 | 9.456 | 4220.4 | 899.99 | 1028.4 | 411.99 | 546.56 | 11.084 |
| | | 163.92 | 86.114 | 48.873 | 16.798 | 1334.1 | 244.78 | 244.54 | 171.12 | 212.56 | 22.94 |
| 6328 | SCN3A | 9.889 | 14.549 | 3.845 | 5.263 | 722.63 | 159.39 | 75.757 | 124.23 | 81.037 | 22.318 |
| 84518 | CNFN | 17.525 | 20.474 | 15.638 | 15.914 | 223.47 | 42.35 | 25.314 | 47.823 | 161.18 | 31.814 |
| 8115 | TCL1A | 413.37 | 244.87 | 53.831 | 83.168 | 11712 | 2673.8 | 2889.4 | 498.98 | 831.45 | 293.26 |
| 29802 | VPREB3 | 155.11 | 68.624 | 42.995 | 57.886 | 2850.9 | 755.75 | 264.61 | 1466.2 | 1287.6 | 178.78 |
| 79856 | SNX22 | 47.638 | 48.233 | 40.53 | 35.855 | 607.56 | 124.46 | 101.79 | 348.5 | 401.1 | 104.37 |
| 115123 | 3-Mar | 81.96 | 51.288 | 73.467 | 72.773 | 345.25 | 86.994 | 110.34 | 75.808 | 75.208 | 61.51 |
| 8115 | TCL1A | 641.02 | 399.85 | 114.45 | 76.966 | 13310 | 3401.9 | 2895.9 | 575.35 | 902.55 | 275.69 |
| | | 915.56 | 281.78 | 499.02 | 263.74 | 3325 | 798.08 | 831.82 | 778 | 802.7 | 432.14 |
| | | 221.07 | 128.11 | 19.172 | 32.89 | 1833 | 502.73 | 422.8 | 785.39 | 690.67 | 272.74 |
| 283663 | LINC00926 | 726.74 | 166.83 | 123 | 126.47 | 23022 | 6478.5 | 4462 | 6503.1 | 7265.8 | 226.35 |
| 283663 | LINC00926 | 692.64 | 453.05 | 520.19 | 392.15 | 13389 | 4016.6 | 2195.3 | 3613.9 | 4563.9 | 556.32 |
| 933 | CD22 | 390.89 | 254.78 | 175.49 | 153.74 | 5953.5 | 1829.8 | 1631.8 | 1793.3 | 2011 | 266.97 |
| 55278 | QRSL 1 | 387.5 | 465.19 | 526.61 | 783.83 | 2643 | 875.1 | 727.76 | 1464.3 | 1714.3 | 621.96 |
| 94235 | GNG8 | 53.579 | 611.56 | 23.299 | 184.36 | 51.681 | 8705.2 | 681.42 | 43.5 | 64.063 | 57.214 |
| 23089 | PEG10 | 46.498 | 31.167 | 66.482 | 18.336 | 253.9 | 2036.6 | 121.93 | 81.024 | 103.77 | 73.413 |
| 7782 | SLC30A4 | 743.61 | 680.67 | 534.2 | 492.15 | 1318.3 | 7943.9 | 326.7 | 1065.5 | 710.81 | 847.84 |
| | | 72.247 | 269.15 | 243.03 | 479.83 | 451.44 | 3818.5 | 2789.4 | 150.52 | 188.22 | 240.7 |
| | | 121.46 | 90.123 | 93.263 | 76.315 | 493.97 | 2956.6 | 774.48 | 232.44 | 167.55 | 182.61 |
| 148932 | MOB3C | 879.19 | 2018.4 | 1343.5 | 1029.6 | 825.81 | 13894 | 717.4 | 604.51 | 685.16 | 895.23 |
| 80237 | ELL3 | 156.69 | 158.02 | 95.262 | 104.13 | 503.88 | 2672.8 | 627.44 | 196.76 | 187.92 | 102 |
| 1184 | CLCN5 | 757.15 | 676.29 | 495.2 | 584.13 | 623.52 | 4105.2 | 524.36 | 1044.6 | 1151.2 | 764.8 |
| 653121 | ZBTB8A | 163.85 | 107.16 | 57.552 | 97.531 | 219.78 | 2444 | 154.17 | 222.47 | 278.34 | 440.52 |
| | | 53.024 | 126.27 | 68.75 | 269.56 | 451.93 | 3470.4 | 881.09 | 127.17 | 231.04 | 56.611 |
| 1184 | CLCN5 | 155.94 | 162.89 | 109.18 | 181.57 | 159.9 | 1331.7 | 179.77 | 216.7 | 184.82 | 145.23 |
| 94274 | PPP1R14A | 30.346 | 9.07 | 9096 | 9.518 | 392.81 | 2681.6 | 109.95 | 196.29 | 198.03 | 100.7 |
| 80237 | ELL3 | 159.7 | 130.7 | 92.114 | 118.93 | 420.44 | 1706 | 524.31 | 229.49 | 264.89 | 170.12 |
| 1490 | CTGF | 22.249 | 22.905 | 6.739 | 3.515 | 43.341 | 265.32 | 34.172 | 29.803 | 13.406 | 12.285 |
| 148932 | MOB3C | 588.25 | 1078.3 | 640.07 | 622.65 | 514.18 | 4626 | 496.97 | 611.27 | 530.52 | 453.65 |
| 140733 | MACROD2 | 210.22 | 110.16 | 58.043 | 68.735 | 3510.6 | 11697 | 5016.8 | 124.2 | 181.28 | 644.92 |
| 116449 | CLNK | 177.14 | 95.259 | 75.054 | 51.036 | 183.49 | 657.76 | 44.223 | 102.93 | 129.5 | 188.89 |
| 51237 | MZB1 | 768.21 | 1072 | 205.82 | 123.61 | 2046.6 | 1716.7 | 785.93 | 740.4 | 838.1 | 37183 |
| 3514 | IGKC | 17.024 | 12.551 | 9.378 | 10.146 | 26.928 | 11.946 | 14.494 | 23.924 | 21.578 | 677.99 |
| 3537 | IGLC1 | 27.368 | 28.913 | 16.45 | 26.404 | 135.1 | 40.005 | 74.637 | 57.895 | 39.922 | 5202.1 |
| | | 476.89 | 528.5 | 108.54 | 42.032 | 957.48 | 592.6 | 712.36 | 296.25 | 472.3 | 32592 |
| 81618 | ITM2C | 585.42 | 318.44 | 534.58 | 194.83 | 1031.8 | 738.28 | 247.2 | 577.24 | 779.46 | 11437 |
| 51237 | MZB1 | 506.82 | 307.29 | 21.088 | 21.828 | 1374.2 | 1319.5 | 490.69 | 260.48 | 239.29 | 17479 |
| 608 | TNFRSF 17 | 481.56 | 79.328 | 7.069 | 4.69 | 1187.8 | 365.71 | 263.56 | 626.98 | 442.39 | 14375 |
| | | 669.73 | 445.68 | 99.639 | 82.5 | 1858 | 900.13 | 604.22 | 1247.4 | 2371.6 | 22774 |
| 96610 | BMS1P20 | 1159.5 | 1240.9 | 243.81 | 144.13 | 4950.9 | 3547.4 | 1665.6 | 1460.3 | 1353.7 | 44647 |
| | | 132.76 | 183.85 | 15.044 | 25.193 | 757.95 | 436.2 | 527.22 | 512.07 | 467.82 | 6583.2 |
| 51303 | FKBP11 | 1010.8 | 1771.9 | 1287 | 1715.5 | 833.49 | 736.14 | 618.66 | 596.84 | 431.99 | 13740 |
| | | 107.22 | 119.57 | 35.991 | 31.263 | 429.37 | 167.16 | 108.45 | 65.809 | 55.434 | 4308.9 |
| | | 18.285 | 15.852 | 9.172 | 7.818 | 47.27 | 47.245 | 16.959 | 37.188 | 22.782 | 555.37 |
| | | 589.65 | 458.26 | 148.22 | 85.462 | 3392.6 | 2245 | 842.29 | 1265.4 | 1225.6 | 26624 |
| 79694 | MANEA | 87.389 | 192.19 | 113.36 | 190.16 | 109.12 | 128.83 | 61.721 | 57.43 | 51.351 | 1510.6 |
| 28823 | IGLV1-44 | 316.7 | 249.63 | 21.981 | 8.441 | 1613 | 1120.6 | 591.59 | 697.15 | 670.13 | 13019 |
| 857 | CAV1 | 45.356 | 22.639 | 15.996 | 72.569 | 96.76 | 64.758 | 65.577 | 46.166 | 24.508 | 1889.2 |
| 10316 | NMUR1 | 16.485 | 10.581 | 10.655 | 9.429 | 23.474 | 16.968 | 11.241 | 28.366 | 24.025 | 21.331 |
| 2043 | EPHA4 | 232.57 | 88.394 | 232.39 | 42.03 | 171.22 | 92.377 | 36.349 | 140.79 | 81.496 | 54.073 |
| 10079 | ATP9A | 11.562 | 14.731 | 6.817 | 9.387 | 12.617 | 10.289 | 11.696 | 19.097 | 25.438 | 10.548 |
| 9289 | ADGRG 1 | 450.23 | 46.85 | 680.21 | 378.36 | 273.85 | 146.6 | 17.949 | 47.686 | 42.818 | 31.702 |
| 2043 | EPHA4 | 304.21 | 44.721 | 162.55 | 12.343 | 158.35 | 137.15 | 20.26 | 166.81 | 42.352 | 38.186 |
| 79901 | CYBRD1 | 7.287 | 5.64 | 5.593 | 5.551 | 9.848 | 8.445 | 7.804 | 19.536 | 17.719 | 8.519 |
| | | 56.285 | 18.537 | 102.77 | 15.889 | 50.579 | 25.757 | 25.26 | 112.58 | 61.378 | 25.283 |
| 151742 | PPM1L | 264.55 | 96.483 | 257.25 | 150.53 | 209.8 | 240.56 | 152.7 | 259.38 | 192.84 | 210.88 |
| 81563 | C1orf21 | 150.22 | 48.558 | 482.41 | 176.14 | 151.32 | 97.005 | 29.468 | 93.735 | 57.454 | 63.917 |
| 2619 | GAS1 | 12.055 | 10.149 | 9.647 | 9.038 | 20.012 | 12.016 | 8.361 | 24.734 | 25.129 | 12.794 |
| 59338 | PLEKHA 1 | 2609.8 | 1355.1 | 1304.4 | 665.37 | 1768.9 | 1304.9 | 762.61 | 848.99 | 955.81 | 356.19 |
| 2043 | EPHA4 | 900.99 | 422.48 | 455.53 | 254.47 | 417.9 | 429.13 | 273.55 | 717.74 | 602.07 | 596.28 |
| 4068 | SH2D1A | 390.85 | 464.3 | 737.65 | 455.64 | 77.492 | 72.491 | 74.097 | 47.594 | 46.695 | 20.921 |
| 2043 | EPHA4 | 634.35 | 162.79 | 615.75 | 128.7 | 195.17 | 190.16 | 66.138 | 395.22 | 109.34 | 155.65 |
| 2774 | GNAL | 85.932 | 63.9 | 41.075 | 28.466 | 74.428 | 43.235 | 33.021 | 90.869 | 85.197 | 79.187 |
| 5243 | ABCB1 | 281.26 | 147.7 | 424.32 | 150.74 | 410.31 | 274.03 | 186.01 | 228.02 | 225.67 | 224.27 |
| 11098 | PRSS23 | 337.78 | 158.02 | 147.29 | 189.44 | 245.58 | 359.39 | 132.8 | 330.03 | 377.04 | 351.5 |
| 127254 | ERICH3 | 53.192 | 88.232 | 19.387 | 54.307 | 43.526 | 75.933 | 42.083 | 112.26 | 106.76 | 33.029 |
| 57489 | ODF2L | 402.52 | 524.06 | 396.31 | 137.76 | 355.88 | 155.38 | 159.46 | 183.49 | 113.26 | 312.66 |
| 57489 | ODF2L | 361.13 | 401.41 | 336.7 | 142.21 | 219.51 | 187.77 | 125.38 | 240.69 | 116.48 | 274.85 |
| 257019 | FRMD3 | 304.9 | 315.32 | 121.07 | 156.3 | 292.62 | 214.85 | 117.87 | 312.93 | 474.35 | 213.8 |
| 10974 | ADIRF | 13.1 | 8.346 | 7.777 | 5.934 | 22.182 | 27.798 | 11.084 | 22.65 | 20.283 | 15.804 |
| 6672 | SP100 | 301.3 | 711.82 | 491.39 | 248.45 | 889.83 | 574.84 | 396.32 | 225.06 | 197.43 | 120.03 |
| 64108 | RTP4 | 156.03 | 1646.5 | 521.18 | 124.92 | 92.382 | 560.78 | 58.119 | 59.991 | 60.383 | 224.17 |
| 55603 | FAM46A | 209.46 | 254.63 | 56.082 | 30.957 | 262.27 | 338.22 | 140.46 | 70.072 | 70.491 | 96.183 |
| 54809 | SAMD9 | 878.92 | 5053.9 | 3029.7 | 540.35 | 1463 | 1883.7 | 500 | 389.29 | 544.59 | 1078.5 |
| 257019 | FRMD3 | 290.98 | 504.45 | 214.86 | 121.57 | 355.76 | 267.45 | 226.52 | 846.65 | 811.22 | 387.72 |
| 91624 | NEXN | 67.656 | 627 | 67.589 | 118.05 | 87.618 | 101.62 | 150.77 | 82.89 | 38.41 | 30.593 |
| | | 34.715 | 451.51 | 34.551 | 44.167 | 32.135 | 29.07 | 32.918 | 52.246 | 56.176 | 76.157 |
| 85363 | TRIM5 | 177.65 | 345.55 | 336.22 | 108.71 | 330.94 | 318.82 | 274.03 | 143.37 | 183.16 | 317.66 |
| 50650 | ARHGEF3 | 1839.8 | 2785.7 | 3143.2 | 928.31 | 802.1 | 999.06 | 266.06 | 374.46 | 472.91 | 440.33 |
| 100131733 | USP30-AS1 | 321.04 | 662.29 | 499.97 | 368.98 | 350.57 | 250.69 | 163.87 | 200.3 | 148.86 | 637.26 |
| | | 190.68 | 1376.2 | 626.48 | 604.37 | 248.31 | 303.43 | 158.5 | 49.547 | 60.247 | 882.36 |
| 2635 | GBP3 | 70407 | 2327.8 | 2251.5 | 1177 | 507.74 | 597.15 | 260.27 | 260.49 | 282.58 | 680.29 |
| 5654 | HTRA1 | 298.66 | 211.29 | 129.67 | 158.13 | 244.18 | 325.94 | 217.11 | 254.78 | 217.39 | 378.02 |
| 2048 | EPHB2 | 18.128 | 14.747 | 13.441 | 10.721 | 22.435 | 19.212 | 13.813 | 41.378 | 38.509 | 19.184 |
| 10461 | MERTK | 170.5 | 109.41 | 114.19 | 123.07 | 217.36 | 151.04 | 110.88 | 131.29 | 147.68 | 167.79 |
| 4048 | LTA4H | 2762.8 | 3065.3 | 1893.5 | 2088.7 | 5716.5 | 5135.1 | 4152 | 2095.9 | 2279.2 | 3178.3 |
| 2048 | EPHB2 | 30.863 | 44.763 | 17.499 | 32.405 | 59.756 | 19.794 | 14.376 | 68.248 | 49.623 | 86.171 |
| 10461 | MERTK | 190.69 | 107.8 | 58.619 | 53.798 | 117.56 | 104.65 | 56.653 | 236.55 | 207.85 | 107.1 |
| 340526 | RGAG4 | 78.208 | 111.4 | 61.529 | 63.163 | 103.33 | 94.134 | 75.628 | 96.029 | 94.257 | 95.477 |
| 284013 | VMO1 | 34.258 | 39.726 | 11.115 | 11.268 | 16.153 | 14.451 | 19.847 | 23.683 | 20.328 | 23.967 |
| 120939 | TMEM52B | 47.859 | 43.294 | 34.943 | 30.558 | 36.092 | 85.694 | 45.534 | 69.956 | 54.285 | 134.15 |
| 408 | ARRB1 | 34.25 | 29.224 | 38.111 | 72.62 | 31.272 | 19.392 | 15.134 | 37.238 | 34.008 | 20.44 |
| 2048 | EPHB2 | 197.37 | 142.57 | 136.13 | 141.82 | 204.37 | 159.93 | 109.4 | 240.71 | 226.39 | 202.71 |
| 2517 | FUCA1 | 242.55 | 302.08 | 495.6 | 216.27 | 440.56 | 450.22 | 469.94 | 416.64 | 325.97 | 713.21 |
| | | 170.59 | 108.76 | 126.74 | 100.47 | 271.42 | 210.77 | 199.36 | 122.69 | 123.37 | 38.037 |
| 2335 | FN1 | 26.448 | 27.259 | 17.197 | 26.945 | 30.48 | 25.495 | 42.183 | 81.327 | 103.35 | 16.885 |
| 11326 | VSIG4 | 233 | 137.81 | 120.47 | 81.189 | 213.86 | 154.35 | 61.94 | 352.53 | 358.35 | 198.53 |
| 2335 | FN1 | 70.789 | 23.169 | 29.882 | 44.304 | 59.421 | 56.337 | 17.899 | 52.106 | 56.731 | 16.433 |
| 51063 | CALHM2 | 314.56 | 124.06 | 776.91 | 70.875 | 230.96 | 151.41 | 104.78 | 139.07 | 174.98 | 129.8 |
| 55244 | SLC47A1 | 18.184 | 27.079 | 14.001 | 17.424 | 21.488 | 30.656 | 13.634 | 24.757 | 22.461 | 30.17 |
| 2162 | F13A1 | 289.19 | 154.99 | 124.88 | 105.2 | 313.25 | 233.58 | 115.91 | 479.91 | 402.89 | 247.61 |
| 10462 | CLEC10A | 117.66 | 77.553 | 54.795 | 58.318 | 113.97 | 116.72 | 20.433 | 119.71 | 111.38 | 104.15 |
| 246 | ALOX15 | 10.612 | 25.339 | 6.831 | 6.378 | 13.36 | 11.346 | 9.715 | 19.972 | 58.372 | 10.044 |
| 23475 | QPRT | 22.06 | 16.746 | 54.589 | 25.22 | 24.631 | 16.766 | 31.21 | 87.88 | 56.88 | 141.4 |
| 154092 | LINC01010 | 40.498 | 30.122 | 31.198 | 25.411 | 32.529 | 28.516 | 36.734 | 69.953 | 64.71 | 44.851 |
| 23017 | FAIM2 | 53.577 | 29.106 | 14.683 | 16.222 | 41.65 | 21.3 | 18.821 | 28.089 | 25.181 | 28.634 |
| 79839 | CCDC102B | 13.112 | 8.355 | 4.523 | 5.864 | 5.023 | 8.302 | 1.015 | 37.086 | 9.202 | 8.124 |
| 5445 | PON2 | 153.68 | 113.96 | 189.29 | 208.72 | 203.37 | 121.68 | 97.311 | 120.96 | 128.04 | 130.15 |
| 30835 | CD209 | 220.38 | 169.13 | 83.777 | 91.853 | 207.13 | 163.27 | 99.57 | 313.17 | 251 | 187.27 |
| 51477 | ISYNA1 | 285.49 | 212.26 | 179.91 | 183.44 | 292.53 | 198.33 | 85.288 | 65.867 | 135.68 | 52.564 |
| 30835 | CD209 | 106.67 | 65.52 | 19.824 | 18.065 | 59.958 | 52.787 | 21.539 | 35.029 | 47.617 | 24.918 |
| 2878 | GPX3 | 207.97 | 169.7 | 117.12 | 135.49 | 237.29 | 182.73 | 161.49 | 414.11 | 438.13 | 229.3 |
| 2878 | GPX3 | 202.77 | 174.29 | 157.95 | 111.93 | 261.65 | 236.72 | 154.17 | 295.06 | 301.73 | 234.74 |
| 5445 | PON2 | 142.99 | 90.515 | 112.25 | 122 | 90.488 | 64.661 | 62.882 | 113.24 | 149.34 | 50.288 |
| 56670 | SUCNR1 | 181.08 | 728.19 | 164.8 | 113.29 | 340.37 | 277.13 | 142.74 | 423.79 | 260.43 | 305.45 |
| 30850 | CDR2L | 14.937 | 10.283 | 11.864 | 17.984 | 14.459 | 8.9 | 13.077 | 13.102 | 13.026 | 10.069 |
| 11067 | C10orf10 | 37.582 | 61.905 | 18.124 | 17.372 | 28.084 | 23.813 | 25.516 | 43.537 | 39.406 | 29.808 |
| 6624 | FSCN1 | 21.011 | 314.03 | 33.576 | 161.6 | 16.286 | 63.516 | 55.39 | 54.472 | 29.717 | 24.173 |
| 54662 | TBC1D13 | 76.209 | 49.734 | 69.905 | 50.81 | 70.849 | 141.92 | 114.81 | 54.299 | 51.949 | 51.197 |
| 101930114 | LOC101930114 | 333.43 | 340.39 | 283.22 | 277.53 | 237.07 | 137.2 | 75.169 | 147.68 | 206.61 | 305.57 |
| 5157 | PDGFRL | 36.427 | 9.53 | 14.258 | 12.599 | 34.964 | 16.96 | 44.181 | 40.21 | 22.475 | 13.408 |
| 3429 | IFI27 | 162.25 | 912.04 | 119.25 | 65.958 | 75.112 | 112.25 | 46.373 | 50.78 | 94.163 | 119.78 |
| | | 78.773 | 668.53 | 20.265 | 24.835 | 25.86 | 39.337 | 23.056 | 114.36 | 60.898 | 40.013 |
| 80045 | GPR157 | 1127.6 | 936.33 | 442.99 | 373.89 | 792.28 | 765.69 | 864.41 | 672.41 | 417.83 | 1021.2 |
| 80380 | PDCD1LG2 | 37.157 | 39.408 | 26.364 | 31.244 | 35.784 | 26.589 | 26.191 | 63.203 | 53.796 | 59.15 |
| 11067 | C10orf10 | 86.486 | 66.895 | 25.297 | 43.289 | 87.619 | 102.61 | 23.724 | 58.954 | 61.644 | 63.256 |
| 8820 | HESX1 | 15.962 | 20.309 | 9.002 | 16.894 | 20.791 | 22.89 | 13.287 | 26.034 | 26.317 | 17.264 |
| 6624 | FSCN1 | 20.332 | 566.4 | 33.336 | 168.56 | 71.603 | 102.41 | 67.491 | 10.366 | 6.506 | 7.758 |
| 11167 | FSTL1 | 192.12 | 120.83 | 113.92 | 109.45 | 195.78 | 158.41 | 118.18 | 353.01 | 375.64 | 162.49 |
| 54662 | TBC1D13 | 624.15 | 537.35 | 501.78 | 399.81 | 665.39 | 730.1 | 446.29 | 528.19 | 571.03 | 405.15 |
| 9175 | MAP3K13 | 6.504 | 4.804 | 3.886 | 3.113 | 5.906 | 5.464 | 4.814 | 8.781 | 9.781 | 11.148 |
| 3357 | HTR2B | 34.96 | 28.74 | 11.051 | 3.945 | 26.466 | 10.004 | 15.287 | 25.989 | 17.253 | 29.89 |
| 94015 | TTYH2 | 607.39 | 287.84 | 245.13 | 104.92 | 237.65 | 192.38 | 136.59 | 76.097 | 105.62 | 369.95 |
| 56300 | IL36G | 91.241 | 91.419 | 44.993 | 41.394 | 72.339 | 78.498 | 53.339 | 171.73 | 175.77 | 85.443 |
| 3036 | HAS1 | 83.836 | 49.02 | 39.758 | 51.693 | 82.304 | 62.179 | 22.661 | 135.41 | 153.81 | 107.37 |
| 7980 | TFPI2 | 15.299 | 25.449 | 6.514 | 7.562 | 15.212 | 135.04 | 88.061 | 22.203 | 21.938 | 12.314 |
| 11009 | IL24 | 85.352 | 83.076 | 38.318 | 38.162 | 251.23 | 72.213 | 118.18 | 67.58 | 126.55 | 22.751 |
| 4312 | MMP1 | 9.388 | 26.327 | 2.994 | 3.6 | 8.573 | 16.557 | 7.346 | 33.165 | 45.886 | 6.634 |
| 7980 | TFPI2 | 25.095 | 20.044 | 10.747 | 5.447 | 35.849 | 74.723 | 52.423 | 70.474 | 69.994 | 34.393 |
| 1440 | CSF3 | 18.01 | 24.088 | 13.039 | 11.862 | 29.41 | 19.209 | 19.475 | 26.511 | 26.774 | 20.875 |
| 3569 | IL6 | 207.34 | 440.66 | 94.669 | 75.292 | 320.72 | 1588 | 284.03 | 341.25 | 435.91 | 145.62 |
| 4233 | MET | 11.918 | 12.065 | 5.665 | 56.352 | 11.912 | 10.115 | 35.396 | 24.648 | 16.854 | 32.194 |
| 169792 | GLIS3 | 51.725 | 42.231 | 9.847 | 76.931 | 13.202 | 16.12 | 14.462 | 83.659 | 111.02 | 64.578 |
| 51334 | PRR16 | 11.96 | 4.882 | 2.492 | 0.985 | 3.236 | 8.931 | 22.338 | 34.53 | 23.283 | 19.207 |
| 6374 | CXCL5 | 31.673 | 28.613 | 23.431 | 24.093 | 36.395 | 9.425 | 17.418 | 54.262 | 77.543 | 38.061 |
| 6660 | SOX5 | 9.836 | 7.861 | 7.661 | 6.475 | 29.154 | 8.14 | 11.336 | 19.539 | 23.041 | 16.023 |
| 79931 | TNIP3 | 30.483 | 496.06 | 87 | 200.55 | 28.186 | 19.561 | 27.081 | 64.546 | 62.118 | 37.863 |
| 8710 | SERPINB7 | 31.11 | 25.351 | 15.255 | 11.639 | 31.797 | 20.7 | 20.595 | 48.02 | 44.575 | 24.085 |
| 3690 | ITGB3 | 77.937 | 52.599 | 25.61 | 12.752 | 87.344 | 100.16 | 79.679 | 27.204 | 28.815 | 13.054 |
| 5743 | PTGS2 | 75.522 | 58.612 | 20.681 | 22.695 | 16.161 | 26.012 | 21.551 | 28.498 | 43.676 | 25.445 |
| 8794 | TNFRSF10C | 24.745 | 17.582 | 11.933 | 10.705 | 16.047 | 12.525 | 12.11 | 37.874 | 17.543 | 9.775 |
| 53829 | P2RY13 | 20.207 | 15.818 | 7.568 | 6.407 | 29.631 | 76.257 | 13.312 | 70.916 | 33.945 | 22.424 |
| 4311 | MME | 52.68 | 35.537 | 27.734 | 32.273 | 47.653 | 44.56 | 32.565 | 106.81 | 102.32 | 68.168 |
| 146225 | CMTM2 | 404.54 | 263.4 | 375.27 | 305.18 | 320.72 | 272.32 | 165.77 | 841.78 | 212.16 | 494.7 |
| 8794 | TNFRSF10C | 176.1 | 142.03 | 83.535 | 142.24 | 49.475 | 43.257 | 26.589 | 45.615 | 59.935 | 125.79 |
| 8794 | TNFRSF10C | 122.04 | 82.36 | 80.849 | 72.071 | 101.24 | 94.444 | 66.289 | 143.19 | 149.05 | 94.772 |
| 6286 | S100P | 341.03 | 96.34 | 29.987 | 26.748 | 230.68 | 299.56 | 120.18 | 69.519 | 41.807 | 30.985 |
| 3577 | CXCR1 | 55.571 | 34.928 | 30.255 | 30.118 | 51.796 | 46.036 | 23.438 | 66.93 | 68.085 | 27.242 |
| 60675 | PROK2 | 25.175 | 26.004 | 158.35 | 155.29 | 82.893 | 49.74 | 28.545 | 24.378 | 16.613 | 53.691 |
| 54682 | MANSC 1 | 75.87 | 57.915 | 31.179 | 33.702 | 98.717 | 103.16 | 34.426 | 291.74 | 249.55 | 97.194 |
| 144423 | GLT1D1 | 48.013 | 28.57 | 27.015 | 34.934 | 109.92 | 33.965 | 25.369 | 54.365 | 48.216 | 45.415 |
| 3579 | CXCR2 | 48.606 | 7.934 | 13.365 | 4.607 | 34.921 | 15.821 | 19.504 | 13.723 | 15.532 | 19.566 |
| 25984 | KRT23 | 21.721 | 25.964 | 13.516 | 29.433 | 77.001 | 62.235 | 52.891 | 151.99 | 138.74 | 106.63 |
| 2215 | FCGR3B | 420.69 | 197.38 | 238.39 | 130.46 | 284.33 | 180.1 | 91.841 | 248.53 | 239.36 | 162.95 |
| 4311 | MME | 21.906 | 20.104 | 7.349 | 5.374 | 33.004 | 64.683 | 12.237 | 32.656 | 30.344 | 18.396 |
| 79908 | BTNL8 | 18.42 | 11.019 | 9.26 | 19.771 | 28.565 | 22.44 | 13.582 | 25.459 | 26.706 | 26.866 |
| | | 86.434 | 63.098 | 71.649 | 52.289 | 87.488 | 50.563 | 44.979 | 99.362 | 61.429 | 110.7 |
| | | 1854.5 | 61.454 | 48.362 | 44.29 | 999.91 | 57.159 | 47.999 | 62.948 | 64.1 | 66.571 |
| 80201 | HKDC 1 | 442.35 | 35.677 | 47.464 | 21.902 | 45.899 | 34.716 | 31.414 | 65.151 | 73.108 | 71.596 |
| | | 297.62 | 19.749 | 6.99 | 17.411 | 66.318 | 13.655 | 10.1 | 11.711 | 9.278 | 13.786 |
| 3572 | IL6ST | 500.94 | 54.356 | 55.673 | 34.638 | 18.455 | 33.371 | 45.084 | 127.48 | 253.1 | 93.452 |
| 6711 | SPTBN1 | 1419.8 | 51.589 | 46.397 | 44.595 | 332.99 | 37.121 | 54.203 | 108.92 | 253.63 | 122.46 |
| 6920 | TCEA3 | 1084.5 | 225.21 | 238.7 | 62.339 | 68.575 | 50.839 | 46.818 | 75.369 | 171.03 | 152.37 |
| | | 1279.9 | 295.26 | 403.78 | 149.61 | 92.396 | 109.06 | 137.22 | 125.98 | 246.18 | 43.598 |
| 26119 | LDLRAP1 | 2504.4 | 344.2 | 835.09 | 62.217 | 197.35 | 98.984 | 173 | 135.23 | 159.29 | 284.72 |
| | | 2596.8 | 466.59 | 585.78 | 390.5 | 474.34 | 448.8 | 270.5 | 136.42 | 258 | 408.3 |
| 3562 | IL3 | 11.796 | 382.21 | 7.019 | 134.51 | 10.679 | 10.166 | 9.046 | 36.347 | 16.231 | 9.837 |
| 50616 | IL22 | 51.201 | 3661.6 | 26.909 | 880.39 | 42.27 | 53.862 | 16.571 | 102.85 | 70.52 | 146.28 |
| 64788 | LMF1 | 1219.7 | 1270.8 | 138.68 | 191.49 | 111.82 | 123.14 | 198.43 | 173.84 | 193.14 | 182.04 |
| | | 1125 | 2341.9 | 195.23 | 381.74 | 356.46 | 283.78 | 174.75 | 205.37 | 190.32 | 240.26 |
| 959 | CD40LG | 492.38 | 838.31 | 67.853 | 167.51 | 104.76 | 46.611 | 20.655 | 64.876 | 58.402 | 42.182 |
| 50943 | FOXP3 | 322.53 | 1418.7 | 167.46 | 635.89 | 89.541 | 65.338 | 48.38 | 220.77 | 206.11 | 146.06 |
| 54602 | NDFIP2 | 562.06 | 2564.1 | 486.91 | 2284.5 | 335.08 | 441.29 | 350.3 | 661.08 | 704.09 | 614.13 |
| 1493 | CTLA4 | 1240.2 | 4434.9 | 489.94 | 1618.8 | 53.729 | 52.941 | 16.366 | 134.12 | 85.046 | 151.54 |
| 55423 | SIRPG | 1279.7 | 344.58 | 1870.4 | 377.07 | 176.03 | 122.11 | 36.387 | 183.64 | 157.75 | 97.431 |
| 917 | CD3G | 2246.6 | 916.04 | 3622.2 | 876.21 | 201.71 | 87.324 | 19.905 | 53.472 | 55.177 | 32.644 |
| 10663 | CXCR6 | 147.04 | 969.58 | 1662.6 | 350.63 | 135.37 | 191.12 | 73.848 | 262.77 | 265.17 | 136.31 |
| 3090 | HIC1 | 37.26 | 320.67 | 1359.6 | 1251.4 | 43.644 | 62.872 | 53.679 | 62.066 | 56.536 | 43.042 |
| 27240 | SIT1 | 823.62 | 686.41 | 2063 | 786.65 | 1305.4 | 447.22 | 556.99 | 658.06 | 732.74 | 394.33 |
| 51676 | ASB2 | 380.33 | 402.91 | 2482.4 | 1135.9 | 242.59 | 289.52 | 203.62 | 242.59 | 200.29 | 612.31 |
| 91978 | TPGS1 | 147.3 | 83.667 | 652.05 | 427.54 | 76.829 | 56.022 | 175.17 | 42.183 | 46.635 | 136.02 |
| 28755 | TRAC | 9535.5 | 4889.7 | 11371 | 4324.1 | 989.81 | 734 | 297.26 | 509.97 | 406.85 | 471.19 |
| 3932 | LCK | 4328.6 | 1960.6 | 5769.7 | 2096.1 | 582.59 | 401.34 | 265.26 | 313.1 | 297.33 | 85.982 |
| 79413 | ZBED2 | 153.9 | 3485.5 | 158.99 | 5721.5 | 244.85 | 328.8 | 549.38 | 314.44 | 244.52 | 123.05 |
| 993 | CDC25A | 13.246 | 24.781 | 88.757 | 868.1 | 16.454 | 12.278 | 11.699 | 27.212 | 27.494 | 47.288 |
| 151230 | KLHL23 | 60.364 | 94.954 | 220.96 | 1774.3 | 72.694 | 58.921 | 60.413 | 79.842 | 48.502 | 36.977 |
| 29128 | UHRF 1 | 236.33 | 1119.1 | 3816.9 | 11233 | 321.11 | 227.75 | 796.15 | 223.23 | 171.58 | 1438.7 |
| | | 233.92 | 290.59 | 813.35 | 3551 | 308.18 | 279.31 | 439.72 | 282.41 | 256.85 | 363.93 |
| 29089 | UBE2T | 174.69 | 798.98 | 1705.3 | 6581 | 117.92 | 255.28 | 393.67 | 100.27 | 84.534 | 842.79 |
| 3070 | HELLS | 47.141 | 87.792 | 454.04 | 1303.1 | 93.846 | 106.47 | 136.72 | 41.992 | 43.732 | 61.13 |
| 8438 | RAD54L | 37.236 | 27.188 | 174 | 529.88 | 29.891 | 30.037 | 23.372 | 35.105 | 31.035 | 17.64 |
| 3070 | HELLS | 284.84 | 423.31 | 1327.9 | 3798.5 | 346.27 | 421.1 | 591.66 | 309.23 | 325.53 | 424.17 |
| 10563 | CXCL 13 | 19.513 | 1354.7 | 29.476 | 1874.5 | 18.396 | 16.105 | 190.19 | 60.868 | 41.46 | 23.525 |
| 79075 | DSCC1 | 41.096 | 98.875 | 266.43 | 1054.4 | 34.983 | 45.943 | 45.53 | 38.164 | 54.297 | 115.2 |
| 4049 | LTA | 36.869 | 2186 | 19.704 | 2896.6 | 23.214 | 305.93 | 19.847 | 22.388 | 21.473 | 14.365 |
| 10328 | EMC8 | 27.346 | 73.761 | 32.207 | 695.77 | 25.677 | 34.441 | 63.591 | 60.775 | 60.12 | 98.738 |
| 84824 | FCRLA | 130.31 | 41.849 | 42.774 | 32.087 | 3782.4 | 1105.1 | 2016.2 | 1131.8 | 1993.1 | 118.29 |
| | | 668.07 | 294.22 | 50.476 | 56.156 | 21387 | 11410 | 6419.6 | 332.54 | 1278.4 | 329.1 |
| 3899 | AFF3 | 533.25 | 92.106 | 343.14 | 118.34 | 9753.7 | 6992 | 4487.8 | 1347.4 | 2163 | 17.917 |
| 55024 | BANK1 | 131.67 | 62.309 | 17.332 | 13.043 | 5063.1 | 3369.7 | 1609 | 4849.6 | 4389 | 269.4 |
| 931 | MS4A1 | 756 | 462.64 | 129.79 | 55.3 | 13482 | 12327 | 7914.7 | 12484 | 14351 | 330.25 |
| | | 743.39 | 815.94 | 246.9 | 284.77 | 15139 | 11615 | 7550.3 | 4127.6 | 4829.9 | 1153.1 |
| 931 | MS4A1 | 909.27 | 610.61 | 159.55 | 92.926 | 15292 | 14082 | 8618.6 | 14009 | 16289 | 294.53 |
| 199786 | FAM129C | 1149.9 | 781.16 | 583.52 | 548.37 | 15403 | 7850.1 | 4753.7 | 3450 | 6123.6 | 822.34 |
| 115350 | FCRL1 | 416.95 | 132.19 | 110.29 | 114.67 | 10113 | 3156.3 | 1094.1 | 1986.9 | 2423.5 | 875.88 |
| | | 415.85 | 176.56 | 284.27 | 153.54 | 7089.1 | 3593 | 3206.7 | 1259.2 | 1392.3 | 207.42 |
| 931 | MS4A1 | 291.55 | 183.21 | 166.4 | 186.99 | 8517.3 | 10792 | 5230.7 | 3721.6 | 4253.6 | 312.53 |
| 931 | MS4A1 | 1316.4 | 1018.6 | 104.4 | 58.217 | 33100 | 35052 | 20987 | 23579 | 26239 | 395.68 |
| 53335 | BCL11A | 338.58 | 370.66 | 69.794 | 59.023 | 13141 | 14169 | 6339.9 | 3436.7 | 3985.5 | 528.8 |
| 26040 | SETBP1 | 164.27 | 180.62 | 200.7 | 219.58 | 1157 | 3083.3 | 786.02 | 716.76 | 599.74 | 526.88 |
| 53335 | BCL11A | 138.73 | 133.94 | 25.675 | 41.093 | 2742.6 | 3364.6 | 1258.2 | 1038.8 | 1261.6 | 187.64 |
| | | 41.785 | 67.193 | 9.726 | 5.421 | 163.95 | 47.628 | 32.218 | 45.183 | 41.792 | 1278.4 |
| | | 13.19 | 15.156 | 7.21 | 7.64 | 141.74 | 45.987 | 36.33 | 21.507 | 51.597 | 827.93 |
| | | 968.69 | 815.12 | 23.298 | 23.718 | 5042.3 | 2888.9 | 2118.1 | 2838.8 | 3291.2 | 34965 |
| 3514 | IGKC | 443.22 | 466.97 | 17.373 | 12.526 | 4208.7 | 2671.2 | 1577.3 | 1877 | 2561.6 | 34431 |
| 857 | CAV1 | 78.802 | 62.756 | 35.597 | 78.088 | 88.197 | 45.911 | 78.45 | 119.82 | 124.39 | 1721.2 |
| | | 677.66 | 476.06 | 157.64 | 134.86 | 2782.3 | 1358.7 | 976.07 | 737.17 | 1560.2 | 21772 |
| 100379345 | MIR181A2HG | 21.964 | 34.023 | 28.871 | 23.483 | 28.206 | 39.402 | 43.26 | 60.684 | 63.493 | 39.157 |
| 53637 | S1PR5 | 15.586 | 12.486 | 8.636 | 8.962 | 16.528 | 11.045 | 13.645 | 18.64 | 16.741 | 10.684 |
| 53637 | S1PR5 | 67.846 | 44.061 | 34.084 | 28.856 | 38.968 | 48.293 | 33.393 | 82.363 | 80.263 | 80.665 |
| 53637 | S1PR5 | 717.93 | 282.15 | 666.48 | 162.36 | 414.42 | 319.18 | 33.136 | 75.413 | 64.085 | 83.253 |
| 9231 | DLG5 | 48.54 | 24.378 | 25.433 | 19.693 | 44.627 | 16.9 | 23.514 | 39.08 | 38.174 | 37.877 |
| 90102 | PHLDB2 | 357.59 | 187.77 | 433.56 | 256.29 | 505.27 | 522.21 | 174.05 | 345.23 | 277.7 | 248.14 |
| 79899 | PRR5L | 180.15 | 197.03 | 170.88 | 145.01 | 166.91 | 153.61 | 34.386 | 135.85 | 178.07 | 56.217 |
| 7049 | TGFBR3 | 533.63 | 343.64 | 541.31 | 190.35 | 192.1 | 131.58 | 96.953 | 183.11 | 213.56 | 161.31 |
| 1524 | CX3CR1 | 1329.3 | 195.08 | 634.97 | 42.747 | 441.76 | 79.278 | 23.436 | 156.29 | 157.04 | 89.718 |
| 51348 | KLRF1 | 728.3 | 114.8 | 131.7 | 24.566 | 751.36 | 240.92 | 38.515 | 203.31 | 195.7 | 100.14 |
| 5775 | PTPN4 | 960.31 | 792.99 | 1209.5 | 415.43 | 695.24 | 526.92 | 334.2 | 378.6 | 407.28 | 491.97 |
| 5775 | PTPN4 | 424.84 | 365.3 | 571.27 | 223.34 | 289.89 | 221.9 | 158.53 | 250.08 | 392.4 | 343.3 |
| 7049 | TGFBR3 | 2940.3 | 2267.2 | 2912 | 1216.9 | 588.96 | 522.78 | 231.16 | 741.98 | 630.24 | 599.46 |
| 83888 | FGFBP2 | 2087.8 | 79.084 | 8123.5 | 2358.6 | 435.28 | 230.64 | 175.08 | 88.643 | 54.91 | 54.863 |
| 114879 | OSBPL5 | 301 | 127.76 | 248.21 | 171.33 | 202.29 | 110.69 | 69.018 | 132.79 | 113.05 | 114.13 |
| 219285 | SAMD9L | 768.39 | 9098.1 | 1581.8 | 440.99 | 1694.1 | 2391.7 | 104.07 | 157.76 | 419.91 | 2176.5 |
| 54877 | ZCCHC2 | 2470.2 | 5750.5 | 1562.2 | 2493.9 | 1912.9 | 2185.9 | 1046.7 | 922.62 | 816.44 | 599.11 |
| 356 | FASLG | 272.33 | 789.32 | 739.24 | 912.05 | 263.67 | 181.14 | 81.324 | 227.78 | 206.89 | 174.67 |
| 5920 | RARRES3 | 2719.8 | 2664.6 | 3060.1 | 235.33 | 849.11 | 278.5 | 127.62 | 432.41 | 367.02 | 1801.9 |
| 388228 | SBK1 | 913.96 | 523.96 | 775.1 | 283.02 | 343.93 | 279.65 | 196.79 | 279.46 | 191.22 | 270.74 |
| 219285 | SAMD9L | 1329.8 | 9480.5 | 1860.7 | 712.66 | 1880.3 | 2386.9 | 353.87 | 846.09 | 942.06 | 2698.5 |
| 2219 | FCN1 | 467.99 | 60.392 | 78.933 | 58.575 | 284.26 | 339.72 | 113.46 | 134.87 | 123.98 | 180.89 |
| 9332 | CD163 | 101.82 | 119.76 | 79.024 | 61.356 | 118.4 | 74.272 | 44.964 | 221.24 | 226.95 | 210.04 |
| 23601 | CLEC5A | 119.11 | 95.737 | 92.389 | 84.429 | 228.91 | 150.82 | 73.627 | 226.83 | 219.5 | 159.9 |
| 9332 | CD163 | 71.858 | 85.886 | 38.251 | 24.845 | 68.466 | 53.077 | 31.555 | 174.52 | 279.05 | 140.63 |
| 51313 | FAM198B | 156.34 | 93.035 | 118.44 | 123.52 | 130.26 | 140.87 | 68.801 | 294.18 | 302.38 | 218.98 |
| 23166 | STAB1 | 128.66 | 107.3 | 93.651 | 72.915 | 83.267 | 94.68 | 113.24 | 128.71 | 106.57 | 64 |
| 10501 | SEMA6B | 33.318 | 21.347 | 24.505 | 33.317 | 76.208 | 44.16 | 22.369 | 63.408 | 40.779 | 118.86 |
| 7045 | TGFBI | 314.89 | 110.94 | 91.258 | 36.355 | 79.571 | 101.04 | 43.168 | 62.25 | 132.31 | 102.34 |
| 8536 | CAMK1 | 231.77 | 200.59 | 264.45 | 485.72 | 194.55 | 167.43 | 176.85 | 157.92 | 185.87 | 191.37 |
| 23166 | STAB1 | 161.92 | 111.78 | 150.14 | 170.8 | 190.98 | 219.39 | 144.43 | 301.11 | 260.52 | 81.386 |
| 206358 | SLC36A1 | 69.352 | 112.18 | 167.49 | 162.58 | 136.7 | 242.86 | 134.87 | 81.805 | 73.633 | 100.04 |
| 913 | CD1E | 23.446 | 31.112 | 24.51 | 19.626 | 10.493 | 18.039 | 17.494 | 38.018 | 79.575 | 21.279 |
| 713 | C1QB | 57.155 | 68.223 | 38.545 | 24.608 | 93.249 | 62.42 | 33.698 | 50.778 | 36.277 | 18.398 |
| 712 | C1QA | 62.558 | 24.101 | 23.867 | 20.628 | 32.379 | 49.396 | 11.998 | 83.199 | 44.593 | 74.948 |
| 910 | CD1B | 116.44 | 64.685 | 43.037 | 23.273 | 75.038 | 76.53 | 60.891 | 184.23 | 121.28 | 202.85 |
| 913 | CD1E | 76.574 | 73.689 | 34.628 | 34.141 | 101.33 | 67.955 | 48.996 | 74.338 | 72.827 | 86.424 |
| 714 | C1QC | 110.65 | 140.74 | 27.702 | 61.741 | 34.068 | 71.675 | 33.642 | 118.86 | 56.562 | 125.48 |
| 5480 | PPIC | 47.172 | 48.238 | 37.09 | 16.31 | 46.384 | 27.873 | 72.802 | 76.562 | 100.23 | 28.991 |
| 945 | CD33 | 23.86 | 26.068 | 11.41 | 9.258 | 25.716 | 20.375 | 14.645 | 37.029 | 35.662 | 17.374 |
| 909 | CD1A | 334.06 | 215.3 | 239.04 | 192.86 | 595.37 | 330.27 | 283.03 | 765.57 | 729.57 | 334.33 |
| 2 | A2M | 56.154 | 90.243 | 91.964 | 68.09 | 88.213 | 61.413 | 70.048 | 110.28 | 217.55 | 116.79 |
| 6357 | CCL 13 | 103.8 | 102.11 | 70.412 | 68.673 | 165.5 | 111.1 | 53.946 | 134.15 | 164.72 | 100.83 |
| 1193 | CLIC2 | 19.734 | 13.509 | 4.991 | 7.239 | 27.693 | 27.378 | 16.056 | 17.633 | 17.894 | 9.958 |
| 5577 | PRKAR2B | 109.78 | 78.126 | 61.402 | 92.489 | 117.09 | 64.795 | 78.53 | 96.114 | 111.03 | 96.123 |
| 6614 | SIGLEC1 | 23.821 | 16.503 | 13.074 | 11.874 | 18.704 | 14.555 | 17.02 | 27.273 | 27.555 | 13.05 |
| 80380 | PDCD1LG2 | 16.213 | 14.334 | 24.966 | 7.891 | 14.958 | 12.758 | 13.968 | 20.981 | 22.152 | 13.788 |
| 942 | CD86 | 183.62 | 162.09 | 106.74 | 90.275 | 293.85 | 267.82 | 183.33 | 618.49 | 400.78 | 281.29 |
| 629 | CFB | 136.58 | 132.94 | 46.834 | 56.07 | 131.35 | 83.048 | 43.087 | 129.28 | 79.264 | 59.938 |
| 5055 | SERPINB2 | 104.82 | 108.06 | 56.517 | 32.963 | 71.147 | 96.217 | 41.219 | 113.71 | 115.17 | 71.027 |
| 55022 | PID1 | 68.932 | 25.883 | 27.311 | 36.14 | 27.062 | 43.915 | 33.14 | 85.735 | 108.6 | 45.643 |
| 2921 | CXCL3 | 24.788 | 25.074 | 21.049 | 26.677 | 53.966 | 18.063 | 38.137 | 56.646 | 50.331 | 87.434 |
| 6374 | CXCL5 | 32.322 | 5.255 | 14.219 | 3.561 | 33.529 | 12.421 | 21.868 | 49.028 | 37.239 | 15.912 |
| 2919 | CXCL 1 | 102.29 | 72.235 | 49.323 | 38.146 | 109.13 | 94.808 | 78.084 | 156.84 | 130.59 | 91.55 |
| 3552 | IL1A | 88.623 | 145.24 | 90.422 | 35.318 | 155.24 | 63.8 | 81.281 | 287.8 | 220.03 | 111.54 |
| 718 | C3 | 59.883 | 26.757 | 34.401 | 17.188 | 28.973 | 26.25 | 17.639 | 27.042 | 25.763 | 39.066 |
| 6369 | CCL24 | 44.054 | 21.628 | 24.646 | 14.316 | 57.382 | 49.296 | 26.492 | 25.721 | 26.113 | 19.318 |
| 3624 | INHBA | 16.237 | 135.48 | 7.304 | 7.813 | 26.306 | 14.216 | 20.578 | 20.333 | 18.133 | 8.488 |
| 8875 | VNN2 | 269.9 | 77.17 | 513.82 | 12.6 | 355.24 | 189.21 | 167.27 | 287.16 | 314.52 | 168.83 |
| 1441 | CSF3R | 106.53 | 44.825 | 45.942 | 38.679 | 176.05 | 82.909 | 41.943 | 74.399 | 38.012 | 29.283 |
| 64407 | RGS18 | 348.74 | 282.09 | 132.72 | 112.99 | 257.38 | 151.54 | 93.752 | 264.46 | 229.04 | 186.15 |

**Table 8B**

| ***Deconvolution basis signature*** | | | | | | | | |
|---|---|---|---|---|---|---|---|---|
| ***Adaptive immune cell subsets*** | | | | | | | | |
| ***ENTREZ ID*** | ***Symbol*** | ***NK*** | ***NK activated*** | ***Monocyte*** | ***Activated monocytes*** | ***mDC*** | ***mDC activated*** | ***Neutrophil*** |
| | | 234.22 | 193.38 | 60.43 | 94.563 | 44.379 | 42.333 | 528.59 |
| 83481 | EPPK1 | 22.594 | 25.374 | 15.907 | 23.304 | 17.794 | 14.671 | 49.81 |
| 678 | ZFP36L2 | 944.54 | 829.88 | 947.23 | 303.25 | 768 | 224.49 | 685.29 |
| | | 118.91 | 74.872 | 49.12 | 36.745 | 26.144 | 19.642 | 24.981 |
| 4929 | NR4A2 | 16.994 | 108.1 | 62.992 | 220.11 | 49.329 | 100.96 | 106.7 |
| | | 102.67 | 116.49 | 189.11 | 128.72 | 103 | 82.624 | 72.219 |
| 26289 | AK5 | 199.32 | 175.49 | 27.348 | 33.242 | 26.782 | 42.736 | 114.44 |
| 3707 | ITPKB | 2300 | 1248 | 134.46 | 135.6 | 290.04 | 94.781 | 610.74 |
| | | 192.38 | 340.33 | 209.66 | 263.31 | 151.63 | 182.99 | 1779.9 |
| 9241 | NOG | 108.3 | 65.022 | 67.638 | 107.4 | 66.928 | 68.087 | 245.57 |
| 3337 | DNAJB1 | 913.3 | 898.93 | 1674.3 | 1546.3 | 1153.8 | 890.5 | 815.13 |
| 2935 | GSPT1 | 460.41 | 371.28 | 148.31 | 138.81 | 121.14 | 117.47 | 234.13 |
| | | 773.73 | 774.08 | 460.05 | 327.9 | 234.19 | 363.43 | 1545.7 |
| 4929 | NR4A2 | 167.22 | 211.2 | 153.38 | 325.82 | 153.57 | 127.94 | 217.77 |
| 90139 | TSPAN18 | 93.089 | 110.13 | 83.97 | 121.68 | 134.12 | 124.52 | 230.54 |
| 146330 | FBXL16 | 311.67 | 323.29 | 224.19 | 245.56 | 351.13 | 401.22 | 1170.8 |
| 678 | ZFP36L2 | 893.78 | 701.58 | 1109.2 | 276.4 | 869.58 | 278.41 | 706.3 |
| 112744 | IL 17F | 132.08 | 57.788 | 66.779 | 62.439 | 48.914 | 67.21 | 192.07 |
| 3605 | IL 17A | 6.173 | 21.649 | 12.938 | 15.326 | 13.615 | 19.02 | 18.389 |
| 1493 | CTLA4 | 280.2 | 272.35 | 127.25 | 202.8 | 219.86 | 213.03 | 546.89 |
| 1493 | CTLA4 | 105.53 | 117.46 | 74.196 | 74.198 | 166.69 | 144.29 | 185.74 |
| 940 | CD28 | 54.782 | 99.024 | 69.36 | 108.31 | 103.85 | 99.261 | 313.67 |
| 51339 | DACT1 | 56.953 | 114.38 | 164.18 | 108.19 | 238.82 | 129.36 | 226.99 |
| 50616 | IL22 | 130.08 | 140.77 | 160.41 | 208.94 | 195.76 | 176.95 | 458.47 |
| 143686 | SESN3 | 479.75 | 251.81 | 70.653 | 94.283 | 209.79 | 790.54 | 79.465 |
| 128553 | TSHZ2 | 169.31 | 130.05 | 81.735 | 121.52 | 97.362 | 109.71 | 253.85 |
| 145864 | HAPLN3 | 563.5 | 2809.2 | 254.6 | 430.41 | 267.09 | 1769.6 | 644.6 |
| 30812 | SOX8 | 39.268 | 45.119 | 15.518 | 25.178 | 50.898 | 22.052 | 25.256 |
| 940 | CD28 | 65.611 | 35.191 | 29.141 | 49.231 | 19.951 | 27.802 | 37.221 |
| 1493 | CTLA4 | 124.66 | 116.43 | 92.171 | 92.422 | 121.75 | 109.83 | 501.04 |
| 10320 | IKZF 1 | 571.49 | 213.94 | 254.88 | 233.08 | 271.55 | 439.89 | 210.19 |
| 29968 | PSAT1 | 199.65 | 207.83 | 202.32 | 287.5 | 581.74 | 510.86 | 477.33 |
| 3578 | IL9 | 95.923 | 63.72 | 52.85 | 60.811 | 68.764 | 52.046 | 88.46 |
| 128553 | TSHZ2 | 102.72 | 72.86 | 28.29 | 63.443 | 42.089 | 40.373 | 163.89 |
| 926 | CD8B | 66.688 | 29.283 | 42.361 | 57.728 | 46.062 | 30.886 | 58.733 |
| 54674 | LRRN3 | 46.41 | 34.332 | 99.24 | 152.85 | 121.16 | 87.014 | 107.64 |
| 925 | CD8A | 1747.3 | 1021.5 | 176.49 | 358.05 | 191.57 | 212.95 | 659.97 |
| 926 | CD8B | 239.29 | 169.83 | 138.51 | 232.77 | 128.76 | 116.27 | 422.64 |
| 54674 | LRRN3 | 111.6 | 105.81 | 175.1 | 267.95 | 204.23 | 201.49 | 495.58 |
| 51676 | ASB2 | 31.89 | 23.954 | 18.171 | 22.51 | 23.759 | 68.321 | 32.828 |
| 9666 | DZIP3 | 35.794 | 21.865 | 21.678 | 28.811 | 39.256 | 44.789 | 30.134 |
| 10730 | YME1L1 | 23.72 | 13.456 | 19.854 | 21.666 | 14.435 | 20.995 | 53.299 |
| 85315 | PAQR8 | 945.21 | 671.94 | 472.6 | 224.57 | 882.61 | 233.68 | 556.69 |
| 9402 | GRAP2 | 189.79 | 247.42 | 18.973 | 24.599 | 22.105 | 22.97 | 27.632 |
| 2833 | CXCR3 | 602.71 | 345.71 | 49.709 | 78.357 | 64.907 | 28.424 | 62.056 |
| 3820 | KLRB1 | 278.26 | 253.8 | 41.45 | 39.372 | 63.174 | 30.798 | 241.79 |
| 4676 | NAP1L4 | 1028.9 | 774.41 | 1198.6 | 1045.5 | 896.23 | 1096.3 | 451.85 |
| 1731 | 1-Sep | 1159.2 | 975.22 | 21.243 | 61.854 | 26.316 | 69.847 | 148.47 |
| 814 | CAMK4 | 139.73 | 101.14 | 25.46 | 75.621 | 50.817 | 142.6 | 204.37 |
| 57124 | CD248 | 213.8 | 213.93 | 317.03 | 255.8 | 371.85 | 289.84 | 453.31 |
| | | 49.748 | 27.014 | 31.759 | 55.044 | 32.022 | 36.939 | 217.53 |
| 283869 | NPW | 4.098 | 4.461 | 7.843 | 11.752 | 8.392 | 9.127 | 7.039 |
| 199953 | TMEM201 | 134.75 | 135.83 | 100.88 | 112.42 | 107.51 | 102.01 | 213.66 |
| 399694 | SHC4 | 21.764 | 31.468 | 18.077 | 29.599 | 26.114 | 25.334 | 60.573 |
| 3976 | LIF | 20.468 | 84.797 | 104.49 | 625.31 | 55.962 | 48.533 | 66.225 |
| 23176 | 8-Sep | 13.231 | 10.118 | 20.444 | 23.372 | 23.637 | 21.66 | 24.944 |
| 990 | CDC6 | 80.572 | 41.998 | 52.73 | 66.45 | 58.133 | 41.306 | 272.02 |
| 51010 | EXOSC3 | 499.31 | 691.75 | 285.35 | 269.96 | 183.8 | 226.82 | 93.13 |
| 1021 | CDK6 | 1248.6 | 830.99 | 475.77 | 235.09 | 1131.8 | 1444.6 | 318.97 |
| 51293 | CD320 | 113.03 | 29.97 | 59.036 | 145.06 | 75.737 | 63.493 | 39.181 |
| 1503 | CTPS1 | 223.21 | 303.98 | 430.55 | 239.9 | 573.47 | 332.52 | 293.59 |
| 84319 | CMSS1 | 427.68 | 588.48 | 366.89 | 183.34 | 348.61 | 236.71 | 368.07 |
| 10622 | POLR3G | 65.923 | 66.872 | 62.421 | 96.927 | 95.214 | 73.507 | 98.346 |
| 199953 | TMEM201 | 115.85 | 116.68 | 56.981 | 51.74 | 109.25 | 33.679 | 89.197 |
| 1021 | CDK6 | 830.37 | 552.98 | 397.79 | 296.18 | 766.85 | 720.85 | 518.97 |
| 1841 | DTYMK | 178.37 | 112.05 | 210.83 | 157.39 | 223.51 | 179.2 | 45.123 |
| 23464 | GCAT | 17.535 | 9.065 | 24.936 | 33.227 | 23.741 | 24.881 | 37.288 |
| 3336 | HSPE1 | 605.63 | 767.69 | 1753.7 | 1231 | 2327 | 1763.6 | 188.67 |
| | | 60.648 | 39.065 | 34.083 | 55.897 | 28.843 | 37.901 | 114.38 |
| 971 | CD72 | 140.01 | 70.636 | 86.43 | 25.64 | 98.177 | 38.692 | 20.212 |
| 933 | CD22 | 17.766 | 12.93 | 105.23 | 68.891 | 59.415 | 28.518 | 31.877 |
| | | 70.721 | 45.52 | 19.657 | 34.482 | 25.068 | 27.183 | 27.135 |
| 6328 | SCN3A | 9.035 | 16.917 | 20.771 | 16.759 | 15.008 | 14.52 | 30.192 |
| 84518 | CNFN | 13.975 | 11.258 | 14.451 | 23.411 | 16.705 | 19.943 | 27.996 |
| 8115 | TCL1A | 180.04 | 170.35 | 119.83 | 297.68 | 185.86 | 228.53 | 348.28 |
| 29802 | VPREB3 | 61.713 | 94.775 | 90.293 | 96.477 | 78.715 | 97.26 | 123.4 |
| 79856 | SNX22 | 44.03 | 51.925 | 27.59 | 49.441 | 36.764 | 35.319 | 111.72 |
| 115123 | 3-Mar | 39.875 | 54.774 | 28.204 | 37.371 | 32.484 | 30.718 | 88.844 |
| 8115 | TCL1A | 286.46 | 251.84 | 247 | 312.07 | 191.15 | 185.85 | 392.58 |
| | | 407.7 | 331.35 | 113.97 | 184.05 | 84.152 | 122.42 | 456.53 |
| | | 31.909 | 47.045 | 11.475 | 22.142 | 16.219 | 13.423 | 120.6 |
| 283663 | LINC00926 | 265.95 | 221.32 | 914.55 | 1401.3 | 239.32 | 423.65 | 219.41 |
| 283663 | LINC00926 | 476.24 | 428.56 | 669.75 | 1095.6 | 309.15 | 406.34 | 897.64 |
| 933 | CD22 | 243.9 | 251.42 | 697.13 | 618.3 | 649.58 | 454.74 | 714.06 |
| 55278 | QRSL1 | 354.9 | 363.68 | 464.82 | 337.89 | 791.38 | 630.87 | 176.49 |
| 94235 | GNG8 | 16.072 | 16.477 | 17.853 | 61.13 | 14.916 | 18.059 | 41.406 |
| 23089 | PEG10 | 54.027 | 25.162 | 34.173 | 64.445 | 37.859 | 21.48 | 90.119 |
| 7782 | SLC30A4 | 805.39 | 1089.5 | 85.611 | 279.09 | 740.4 | 422.82 | 497.14 |
| | | 122.51 | 111.59 | 513.67 | 723.39 | 598.57 | 235.17 | 29.861 |
| | | 91.025 | 91.24 | 45.43 | 58.428 | 42.262 | 45.095 | 185.27 |
| 148932 | MOB3C | 1533.5 | 3005.6 | 1422 | 1492.7 | 1051.1 | 937.14 | 980.55 |
| 80237 | ELL3 | 132.33 | 109.34 | 241.61 | 198.86 | 252.73 | 569.53 | 256.98 |
| 1184 | CLCN5 | 628.32 | 554.57 | 767.28 | 555.77 | 891.8 | 452.43 | 1058.2 |
| 653121 | ZBTB8A | 133.21 | 138.24 | 97.11 | 73.722 | 383.62 | 117.5 | 352.18 |
| | | 98.693 | 36.689 | 294.02 | 279.9 | 911.37 | 208.28 | 38.002 |
| 1184 | CLCN5 | 123.85 | 106.44 | 244.96 | 215.04 | 243.33 | 146.17 | 260.36 |
| 94274 | PPP1R14A | 11.632 | 11.142 | 9.568 | 26.704 | 96.818 | 610.68 | 22.289 |
| 80237 | ELL3 | 146.24 | 169.55 | 197.8 | 185.91 | 202.84 | 505.74 | 244.67 |
| 1490 | CTGF | 15.781 | 21.534 | 11.813 | 18.578 | 17.114 | 41.291 | 35.862 |
| 148932 | MOB3C | 829.5 | 1255.6 | 710.25 | 607.66 | 533.21 | 447.56 | 630.08 |
| 140733 | MACROD2 | 79.989 | 66.195 | 42.426 | 76.844 | 58.566 | 40.426 | 140.71 |
| 116449 | CLNK | 144.92 | 97.92 | 42.783 | 47.617 | 43.627 | 37.469 | 164 |
| 51237 | MZB1 | 52.632 | 104.09 | 19.182 | 52.139 | 32.979 | 18.784 | 67.427 |
| 3514 | IGKC | 15.836 | 17.732 | 23.55 | 32.221 | 33.423 | 26.31 | 32.073 |
| 3537 | IGLC1 | 22.518 | 18.29 | 18.087 | 22.137 | 20.973 | 43.664 | 43.749 |
| | | 92.062 | 77.439 | 21.719 | 38.212 | 25.846 | 35.352 | 64.939 |
| 81618 | ITM2C | 542.99 | 323.12 | 33.527 | 83.342 | 50.067 | 43.392 | 129.57 |
| 51237 | MZB1 | 16.949 | 17.981 | 29.661 | 37.977 | 33.723 | 31.289 | 40.291 |
| 608 | TNFRSF 17 | 25.957 | 9.114 | 21.037 | 21.426 | 26.508 | 35.455 | 32.888 |
| | | 146.08 | 117.63 | 154.76 | 164.45 | 135.49 | 142.59 | 353.57 |
| 96610 | BMS1P20 | 76.908 | 111.4 | 74.646 | 119.1 | 83.672 | 98.779 | 225.79 |
| | | 44.886 | 41.648 | 29.585 | 73.693 | 31.832 | 47.005 | 75.732 |
| 51303 | FKBP11 | 951.93 | 1748.7 | 153.69 | 225.52 | 152.68 | 102.36 | 170.24 |
| | | 28.589 | 30.469 | 25.767 | 36.928 | 23.977 | 25.044 | 43.659 |
| | | 14.155 | 11.369 | 21.694 | 31.114 | 22.223 | 19.143 | 23.746 |
| | | 43.581 | 136.18 | 116.63 | 117.3 | 107.23 | 160.4 | 243.01 |
| 79694 | MANEA | 85.751 | 124.28 | 111.99 | 82.277 | 196.15 | 182.86 | 5.879 |
| 28823 | IGLV1-44 | 31.014 | 20.293 | 21.717 | 30.131 | 21.158 | 20.484 | 70.638 |
| 857 | CAV1 | 14.734 | 23.886 | 23.042 | 136.13 | 51.28 | 156.41 | 46.812 |
| 10316 | NMUR1 | 172.26 | 28.295 | 24.576 | 43.296 | 32.587 | 28.674 | 25.14 |
| 2043 | EPHA4 | 2761.4 | 735.14 | 25.869 | 40.016 | 27.074 | 27.474 | 82.083 |
| 10079 | ATP9A | 948.85 | 203.48 | 40.959 | 55.259 | 160.09 | 41.003 | 27.762 |
| 9289 | ADGRG1 | 5945.4 | 1929.6 | 50.639 | 140.34 | 46.329 | 72.247 | 180.88 |
| 2043 | EPHA4 | 1388.8 | 396.3 | 25.82 | 30.08 | 30.872 | 25.258 | 53.499 |
| 79901 | CYBRD1 | 121.89 | 7.789 | 21.734 | 21.921 | 33.831 | 21.927 | 19.441 |
| | | 535.44 | 147.87 | 18.017 | 23.31 | 13.838 | 26.439 | 28.242 |
| 151742 | PPM1L | 1220.4 | 304.67 | 157.85 | 96.387 | 413.32 | 116.43 | 274.48 |
| 81563 | C1orf21 | 2150.6 | 903.11 | 84.932 | 390.11 | 31.397 | 23.844 | 159.08 |
| 2619 | GAS1 | 178.7 | 35.304 | 17.643 | 34.257 | 42.159 | 26.652 | 44.204 |
| 59338 | PLEKHA1 | 7208.2 | 2925.4 | 617.2 | 652.31 | 909.71 | 1482.1 | 442.03 |
| 2043 | EPHA4 | 2364.5 | 967.61 | 145.92 | 239.82 | 138.98 | 172.69 | 440 |
| 4068 | SH2D1A | 1693 | 746.65 | 51.221 | 51.22 | 39.309 | 41.259 | 66.209 |
| 2043 | EPHA4 | 3974.9 | 1121.7 | 19.352 | 28.697 | 24.173 | 24.334 | 157.95 |
| 2774 | GNAL | 193.61 | 90.085 | 39.564 | 54.834 | 62.992 | 63.343 | 84.722 |
| 5243 | ABCB1 | 1488.5 | 773.91 | 78.088 | 55.655 | 48.71 | 46.583 | 298.63 |
| 11098 | PRSS23 | 2740.9 | 775.99 | 92.684 | 108.22 | 107.02 | 101.46 | 407.85 |
| 127254 | ERICH3 | 96.537 | 5221.1 | 20.035 | 16.235 | 31.973 | 19.899 | 76.112 |
| 57489 | ODF2L | 469.39 | 2852.3 | 29.954 | 88.016 | 51.95 | 64.323 | 111.68 |
| 57489 | ODF2L | 401.76 | 2054.4 | 33.763 | 61.725 | 39.819 | 107.49 | 86.458 |
| 257019 | FRMD3 | 284.1 | 1797.8 | 111.72 | 76.125 | 133.01 | 202.21 | 294.68 |
| 10974 | ADIRF | 11.696 | 225.5 | 19.308 | 40.143 | 28.946 | 25.319 | 23.581 |
| 6672 | SP100 | 990.02 | 3884 | 572.42 | 755.13 | 276.02 | 661.01 | 630.04 |
| 64108 | RTP4 | 836.07 | 6115.6 | 346.26 | 438.55 | 577.13 | 1663.7 | 64.385 |
| 55603 | FAM46A | 750.31 | 2775.4 | 406.81 | 129.88 | 401.53 | 597.47 | 211.98 |
| 54809 | SAMD9 | 4316.6 | 19829 | 471.56 | 584.64 | 274.6 | 2650.4 | 1593.1 |
| 257019 | FRMD3 | 223.91 | 3689.8 | 94.875 | 77.308 | 140.16 | 266.24 | 748.24 |
| 91624 | NEXN | 115.11 | 2299.9 | 17.823 | 28.107 | 16.964 | 51.955 | 64.69 |
| | | 21.641 | 2082.2 | 39.556 | 27.231 | 22.498 | 96.451 | 70.775 |
| 85363 | TRIM5 | 431.34 | 1212.8 | 328.47 | 236.23 | 326.44 | 417.5 | 213.38 |
| 50650 | ARHGEF3 | 4649.5 | 10693 | 808.73 | 521.37 | 1982.5 | 1260 | 265.32 |
| 100131733 | USP30-AS1 | 430.83 | 2257.5 | 109.55 | 72.205 | 85.077 | 413.35 | 267.85 |
| | | 259.52 | 4757.8 | 25.85 | 56.1 | 24.379 | 190.18 | 128.97 |
| 2635 | GBP3 | 3614.4 | 9689.2 | 235.89 | 408.95 | 477.37 | 1973.1 | 442.26 |
| 5654 | HTRA1 | 182.02 | 100.41 | 4524.5 | 721.17 | 344.37 | 393.46 | 408.27 |
| 2048 | EPHB2 | 36.082 | 33.919 | 750 | 37.251 | 66.137 | 111.51 | 40.488 |
| 10461 | MERTK | 155.64 | 143.83 | 2057.5 | 444.14 | 347.38 | 196.23 | 221.36 |
| 4048 | LTA4H | 2278 | 832.7 | 21704 | 2697.1 | 4330.1 | 1290.8 | 1902.3 |
| 2048 | EPHB2 | 54.592 | 51.961 | 1348.1 | 30.996 | 332.32 | 326.9 | 110.81 |
| 10461 | MERTK | 126.88 | 50.745 | 3131.1 | 768.85 | 422.35 | 132.67 | 247.23 |
| 340526 | RGAG4 | 112.3 | 123.43 | 933.54 | 233.76 | 297.81 | 126.5 | 147.74 |
| 284013 | VMO1 | 362.26 | 11.41 | 2665.1 | 396.29 | 97.002 | 261.12 | 21.038 |
| 120939 | TMEM52B | 72.249 | 101.61 | 821.29 | 191.39 | 106.19 | 115.88 | 143.71 |
| 408 | ARRB1 | 144 | 87.986 | 591.46 | 169.9 | 179.32 | 77.117 | 79.13 |
| 2048 | EPHB2 | 117.7 | 98.776 | 1139.8 | 190.47 | 393.61 | 362.3 | 378.24 |
| 2517 | FUCA1 | 2269 | 1457.2 | 23621 | 1428.6 | 7804.2 | 2832.3 | 348.69 |
| | | 149.94 | 79.806 | 2508.8 | 1320.1 | 1363.3 | 601.22 | 135.64 |
| 2335 | FN1 | 366.11 | 53.143 | 16242 | 58.443 | 2883.5 | 2906 | 165.13 |
| 11326 | VSIG4 | 205.15 | 68.283 | 3604.2 | 370.12 | 157.55 | 209.64 | 455.5 |
| 2335 | FN1 | 698.9 | 104.41 | 19697 | 64.194 | 4322.9 | 4511 | 73.793 |
| 51063 | CALHM2 | 874.44 | 374.83 | 2336.4 | 451.57 | 899.63 | 218.93 | 379.34 |
| 55244 | SLC47A1 | 18.268 | 31.408 | 34.706 | 31.818 | 1294.5 | 62.187 | 37.893 |
| 2162 | F13A1 | 199.86 | 162.12 | 301.41 | 267.62 | 9213.4 | 463.63 | 663.21 |
| 10462 | CLEC10A | 242.18 | 51.339 | 666.23 | 247.07 | 10346 | 234.87 | 219.09 |
| 246 | ALOX15 | 27.642 | 27.956 | 26.953 | 29.212 | 4539.9 | 72.346 | 182.96 |
| 23475 | QPRT | 27.911 | 18.443 | 39.471 | 38.229 | 3854.3 | 108.41 | 77.585 |
| 154092 | LINC01010 | 23.433 | 33.679 | 57.564 | 49.195 | 1113.4 | 122.32 | 43.176 |
| 23017 | FAIM2 | 18.124 | 15.246 | 38.708 | 44.951 | 1986 | 47.632 | 35.496 |
| 79839 | CCDC 102B | 16.601 | 9.295 | 16.732 | 22.858 | 224.25 | 24.244 | 10.698 |
| 5445 | PON2 | 434.92 | 340.34 | 276.18 | 103.43 | 5310.8 | 766.1 | 141.11 |
| 30835 | CD209 | 118.57 | 188.32 | 365.69 | 835.46 | 7460.1 | 742.95 | 370.95 |
| 51477 | ISYNA1 | 172.47 | 121.04 | 178.53 | 181.87 | 2664.7 | 426.65 | 120.38 |
| 30835 | CD209 | 40.149 | 53.679 | 90.689 | 154.74 | 1321.4 | 166.3 | 140.49 |
| 2878 | GPX3 | 289.86 | 166.4 | 2820.8 | 773.89 | 17689 | 2021.5 | 666.64 |
| 2878 | GPX3 | 290.97 | 235.63 | 1813.8 | 724.23 | 10819 | 1542.4 | 612.69 |
| 5445 | PON2 | 188.76 | 161.04 | 168.75 | 41.48 | 3833.2 | 579.46 | 69.345 |
| 56670 | SUCNR1 | 166.02 | 133.08 | 158.29 | 517.6 | 4885.7 | 822.66 | 343.58 |
| 30850 | CDR2L | 15.938 | 20.626 | 54.75 | 40.053 | 904.74 | 77.118 | 39.601 |
| 11067 | C10orf10 | 24.176 | 45.143 | 33.926 | 63.693 | 95.817 | 2692.8 | 36.858 |
| 6624 | FSCN1 | 81.53 | 523.84 | 136.01 | 184.31 | 670.32 | 17366 | 98.237 |
| 54662 | TBC1D13 | 98.944 | 262.08 | 96.11 | 188.52 | 289.66 | 5087.5 | 56.742 |
| 101930114 | LOC101930114 | 150.12 | 378.01 | 86.24 | 121.02 | 294.11 | 15782 | 284.28 |
| 5157 | PDGFRL | 28.375 | 53.462 | 47.721 | 49.514 | 53.746 | 1387.7 | 20.595 |
| 3429 | IFI27 | 134.04 | 1488.9 | 210.18 | 325.75 | 138.05 | 17765 | 151.87 |
| | | 74.727 | 153.21 | 53.552 | 33.817 | 95.649 | 8820.3 | 107.24 |
| 80045 | GPR157 | 990.63 | 1117.5 | 941.2 | 500.12 | 390.29 | 9736.3 | 730.17 |
| 80380 | PDCD1LG2 | 21.387 | 27.365 | 24.291 | 29.793 | 122.17 | 1974.3 | 53.788 |
| 11067 | C10orf10 | 76.594 | 37.144 | 76.86 | 55.657 | 65.272 | 1186.5 | 89.081 |
| 8820 | HESX1 | 13.973 | 59.359 | 33.421 | 25.657 | 40.231 | 1691.5 | 22.801 |
| 6624 | FSCN1 | 126.4 | 620.29 | 171.77 | 344.98 | 1454.9 | 16899 | 52.809 |
| 11167 | FSTL1 | 93.606 | 105.83 | 192.63 | 274.55 | 188.9 | 4875.7 | 381.91 |
| 54662 | TBC1D13 | 582 | 689.43 | 770.72 | 899.75 | 1147.7 | 7639.7 | 681.05 |
| 9175 | MAP3K13 | 9844 | 6.678 | 11.997 | 17.73 | 14.728 | 173.95 | 11.757 |
| 3357 | HTR2B | 44.103 | 23.668 | 20.289 | 20.805 | 23.311 | 815.72 | 23.146 |
| 94015 | TTYH2 | 403.48 | 548.11 | 194.02 | 123.57 | 283.43 | 7052.4 | 203.15 |
| 56300 | IL36G | 75.673 | 95.566 | 193.5 | 13364 | 96.662 | 140.58 | 158.08 |
| 3036 | HAS1 | 52.122 | 23.743 | 85.531 | 6520.7 | 125.32 | 73.135 | 144.91 |
| 7980 | TFPI2 | 14.302 | 264.75 | 228.32 | 10802 | 22.39 | 274.11 | 37.116 |
| 11009 | IL24 | 89.089 | 27.311 | 98.807 | 7265 | 37.723 | 43.914 | 230.31 |
| 4312 | MMP1 | 14.214 | 3.569 | 15.962 | 2464.7 | 22.158 | 26.477 | 38.896 |
| 7980 | TFPI2 | 31.933 | 59772 | 146.36 | 3886.6 | 28.623 | 142.85 | 22.053 |
| 1440 | CSF3 | 47.925 | 24.496 | 27.293 | 2163.5 | 30.881 | 31.705 | 36.188 |
| 3569 | IL6 | 97.655 | 1336.6 | 1632.4 | 34971 | 204.43 | 2510.5 | 240.88 |
| 4233 | MET | 15.715 | 16.009 | 360.15 | 4883.1 | 27.917 | 148.52 | 23.036 |
| 169792 | GLIS3 | 13.486 | 37.029 | 185.27 | 1833 | 46.519 | 15.028 | 127.91 |
| 51334 | PRR16 | 19.619 | 10.144 | 72.589 | 812.71 | 19.002 | 17.112 | 43.945 |
| 6374 | CXCL5 | 9.621 | 15.056 | 175.25 | 1677.9 | 40.346 | 43.681 | 99.159 |
| 6660 | SOX5 | 9.393 | 10.435 | 26.932 | 296.21 | 30.578 | 32.724 | 14.187 |
| 79931 | TNIP3 | 13.75 | 34.176 | 660.08 | 8318.6 | 49.416 | 180.07 | 35.132 |
| 8710 | SERPINB7 | 26.193 | 269.05 | 48.317 | 8770.5 | 32.96 | 803.26 | 45.827 |
| 3690 | ITGB3 | 82.961 | 102.15 | 50.385 | 2003.3 | 53.605 | 172.65 | 112.65 |
| 5743 | PTGS2 | 22.052 | 57.425 | 434.21 | 23846 | 79.633 | 293.33 | 2414 |
| 8794 | TNFRSF10C | 23.394 | 13.984 | 40.419 | 75.161 | 46.084 | 27.389 | 7494 |
| 53829 | P2RY13 | 15.617 | 12.05 | 22.463 | 29.8 | 106.12 | 24.156 | 7096 |
| 4311 | MME | 45.207 | 25.599 | 56.171 | 108.62 | 66.398 | 51.768 | 5834.4 |
| 146225 | CMTM2 | 260.95 | 291.93 | 161.55 | 189.59 | 204.06 | 184.9 | 41610 |
| 8794 | TNFRSF10C | 95.751 | 43.935 | 27.844 | 33.083 | 31.309 | 39.868 | 11321 |
| 8794 | TNFRSF10C | 69.516 | 66.842 | 124.52 | 98.004 | 132 | 127.39 | 7001.1 |
| 6286 | S100P | 35.679 | 40.597 | 196.07 | 287.74 | 71.192 | 50.918 | 21441 |
| 3577 | CXCR1 | 150.19 | 36.452 | 38.777 | 47.861 | 34.59 | 34.741 | 6702.9 |
| 60675 | PROK2 | 27.394 | 7.688 | 94.419 | 58.926 | 11.755 | 29.657 | 13698 |
| 54682 | MANSC1 | 32.466 | 53.104 | 75.79 | 54.837 | 60.247 | 61.276 | 2613.4 |
| 144423 | GLT1D1 | 97.688 | 90.763 | 406.39 | 290.12 | 29.735 | 19.451 | 12243 |
| 3579 | CXCR2 | 392.19 | 100.84 | 111.39 | 20.749 | 453.51 | 83.791 | 12028 |
| 25984 | KRT23 | 36.835 | 49.514 | 68.24 | 67.635 | 90.373 | 68.849 | 3796.8 |
| 2215 | FCGR3B | 901.02 | 693.72 | 1388 | 330.29 | 466.68 | 231.55 | 32749 |
| 4311 | MME | 18.525 | 13.907 | 21.308 | 93.096 | 27.296 | 25.716 | 2580 |
| 79908 | BTNL8 | 19.269 | 15.886 | 38.278 | 55.531 | 50.442 | 42.432 | 1195.4 |
| | | 56.534 | 90.265 | 162.63 | 125.69 | 139.7 | 144.21 | 2915.3 |
| | | 43.711 | 36.304 | 28.181 | 36.962 | 26.687 | 31.636 | 101.33 |
| 80201 | HKDC1 | 26.961 | 45.619 | 23.442 | 29.542 | 32.629 | 24.274 | 58.015 |
| | | 10.559 | 20.107 | 16.677 | 28.563 | 25.355 | 25.463 | 33.51 |
| 3572 | IL6ST | 18.648 | 75.83 | 17.295 | 19.91 | 12.723 | 20.567 | 66.204 |
| 6711 | SPTBN1 | 40.456 | 30.066 | 29.822 | 33.681 | 25.029 | 28.475 | 88.178 |
| 6920 | TCEA3 | 116.74 | 24.173 | 42.689 | 80.724 | 58.11 | 61.04 | 198.9 |
| | | 125.04 | 63.024 | 38.942 | 29.201 | 84.617 | 132.45 | 136.94 |
| 26119 | LDLRAP1 | 512.32 | 232.72 | 316.47 | 283.63 | 570.71 | 174.09 | 140.7 |
| | | 1183.6 | 722.03 | 174.49 | 97.182 | 94.599 | 65.5 | 229.94 |
| 3562 | IL3 | 9.531 | 7.443 | 16.465 | 22.998 | 20.151 | 21.977 | 18.31 |
| 50616 | IL22 | 86.691 | 40.447 | 29.994 | 57.904 | 25.763 | 21.935 | 91.888 |
| 64788 | LMF1 | 146.1 | 153.11 | 42.787 | 41.659 | 142.33 | 46.938 | 114.22 |
| | | 149.76 | 123.63 | 72.559 | 162.63 | 116.99 | 71.606 | 208.72 |
| 959 | CD40LG | 40.464 | 49.649 | 34.919 | 30.912 | 38.454 | 28.851 | 88.189 |
| 50943 | FOXP3 | 127.15 | 81.559 | 32.344 | 77.866 | 46.594 | 46.038 | 122.99 |
| 54602 | NDFIP2 | 370.94 | 370.3 | 183.16 | 259.95 | 348.39 | 267.85 | 711.33 |
| 1493 | CTLA4 | 9094 | 48.237 | 37.288 | 50.691 | 101.61 | 64.781 | 132.91 |
| 55423 | SIRPG | 105.48 | 80.092 | 107.77 | 138.53 | 127.52 | 151.82 | 220.38 |
| 917 | CD3G | 477.55 | 288.48 | 44.127 | 84.826 | 45.794 | 46.494 | 83.39 |
| 10663 | CXCR6 | 406.47 | 375.57 | 152.71 | 177.96 | 163.36 | 154.32 | 292.65 |
| 3090 | HIC1 | 190.21 | 119.43 | 47.963 | 42.613 | 46.522 | 177.53 | 73.215 |
| 27240 | SIT1 | 199.48 | 132.05 | 101.21 | 123.34 | 407.85 | 119.45 | 174.4 |
| 51676 | ASB2 | 274.66 | 272.28 | 135.17 | 143.8 | 94.268 | 172.17 | 376.9 |
| 91978 | TPGS1 | 53.805 | 54.227 | 136.66 | 121.66 | 142.86 | 168.85 | 43.394 |
| 28755 | TRAC | 786.27 | 699.43 | 305.97 | 565.94 | 236.82 | 296.6 | 844.63 |
| 3932 | LCK | 2360.8 | 993.94 | 268.64 | 389.46 | 154.42 | 148.7 | 415.79 |
| 79413 | ZBED2 | 130.91 | 87.84 | 77.241 | 139.93 | 117.45 | 87.879 | 257.46 |
| 993 | CDC25A | 12.808 | 8.661 | 21.27 | 35.54 | 28.316 | 18.804 | 23.371 |
| 151230 | KLHL23 | 86.999 | 91.376 | 27.185 | 36.326 | 19.515 | 22.162 | 51.824 |
| 29128 | UHRF1 | 184.73 | 95.148 | 68.223 | 51.556 | 36.754 | 33.832 | 223.99 |
| | | 137.58 | 95.521 | 32.602 | 48.19 | 34.163 | 57.902 | 99.755 |
| 29089 | UBE2T | 137.04 | 110.9 | 41.216 | 64.275 | 62.825 | 50.933 | 53.295 |
| 3070 | HELLS | 6.926 | 6.673 | 16.983 | 29.67 | 25.008 | 24.039 | 28.993 |
| 8438 | RAD54L | 55.063 | 35.177 | 56.263 | 47.656 | 48.391 | 46.296 | 57.866 |
| 3070 | HELLS | 176.76 | 154 | 34.591 | 65.708 | 34.752 | 50.621 | 298.73 |
| 10563 | CXCL13 | 28.792 | 94.868 | 25.984 | 158.1 | 27.05 | 242.1 | 41.323 |
| 79075 | DSCC1 | 61.206 | 23.793 | 101.91 | 136.67 | 98.441 | 95.428 | 61.939 |
| 4049 | LTA | 18.138 | 118.74 | 20.557 | 29.577 | 27.044 | 28.376 | 32.132 |
| 10328 | EMC8 | 29.093 | 23.251 | 36.363 | 39.324 | 34.294 | 22.123 | 37.046 |
| 84824 | FCRLA | 45.306 | 35.399 | 33.046 | 52.991 | 28.414 | 16.273 | 85.514 |
| | | 45.323 | 42.148 | 14.306 | 59.328 | 16.55 | 23.564 | 65.166 |
| 3899 | AFF3 | 278.65 | 183.3 | 30.892 | 227.31 | 28.426 | 22.836 | 36.823 |
| 55024 | BANK1 | 87.887 | 93.53 | 35.155 | 98.414 | 81.002 | 42.046 | 96.465 |
| 931 | MS4A1 | 161.02 | 229.51 | 115.45 | 406.1 | 130.5 | 190.71 | 468.78 |
| | | 188.29 | 83.886 | 44.602 | 109.46 | 38.954 | 42.475 | 172.67 |
| 931 | MS4A1 | 225.68 | 328.16 | 265.38 | 525.55 | 198.48 | 283.11 | 571.83 |
| 199786 | FAM129C | 702.73 | 706.12 | 256.69 | 301.58 | 217.31 | 334.95 | 1098.2 |
| 115350 | FCRL1 | 108.17 | 132.07 | 64.362 | 69.389 | 74.8 | 93.924 | 289.88 |
| | | 191.43 | 156.86 | 97.122 | 121.18 | 138.05 | 110.89 | 567.87 |
| 931 | MS4A1 | 80.829 | 114.15 | 101.59 | 113.37 | 101.97 | 103.15 | 253.83 |
| 931 | MS4A1 | 326.13 | 440.54 | 128.31 | 417.2 | 81.803 | 158.62 | 326.6 |
| 53335 | BCL11A | 278.77 | 344.03 | 188.3 | 1126.3 | 142.9 | 935.66 | 524.55 |
| 26040 | SETBP1 | 182.44 | 205.55 | 75.283 | 105.32 | 347.1 | 146.98 | 127.52 |
| 53335 | BCL11A | 51.388 | 86.743 | 146.78 | 603.73 | 84.55 | 594.52 | 212.77 |
| | | 6.244 | 13.577 | 15.288 | 17.852 | 17.773 | 16.565 | 14.048 |
| | | 8.998 | 9.784 | 20.519 | 27.983 | 23.596 | 15.285 | 39.095 |
| | | 22.09 | 30.102 | 44.255 | 58.244 | 47.213 | 46.657 | 53.954 |
| 3514 | IGKC | 25.025 | 27.54 | 26.512 | 58.57 | 29.03 | 25.412 | 43.521 |
| 857 | CAV1 | 32.745 | 47.461 | 43.329 | 83.562 | 51.181 | 114.21 | 120.86 |
| | | 156.03 | 222.68 | 145.78 | 228.12 | 167.6 | 153.98 | 378.45 |
| 100379345 | MIR181A2HG | 1357.6 | 1006 | 18.416 | 25.441 | 20.915 | 20.658 | 43.551 |
| 53637 | S1PR5 | 636.77 | 472.14 | 16.048 | 20.789 | 17.434 | 18.811 | 19.978 |
| 53637 | S1PR5 | 1050.1 | 856.24 | 18.334 | 27.73 | 21.672 | 26.124 | 41.053 |
| 53637 | S1PR5 | 10394 | 9089.6 | 32.645 | 56.232 | 50.011 | 38.707 | 78.204 |
| 9231 | DLG5 | 593.45 | 203.75 | 22.159 | 47.671 | 29.067 | 22.446 | 35.15 |
| 90102 | PHLDB2 | 3883.5 | 2824.8 | 57.803 | 75.66 | 44.914 | 112.46 | 259.01 |
| 79899 | PRR5L | 1486.1 | 742.55 | 54.253 | 57.897 | 48.31 | 112.77 | 211.31 |
| 7049 | TGFBR3 | 4462.7 | 3660.1 | 148.41 | 166.84 | 82.078 | 112.41 | 324.45 |
| 1524 | CX3CR1 | 11626 | 7080.3 | 846.43 | 55.954 | 46.831 | 65.088 | 703.38 |
| 51348 | KLRF1 | 7620.2 | 5981.3 | 75.985 | 74.24 | 100.25 | 97.755 | 204.09 |
| 5775 | PTPN4 | 6757.7 | 6224.2 | 130.69 | 179.31 | 485.75 | 147.81 | 541.49 |
| 5775 | PTPN4 | 3242.9 | 2902.9 | 90.022 | 88.882 | 109.14 | 78.403 | 328.46 |
| 7049 | TGFBR3 | 14704 | 12273 | 167.22 | 280.79 | 134 | 140.35 | 1264.7 |
| 83888 | FGFBP2 | 37240 | 11588 | 30.563 | 76.403 | 31.688 | 27.257 | 521.22 |
| 114879 | OSBPL5 | 2031.1 | 2266.5 | 101.74 | 59.833 | 139.28 | 77.974 | 127.23 |
| 219285 | SAMD9L | 2468.3 | 25077 | 389.99 | 535.76 | 573.04 | 4217.1 | 849.73 |
| 54877 | ZCCHC2 | 2777.4 | 13364 | 2181.8 | 2110.6 | 3070.1 | 2447.8 | 1979.3 |
| 356 | FASLG | 1767.8 | 4539.7 | 170.68 | 255.05 | 115.16 | 146.5 | 379.82 |
| 5920 | RARRES3 | 7967.4 | 15536 | 142.96 | 272 | 114.99 | 681.38 | 388.82 |
| 388228 | SBK1 | 1362.5 | 3717.5 | 45.33 | 75.229 | 66.294 | 84.376 | 443.53 |
| 219285 | SAMD9L | 2070.9 | 15246 | 608.57 | 541.2 | 934.09 | 3897.5 | 1137.3 |
| 2219 | FCN1 | 671.24 | 314.21 | 12909 | 835.39 | 177.32 | 267.64 | 5507.7 |
| 9332 | CD163 | 263.89 | 77.313 | 9682.4 | 8620.1 | 827.99 | 239.35 | 265.16 |
| 23601 | CLEC5A | 1117.4 | 316.18 | 19857 | 11344 | 2299.7 | 896.82 | 347.84 |
| 9332 | CD163 | 241.85 | 36.597 | 8883.9 | 7051.5 | 908.66 | 212.78 | 175.95 |
| 51313 | FAM198B | 437.88 | 202.42 | 4948.7 | 822.28 | 4023.9 | 569.82 | 309.26 |
| 23166 | STAB1 | 600.3 | 293.96 | 10839 | 1936.2 | 8595.5 | 1317.1 | 174.1 |
| 10501 | SEMA6B | 107.53 | 73.612 | 1094.7 | 910.27 | 52.127 | 143.82 | 30.585 |
| 7045 | TGFBI | 3343 | 793.16 | 31162 | 3217 | 25407 | 5132.9 | 103.22 |
| 8536 | CAMK1 | 336.18 | 168.74 | 4099.8 | 349.29 | 3162 | 432.58 | 573.02 |
| 23166 | STAB 1 | 705.38 | 383.12 | 13007 | 3057.7 | 9961.8 | 2083.4 | 329.11 |
| 206358 | SLC36A1 | 249.87 | 161.79 | 2534.6 | 715.27 | 1603.8 | 653.16 | 330.92 |
| 913 | CD1E | 40.886 | 10.775 | 97.286 | 121.26 | 16075 | 5709.7 | 69.112 |
| 713 | C1QB | 77.011 | 102.19 | 182.48 | 146.48 | 10592 | 7856.7 | 41.177 |
| 712 | C1QA | 50.142 | 86.203 | 184.27 | 108.09 | 7282.4 | 2928.5 | 76.151 |
| 910 | CD1B | 106.28 | 55.634 | 90.609 | 383.87 | 17114 | 4264.6 | 170.68 |
| 913 | CD1E | 115.69 | 80.099 | 81.505 | 94.16 | 6752.7 | 1417.6 | 118.17 |
| 714 | C1QC | 139.21 | 130.21 | 495.99 | 88.076 | 12507 | 11043 | 156.99 |
| 5480 | PPIC | 26.824 | 12.981 | 32.336 | 55.873 | 707.73 | 310.34 | 39.174 |
| 945 | CD33 | 19.578 | 19.477 | 679.76 | 121.04 | 1593.4 | 133.38 | 60.619 |
| 909 | CD1A | 236.08 | 255.17 | 374.32 | 400.57 | 17758 | 3408.6 | 715.59 |
| 2 | A2M | 212.65 | 114.82 | 533.47 | 194.51 | 14937 | 15010 | 110.11 |
| 6357 | CCL13 | 157.64 | 289.58 | 242.42 | 455.08 | 6434.5 | 11487 | 174.82 |
| 1193 | CLIC2 | 115.54 | 209.36 | 190.72 | 234.49 | 3590.7 | 8375.9 | 22.283 |
| 5577 | PRKAR2B | 108.68 | 84.149 | 166.4 | 144.73 | 669.88 | 3171.9 | 167.26 |
| 6614 | SIGLEC1 | 29.371 | 89.235 | 275.86 | 294.87 | 710.24 | 6005.1 | 49.332 |
| 80380 | PDCD1LG2 | 33.651 | 23.536 | 50.538 | 60.569 | 174.05 | 1520.5 | 26.35 |
| 942 | CD86 | 137.07 | 318.48 | 630.29 | 242.14 | 2070.3 | 8560 | 203.25 |
| 629 | CFB | 110.84 | 127.86 | 139.77 | 1128.5 | 252.42 | 4969.9 | 187.81 |
| 5055 | SERPINB2 | 83.1 | 95.581 | 4287.5 | 24461 | 98.613 | 352.89 | 181.87 |
| 55022 | PID1 | 154.69 | 54.538 | 7487.2 | 11205 | 34.221 | 105.68 | 78.269 |
| 2921 | CXCL3 | 63.616 | 22.828 | 3914.7 | 27356 | 131.59 | 522.63 | 73.882 |
| 6374 | CXCL5 | 20.307 | 12.939 | 4063.1 | 33167 | 20.269 | 445.88 | 102.86 |
| 2919 | CXCL1 | 86.887 | 118.64 | 6214.2 | 35585 | 100.46 | 1191.3 | 1368.5 |
| 3552 | IL1A | 112.61 | 77.232 | 2599.8 | 22595 | 213.69 | 660.82 | 323.06 |
| 718 | C3 | 62.908 | 45.944 | 5647.8 | 8096.8 | 246.51 | 282.92 | 49.628 |
| 6369 | CCL24 | 235.72 | 198.21 | 9587.6 | 13839 | 113.99 | 365.61 | 68.75 |
| 3624 | INHBA | 20.287 | 104.69 | 536.09 | 11449 | 171.22 | 4927.5 | 19.921 |
| 8875 | VNN2 | 499.61 | 320.66 | 223.25 | 4735.8 | 44.71 | 72.248 | 19069 |
| 1441 | CSF3R | 24.289 | 44.759 | 819.72 | 100.75 | 190.43 | 109.83 | 10132 |
| 64407 | RGS18 | 315.04 | 200.03 | 374.78 | 94.765 | 1421.8 | 103.53 | 10444 |

Tables 8A-B describe the data of the deconvolution basis signature matrix from *(26)* that was used by the present inventors to estimate immune cell subset proportions in all discovery cohorts. The present inventors used the version provided by the *CellMix* package *(35).* Rows are Affymetrix HG-U133plusV2 probesets, with the first 4 columns providing the probeset ID and the corresponding ENTREZ gene ID, gene symbol and description (if available), as mapped using Bioconductor annotation package *hgu133plus2.db.* The remaining 17 columns contain the reference expression profiles for each cell subset, which are detailed in Table 10 herein below.

Table 9 herein below, describes the results of the meta-analysis performed on the 3 discovery cohorts. Each row contains the results of testing differences in the proportions of a given cell type in a given cohort between responders and non-responders to the treatment with the Infliximab TNF-alpha inhibitor. The quantity tested was the log2 fold change log2 (Responder/Non-responder). The columns provide the following information:
*Cohort:* cohort ID; *Cell type:* cell type name; *CI. low:* lower bound of the 95% confidence interval of the estimated proportion difference; *CI. up:* upper bound of the 95% confidence interval of the estimated proportion difference; *estimate:* estimated (pseudo-)median proportion difference; *p. value:* nominal p-value for Wilcoxon rank sum test; *Fstat:* Fisher combined probability statistic; *Fpvalue:* nominal p-value for Fisher combined probability test; *Ffdr:* false discovery rate obtained by adjusting *Fpvalue* with Benjamini and Hochberg procedure; *Significance:* significance flag for the nominal Wilcoxon p-values as used in Figure 4.

### Table 9

**Table 9. "mono act" = M1 Macrophage.**

| ***Cohort*** | ***Cell type*** | ***CI. low*** | ***CI. up*** | ***estimate*** | ***p. value*** | ***Fstat*** | ***Fpvalue*** | ***Ffdr*** | ***Significance*** |
|---|---|---|---|---|---|---|---|---|---|
| GSE16879 | PC | -2.008935664 | -0.334124253 | -0.802445899 | 0.005239343 | 18.70073489 | 0.000899793 | 0.004349001 | ≤ 0.05 |
| GSE16879 | mono act | -1.921136072 | -0.555524195 | -1.138916477 | 0.005907491 | 25.07777803 | 4.85303E-05 | 0.000469126 | ≤ 0.05 |
| GSE12251 | mono act | -1.40090001 | -0.146123505 | -0.801394223 | 0.015871126 | 25.07777803 | 4.85303E-05 | 0.000469126 | ≤ 0.05 |
| GSE12251 | mono | 0.083431836 | 0.834009379 | 0.387433072 | 0.020580039 | 11.72217689 | 0.019541354 | 0.047224938 | ≤ 0.05 |
| GSE14580 | PC | -1.711129042 | -0.110985781 | -0.969678819 | 0.022970314 | 18.70073489 | 0.000899793 | 0.004349001 | ≤ 0.05 |
| GSE14580 | DC act | 0.070788646 | 0.848562982 | 0.428528154 | 0.022970314 | 13.38778995 | 0.009528501 | 0.030702949 | ≤ 0.05 |
| GSE14580 | Mem IgM | -4.410775103 | -0.443741036 | -1.876461659 | 0.024455936 | 8.217074503 | 0.083942424 | 0.115920491 | ≤ 0.05 |
| GSE14580 | NK act | 0.158764344 | 1.114754472 | 0.614506832 | 0.029177221 | 7.643379983 | 0.105550528 | 0.133085448 | ≤ 0.05 |
| GSE14580 | Tc | 0.022416538 | 2.518080552 | 0.891074973 | 0.036817534 | 9.070169168 | 0.059369317 | 0.114780679 | ≤ 0.05 |
| GSE14580 | mono act | -1.562598302 | -0.071899857 | -0.685699358 | 0.038231283 | 25.07777803 | 4.85303E-05 | 0.000469126 | ≤ 0.05 |
| GSE12251 | neutro | -1.006490965 | -0.005595542 | -0.409687906 | 0.042570433 | 8.368643695 | 0.078970351 | 0.115920491 | ≤ 0.05 |
| GSE14580 | Tc act | -0.049689147 | 2.400520526 | 1.304423587 | 0.079325765 | 6.093702195 | 0.192258968 | 0.223020403 | NS |
| GSE12251 | DC act | -0.150356751 | 0.693861677 | 0.242245285 | 0.140237472 | 13.38778995 | 0.009528501 | 0.030702949 | NS |
| GSE16879 | mono | -0.315416999 | 0.833763874 | 0.307548902 | 0.226839724 | 11.72217689 | 0.019541354 | 0.047224938 | NS |
| GSE12251 | Tc | -0.836041001 | 2.245527305 | 0.689010828 | 0.303696304 | 9.070169168 | 0.059369317 | 0.114780679 | NS |
| GSE16879 | B aIgM | -0.847957179 | 2.815656569 | 0.850008944 | 0.372727273 | 3.653317908 | 0.454951927 | 0.488652069 | NS |
| GSE16879 | DC act | -0.189549359 | 0.648479142 | 0.160272986 | 0.384456617 | 13.38778995 | 0.009528501 | 0.030702949 | NS |
| GSE14580 | B aIgM | -1.77844547 | 4.402900973 | 1.401951715 | 0.431818182 | 3.653317908 | 0.454951927 | 0.488652069 | NS |
| GSE16879 | neutro | -0.557117307 | 0.627022737 | 0.21094443 | 0.482416448 | 8.368643695 | 0.078970351 | 0.115920491 | NS |
| GSE12251 | DC | -1.099228501 | 0.972877023 | 0.273246793 | 0.548961874 | 1.199452572 | 0.87818874 | 0.891865528 | NS |
| GSE12251 | NK | -0.757662222 | 2.273034201 | 0.285909939 | 0.572603867 | 1.115122267 | 0.891865528 | 0.891865528 | NS |
| GSE16879 | Tc act | -0.959908605 | 1.106714795 | 0.239515529 | 0.598901099 | 6.093702195 | 0.192258968 | 0.223020403 | NS |
| GSE14580 | mono | -0.451452948 | 0.77132921 | 0.23122276 | 0.610093396 | 11.72217689 | 0.019541354 | 0.047224938 | NS |
| GSE12251 | PC | -0.767490557 | 0.396927657 | 0.044086237 | 0.722342673 | 18.70073489 | 0.000899793 | 0.004349001 | NS |
| GSE14580 | neutro | -0.351036164 | 0.636440896 | 0.094296973 | 0.741723331 | 8.368643695 | 0.078970351 | 0.115920491 | NS |
| GSE16879 | NK act | -0.980713316 | 0.729732326 | -0.112269758 | 0.750269339 | 7.643379983 | 0.105550528 | 0.133085448 | NS |
| GSE16879 | Mem IgM | -2.998462888 | 1.445422883 | -0.231855628 | 0.818181818 | 8.217074503 | 0.083942424 | 0.115920491 | NS |
| GSE12251 | Mem IgM | -1.08237294 | 0.875547513 | -0.086327202 | 0.821203564 | 8.217074503 | 0.083942424 | 0.115920491 | NS |
| GSE16879 | Tc | -2.490998581 | 2.028537181 | 0.099652984 | 0.959276018 | 9.070169168 | 0.059369317 | 0.114780679 | NS |

### EXAMPLE 3

### IMMUNE CELL TYPES ANALYZED

Table 10 herein below provides the cell type of each subpopulation which can be analyzed (short name or symbol, and cell description), the cell separation method, and the characteristics markers.

### Table 10

**Table 10.**

| ***Symbol*** | ***Cell Type Description*** | ***Cell Separation Method*** | ***Markers*** |
|---|---|---|---|
| Th | Resting helper T cells | RosetteSep CD4+ T-cell enrichment cocktail | CD45RA-high; CD4+; CD45RO- |
| Th act | Activated helper T cells | Plate-bound anti-CD3 and anti-CD28 | |
| Tc | Resting cytotoxic T cells | RosetteSep CD8+ T-cell enrichment cocktail | CD45RA+; CD8+; CD45RO- |
| Tc act | Activated cytotoxic T cells | Plate-bound anti-CD3 and anti-CD28 | |
| B | Resting B cells | MACS CD138 microbeads and CD19 microbeads | CD19+; CD27-; IgG/A- |
| B act | Activated B cells | Anti-CD40 and IL4, 23 hours | |
| B aIgM | BCR-ligated B cells | Anti-IgM, 24 hours | |
| Mem IgG | IgA/IgG memory B cells | sorted CD19+/CD27+/IgM- | CD19+; CD27+; IgM- |
| Mem IgM | IgM memory B cells | sorted CD19+/CD27+/IgG/A- | CD19+; CD27+; IgG/A- |
| PC | Plasma cells | MACS CD138 microbeads and FACS | CD20 FITC, CD138 PE and CD19 APC |
| NK | Resting NK cells | RosetteSep NK-cell enrichment cocktail plus CD2 microbeads | |
| NK act | Activated NK cells | IL2, 16 hours | |
| mono | Monocytes | MACS CD14 microbeads | |
| mono act M1 macrophages | Activated Monocytes/Macrophages differentiated from monocytes | LPS, 24 hours | M1: CD68, CD86, CCR7; |
| M2 macrophages | | | M2: CD68, CD163, CD206 (MR); |
| DC | Resting dendritic cells | Differentiated from monocytes with IL4 and GMCSF | |
| DC act | Activated dendritic cells | LPS, 24 hours | |
| neutro | Neutrophils | Ficoll gradient centrifugation of heparanized blood | |
| | | | |

Table 11 describes the frequencies of the subpopulation of cells in TNF-alpha inhibitor responders versus non-responders.

### Table 11

**Table 11. Confidence intervals (95% CI) and non-overlapping exemplary ranges [representative (Repr.) range] of proportions estimated by computational deconvolution for cell types that showed significant differences in at least one of the discovery cohorts, and optimal cutoff for the plasma cell clinician index (PC-index) and automated quantitation quantitative score (PC-score) from immunostaining in the validation cohort.**

| ***Cohort*** | ***Cell type*** | ***Non-responders 95% CI*** | ***Responders 95% CI*** | ***Non-responders Repr. range*** | ***Responders Repr. range*** |
|---|---|---|---|---|---|
| GSE16879 | PC | 0.082 - 0.261 | 0.052 - 0.105 | 0.105 - 0.261 | 0.052 - 0.082 |
| GSE16879 | mono act M1 | 0.062 - 0.113 | 0.024 - 0.059 | 0.062 - 0.113 | 0.024 - 0.059 |
| GSE12251 | mono act M1 | 0.089 - 0.171 | 0.052 - 0.094 | 0.094 - 0.171 | 0.052 - 0.089 |
| GSE12251 | mono | 0.057 - 0.079 | 0.073 - 0.129 | 0.057 - 0.073 | 0.079 - 0.129 |
| GSE14580 | PC | 0.123 - 0.264 | 0.057 - 0.145 | 0.145 - 0.264 | 0.057 - 0.123 |
| GSE14580 | DC act | 0.193 - 0.292 | 0.282 - 0.350 | 0.193 - 0.282 | 0.292 - 0.350 |
| GSE14580 | Mem IgM | 0.059 - 0.165 | 0.002 - 0.091 | 0.091 - 0.165 | 0.002 - 0.059 |
| GSE14580 | NK act | 0.052 - 0.095 | 0.073 - 0.139 | 0.052 - 0.073 | 0.095 - 0.139 |
| GSE14580 | Tc | 0.011 - 0.031 | 0.017 - 0.050 | 0.011 - 0.017 | 0.031 - 0.050 |
| GSE14580 | mono act M1 | 0.088 - 0.130 | 0.026 - 0.101 | 0.101 - 0.130 | 0.026 - 0.088 |
| GSE12251 | neutro | 0.115 - 0.212 | 0.089 - 0.135 | 0.135 - 0.212 | 0.089 - 0.115 |
| Validation | PC - Index | ≥ 2 | ≤ 1 | | |
| Validation | PC - Score | ≥ 0.056 | < 0.056 | | |

### EXAMPLE 4

The present inventors have surprisingly uncovered that the predictive power of the gene signatures of some aspects of the disclosure is much higher when the inflammation status of the tissue is accounted for. The present inventors have assessed the training set predictive power as single cellular biomarkers by ROC analysis in each GEO cohort separately. Activated monocyte proportions achieved high AUC values in all cohorts (AUC = 77%, 82% and 89% in the *UC-A*, *UC-B* and *CDc* cohorts respectively). Plasma cell proportions performed similarly well in cohorts *UC-A* and *CDc* (AUC = 79% and 88% respectively), but gave a weaker signal in cohort *UC-B* (AUC = 45%), which was expected since proportion differences were not found significant in this cohort in first place. In exploratory cohorts UC-A and CDc the collected tissues were all from inflamed mucosa sites, as opposed to cohort UC-B wherein the tissue samples included both normal and inflamed biopsies. Hence, the ROC curves for UC-A and CDc have a much higher % AUC than the UC-B cohort.

The present inventors have carried out an additional validation, whereby the present inventors included a cohort of normal and inflamed biopsy samples from IBD patients. Plasma cell numbers from non inflamed biopsies of 9 non responders and 20 responders were collected, and from inflamed tissue sites of 7 responders and 5 non-responders prior to anti-TNF therapy initiation. Thus, as shown in Figure 12, the plasma cell proportions in inflamed colon tissue can predict response to infliximab (IFX) prior to treatment initiation with high and unprecedented accuracy.

It should be noted that a mixed tissue biopsy (i.e., having inflamed and non-inflamed cells) is sufficient for determining the responsiveness of the subject to anti-TNF therapy based on frequencies of macrophages and plasma cells in some cohorts.

In addition, it should be noted that a tissue biopsy from an inflamed area, e.g., which includes mainly inflamed cells, is sufficient for determining the responsiveness of the subject to anti-TNF therapy based on frequencies of plasma cells and macrophages.

### Analysis and Discussion

The treatment of IBDs using monoclonal antibodies against TNF-alpha has shown to be very effective in achieving complete mucosal remission, however only in 60% of patients *(3).* This high failure rate, together with the unavailability of a reliable test to predict response, the high cost of anti-TNF biologics and many major side effects on the patients' immune system greatly undermine the benefit/cost ratio of such an otherwise effective therapy. In this work, the present inventors used a cell-centered approach based on computational methods to elucidate cell subsets whose proportions can predict response to anti-TNF therapy in IBD patients, prior starting treatment. By validating the present inventors' findings in paraffin embedded stained biopsies the present inventors show that such prediction is easily possible in a clinical setting.

Previous attempts to find predictive biomarkers used gene expression assays on bulk colon biopsies *(4, 5).* Traditional analysis of gene expression data look for genes that show differential expression patterns between conditions. However, due to both technical and biological variability, gene-based signatures are commonly difficult to reproduce. In this context, looking at functionally coordinated modules such as pathways or co-expression network is known to greatly improve robustness of findings. In a similar way, cells can be considered as the fundamental functional units whose coordinated gene expression programs are regulated according to conditions and stimuli. In disease conditions, in particular, immune cell subsets home to target tissues where they may turn to fight the cause of disease or in the worst scenario exacerbate the existing pathology if their actions are dis-regulated. This is all the more the case for inflammatory diseases such as IBD where immune activity has a role in pathogenesis. This inflammatory process involves interaction between different subsets of immune cells as well as cross talk with cells of the gut tissue through cytokine signaling, overall forming a complex dynamic system *(17).* The present inventors' approach identified immune cells as major contributors to gene signatures of colon tissue in IBD. Thus, the present inventors focused efforts on looking for biomarkers within the main actors of this system, i.e. the variety of immune cell subsets. For this reason, the present inventors expect these predictions to be more robust and reproducible than gene/pathway based biomarkers. An additional advantage of this cell-centered approach lays in the interpretability of the results, because they directly point to specific cell subsets, from which it is easier to derive immunological and mechanistic hypotheses. Last but not least, cell subset proportions are easily and accurately assayed in clinical settings, for example in the routinely stored biopsies in the case of IBD. In an in-silico discovery phase, the present inventors used computational deconvolution techniques to estimate the proportions of infiltrating immune cell subsets in colon tissues directly from public gene expression data of bulk tissue. While batch effects, tissue or disease heterogeneity makes proportion estimates from separate cohorts not directly comparable, group differences in proportions (fold change) within each cohort are comparable and indicative of differential immune compartment *(27).* By formally integrating these observed differences across multiple cohorts, the present inventors were able to capture the most consistent signal within a heterogeneous technical and biological background, in a similar way as gene-based meta-analysis are performed *(19, 24).* The present inventors' approach detected that non-responders have consistently greater proportions of activated monocytes and plasma cells than responders. When validating these finding, the present inventors found that macrophage proportions were not predictive of response, although showing the most consistent differences across all discovery cohorts. This may be due to a discrepancy between the resolution of their in-silico estimates and their assessment in the stained biopsies. Indeed, the reference gene expression profile used to estimate the proportion of activated monocytes was generated from monocytes 24 hours after *invitro* stimulation with LPS *(26),* which would qualify them as classically activated macrophages (M1). These are also known as inflammatory macrophages, due to their secretion of pro-inflammatory cytokines such as TNFα, IL-1β, IL-6 and IL-12 *(36).* The present inventors are currently investigating if M1 or M2 macrophages proportions could indeed provide accurate response prediction. However, these two cell subsets are thought to be the two extreme of a continuum phenotype, with their respective markers being rather quantitative than binary. This may prevent their distinction by staining, and require more advanced technology like flow-cytometry which are not directly implementable in routine clinical protocols. Nonetheless, the predictive power of plasma cells is remarkable. Moreover, immunostaining for CD138+ cells can be done using antibodies that are known to be very specific and efficient on this cell population, which presents the additional characteristics of being also distinguishable by morphology. Overall, this promises to provide a robust and accurate prediction clinical assay.

Infliximab has been shown to induce monocyte apoptosis in patients with chronic active CD, which could explain its strong anti-inflammatory effect *(28).* Basal plasmacytosis, defined as a dense infiltration of plasma cells in the lower one third of the mucosa *(29),* is considered to be an early feature of IBD *(30).* The presence of basal plasmacytosis in colon biopsies of UC patients has notably been identified as an independent predictor of shorter time to clinical relapse *(29).*

It is well known that dysregulation of various immune cell populations can be seen in the gut of patients with IBD. Their inflamed gut may become massively infiltrated with B cells alongside with IgA+ and IgG+ plasma cells, depending on the severity of inflammation, though the mechanisms of this recruitment are not fully clear *(31-33).* In this context, it is believed that the intestinal microbiota plays a key role in driving inflammatory responses during disease development and progression. Palm et al investigated the involvement of mucosal IgA (secreted by plasma cells) in IBD gut barrier function, and have shown that bacteria taxa-specific levels of IgA might distinguish between members of the microbiota that impact disease susceptibility and/or severity, and the remaining members of the microbiota *(37)* emphasizing the role of IgA+ mucosal plasma cells in gut homeostasis and disease. In UC, plasma cells also produce non-specific Antibodies, such as perinuclear anti-cytoplasmic neutrophil (pANCA) *(38).* Absence of this antibody was strongly associated with better response to Infliximab *(39,40).*

IgG antibodies are the most abundant serum immunoglobulins, involved in the secondary immune response, and their numbers increase in response to infection, chronic inflammation, and autoimmune diseases *(41,42).* IgG-producing plasma cells heavily infiltrate the inflamed mucosa of patients with IBD. It was suggested that IgG plasma cells create immune complexes (IC) with their specific antigens. This IgG-IC activates intestinal macrophages via their FcyRs, and exacerbating intestinal inflammation, demonstrating plasma cell-macrophage cooperation as another potent inducer of intestinal inflammation besides commensal bacteria. Recently, FcγRIIA was also identified as a susceptible gene of UC in Japanese and European descent populations*(43,44).* In vitro IgG-IC stimulation caused increasing number of macrophages in the inflamed mucosa of UC patients, and induced the extensive production of pro-inflammatory cytokines such as TNF, IL-1β and IL-6. In addition, neutrophil expression of FcyRI is upregulated in adult patients with clinically active IBD *(45).* The high numbers of plasma cells together with activated monocytes in the present inventors' predictive signature can point to involvement of this signaling pathway by lamina propria mononuclear cells (LPMCs) *(46).*

The present inventors validated these results for plasma cells in a completely independent set of 20 IBD samples (UC, CD, IBDU) by staining biopsy slides for CD138 positive cells. Proportions obtained by automated quantitation achieved very high accuracy (AUC 82.4%).

Taken together, the present inventors' predictive assay is easily applicable in clinical settings and can dramatically improve the cost/benefit of anti-TNF therapy prescription for IBD patients. In future, a similar approach will be tested to achieve a higher resolution insight into the nature of macrophage subsetting in IBD biopsies, to derive an additional predictive value from biopsies obtained routinely prior to anti-TNF therapy initiation.

### References

### (Additional references are cited in text)

1. S. B. Hanauer, B. G. Feagan, G. R. Lichtenstein, L. F. Mayer, S. Schreiber, J. F. Colombel, D. Rachmilewitz, D. C. Wolf, A. Olson, W. Bao, P. Rutgeerts, Maintenance infliximab for Crohn's disease: The ACCENT I randomised trial, Lancet 359, 1541-1549 (2002).
2. G. R. Lichtenstein, S. Yan, M. Bala, M. Blank, B. E. Sands, Infliximab maintenance treatment reduces hospitalizations, surgeries, and procedures in fistulizing Crohn's disease, Gastroenterology 128, 862-869 (2005).
3. S. Ben-Horin, U. Kopylov, Y. Chowers, Optimizing anti-TNF treatments in inflammatory bowel disease, Autoimmunity Reviews 13, 24-30 (2014).
4. I. Arijs, K. Li, G. Toedter, R. Quintens, L. Van Lommel, K. Van Steen, P. Leemans, G. De Hertogh, K. Lemaire, M. Ferrante, F. Schnitzler, L. Thorrez, K. Ma, X.-Y. R. Song, C. Marano, G. Van Assche, S. Vermeire, K. Geboes, F. Schuit, F. Baribaud, P. Rutgeerts, Mucosal gene signatures to predict response to infliximab in patients with ulcerative colitis., Gut 58, 1612-9 (2009).
5. I. Arijs, R. Quintens, L. Van Lommel, K. Van Steen, G. De Hertogh, K. Lemaire, A. Schraenen, C. Perrier, G. Van Assche, S. Vermeire, K. Geboes, F. Schuit, P. Rutgeerts, Predictive value of epithelial gene expression profiles for response to infliximab in Crohn's disease., Inflammatory bowel diseases 16, 2090-8 (2010).
6. Q. Chen, L. Rabach, P. Noble, T. Zheng, C. G. Lee, R. J. Homer, J. A. Elias, IL-11 receptor alpha in the pathogenesis of IL-13-induced inflammation and remodeling., Journal of immunology (Baltimore, Md. : 1950) 174, 2305-13 (2005).
7. A. J. Ashcroft, S. M. Cruickshank, P. I. Croucher, M. J. Perry, S. Rollinson, J. M. Lippitt, J. A. Child, C. Dunstan, P. J. Felsburg, G. J. Morgan, S. R. Carding, Colonic dendritic cells, intestinal inflammation, and T cell-mediated bone destruction are modulated by recombinant osteoprotegerin., Immunity 19, 849-61 (2003).
8. A. J. Ashcroft, S. R. Carding, RANK ligand and osteoprotegerin: emerging roles in mucosal inflammation., Gut 54, 1345-6 (2005).
9. P. Mannon, W. Reinisch, Interleukin 13 and its role in gut defence and inflammation., Gut 61, 1765-73 (2012).
10. H. Kefalakes, T. J. Stylianides, G. Amanakis, G. Kolios, Exacerbation of inflammatory bowel diseases associated with the use of nonsteroidal anti-inflammatory drugs: myth or reality?, European journal of clinical pharmacology 65, 963-70 (2009).
11. J. L. Wallace, Prostaglandin biology in inflammatory bowel disease., Gastroenterology clinics of North America 30, 971-80 (2001).
12. C. Chen, M. S. Jamaluddin, S. Yan, D. Sheikh-Hamad, Q. Yao, Human stanniocalcin-1 blocks TNF-alpha-induced monolayer permeability in human coronary artery endothelial cells., Arteriosclerosis, thrombosis, and vascular biology 28, 906-12 (2008).
13. B. Mesko, S. Poliska, A. Váncsa, Z. Szekanecz, K. Palatka, Z. Hollo, A. Horvath, L. Steiner, G. Zahuczky, J. Podani, A. Nagy, Peripheral blood derived gene panels predict response to infliximab in rheumatoid arthritis and Crohn's disease, Genome Medicine 5, 59 (2013).
14. B. Khor, A. Gardet, R. J. Xavier, Genetics and pathogenesis of inflammatory bowel disease, Nature 474, 307-317 (2011).
15. M. C. Dubinsky, L. Mei, M. Friedman, T. Dhere, T. Haritunians, H. Hakonarson, C. Kim, J. Glessner, S. R. Targan, D. P. McGovern, K. D. Taylor, J. I. Rotter, Genome wide association (GWA) predictors of anti-TNFalpha therapeutic responsiveness in pediatric inflammatory bowel disease., Inflammatory bowel diseases 16, 1357-66 (2010).
16. M. K. Magnusson, H. Strid, M. Sapnara, A. Lasson, A. Bajor, K.-A. Ung, L. Öhman, Anti-TNF therapy response in patients with ulcerative colitis is associated with colonic anti-microbial peptide expression and microbiota composition, Journal of Crohn's and Colitis, jjw051 (2016).
17. C. Abraham, R. Medzhitov, Interactions between the host innate immune system and microbes in inflammatory bowel disease, Gastroenterology 140, 1729-1737 (2011).
18. S. S. Shen-Orr, R. Gaujoux, Computational deconvolution: extracting cell type-specific information from heterogeneous samples., Current opinion in immunology 25, 571-8 (2013).
19. R. Chen, P. Khatri, P. K. Mazur, M. Polin, Y. Zheng, D. Vaka, C. D. Hoang, J. Shrager, Y. Xu, S. Vicent, A. J. Butte, E. A. Sweet-Cordero, A meta-Analysis of lung cancer gene expression identifies PTK7 as a survival gene in lung adenocarcinoma, Cancer Research 74, 2892-2902 (2014).
20. F. Baribaud, X. K. Li, Markers and Methods for Assessing and Treating Ulcerative Colitis and Related Disorders Using a 20 Gene Panel (available at www(dot)faqs(dot)org/patents/app/20100069256).
21. T. F. Mueller, G. Einecke, J. Reeve, B. Sis, M. Mengel, G. S. Jhangri, S. Bunnag, J. Cruz, D. Wishart, C. Meng, G. Broderick, B. Kaplan, P. F. Halloran, Microarray analysis of rejection in human kidney transplants using pathogenesis-based transcript sets, American Journal of Transplantation 7, 2712-2722 (2007).
22. B. Halloran, J. Chang, D. Q. Shih, D. McGovern, K. Famulski, C. Evaschesen, R. N. Fedorak, A. Thiesen, S. Targan, P. F. Halloran, Molecular patterns in human ulcerative colitis and correlation with response to infliximab., Inflammatory bowel diseases 20, 2353-63 (2014).
23. S. Hänzelmann, R. Castelo, J. Guinney, GSVA: gene set variation analysis for microarray and RNA-seq data., BMC bioinformatics 14, 7 (2013).
24. T. E. Sweeney, A. Shidham, H. R. Wong, P. Khatri, A comprehensive time-course - based multicohort analysis of sepsis and sterile inflammation reveals a robust diagnostic gene set, Science Translational Medicine 7, 1-16 (2015).
25. T. Barrett, D. B. Troup, S. E. Wilhite, P. Ledoux, C. Evangelista, I. F. Kim, M. Tomashevsky, K. a Marshall, K. H. Phillippy, P. M. Sherman, R. N. Muertter, M. Holko, O. Ayanbule, A. Yefanov, A. Soboleva, NCBI GEO: archive for functional genomics data sets-10 years on., Nucleic acids research 39, D1005-10 (2011).
26. A. R. Abbas, K. Wolslegel, D. Seshasayee, Z. Modrusan, H. F. Clark, Deconvolution of blood microarray data identifies cellular activation patterns in systemic lupus erythematosus., PloS one 4, e6098 (2009).
27. P. Karpiński, D. Frydecka, M. M. Sasiadek, B. Misiak, Reduced number of peripheral natural killer cells in schizophrenia but not in bipolar disorder, Brain, Behavior, and Immunity (2016), doi:10.1016/j .bbi.2016.02.005.
28. A. Lügering, M. Schmidt, N. Lügering, H. G. Pauels, W. Domschke, T. Kucharzik, Infliximab induces apoptosis in monocytes from patients with chronic active Crohn's disease by using a caspase-dependent pathway., Gastroenterology 121, 1145-57 (2001).
29. a Bitton, M. a Peppercorn, D. a Antonioli, J. L. Niles, S. Shah, a Bousvaros, B. Ransil, G. Wild, a Cohen, M. D. Edwardes, a C. Stevens, Clinical, biological, and histologic parameters as predictors of relapse in ulcerative colitis., Gastroenterology 120, 13-20 (2001).
30. V. Villanacci, E. Antonelli, G. Reboldi, M. Salemme, G. Casella, G. Bassotti, Endoscopic biopsy samples of naive "colitides" patients: role of basal plasmacytosis., Journal of Crohn's & colitis 8, 1438-43 (2014).
31. F. Schnitzler, H. Fidder, M. Ferrante, M. Noman, I. Arijs, G. Van Assche, I. Hoffman, K. Van Steen, S. Vermeire, P. Rutgeerts, Long-term outcome of treatment with infliximab in 614 patients with Crohn's disease: results from a single-centre cohort., Gut 58, 492-500 (2009).
32. D. Reijasse, C. Le Pendeven, J. Cosnes, A. Dehee, J.-P. Gendre, J.-C. Nicolas, L. Beaugerie, Epstein-Barr virus viral load in Crohn's disease: effect of immunosuppressive therapy., Inflammatory bowel diseases 10, 85-90 (2004).
33. R. S. Wallis, M. S. Broder, J. Y. Wong, M. E. Hanson, D. O. Beenhouwer, Granulomatous Infectious Diseases Associated with Tumor Necrosis Factor Antagonists, Clinical Infectious Diseases 38, 1261-1265 (2004).
34. A. R. Abbas, D. Baldwin, Y. Ma, W. Ouyang, A. Gurney, F. Martin, S. Fong, M. van Lookeren Campagne, P. Godowski, P. M. Williams, a C. Chan, H. F. Clark, Immune response in silico (IRIS): immune-specific genes identified from a compendium of microarray expression data., Genes and immunity 6, 319-31 (2005).
35. R. Gaujoux, C. Seoighe, CellMix: A Comprehensive Toolbox for Gene Expression Deconvolution., Bioinformatics (Oxford, England), 1-2 (2013).
36. F. O. Martinez, S. Gordon, M. Locati, A. Mantovani, Transcriptional profiling of the human monocyte-to-macrophage differentiation and polarization: new molecules and patterns of gene expression., Journal of immunology (Baltimore, Md. : 1950) 177, 7303-11 (2006).
37. N. W. Palm, M. R. De Zoete, T. W. Cullen, N. A. Barry, J. Stefanowski, L. Hao, P. H. Degnan, J. Hu, I. Peter, W. Zhang, E. Ruggiero, J. H. Cho, A. L. Goodman, R. A. Flavell, Immunoglobulin A coating identifies colitogenic bacteria in inflammatory bowel disease, Cell 158, 1000-1010 (2014).
38. J. A. Rump, J. Schölmerich, V. Gross, M. Roth, R. Helfesrieder, A. Rautmann, J. Lüdemann, W. L. Gross, H. H. Peter, A new type of perinuclear anti-neutrophil cytoplasmic antibody (p-ANCA) in active ulcerative colitis but not in Crohn's disease., Immunobiology 181, 406-13 (1990).
39. M. Ferrante, S. Vermeire, K. H. Katsanos, M. Noman, G. Van Assche, F. Schnitzler, I. Arijs, G. De Hertogh, I. Hoffman, J. K. Geboes, P. Rutgeerts, Predictors of early response to infliximab in patients with ulcerative colitis., Inflammatory bowel diseases 13, 123-8 (2007).
40. M. Jürgens, R. P. Laubender, F. Hartl, M. Weidinger, J. Seiderer, J. Wagner, M. Wetzke, F. Beigel, S. Pfennig, J. Stallhofer, F. Schnitzler, C. Tillack, P. Lohse, B. Göke, J. Glas, T. Ochsenkiihn, S. Brand, Disease activity, ANCA, and IL23R genotype status determine early response to infliximab in patients with ulcerative colitis., The American journal of gastroenterology 105, 1811-9 (2010).
41. S. E. Plevy, C. J. Landers, J. Prehn, N. M. Carramanzana, R. L. Deem, D. Shealy, S. R. Targan, A role for TNF-alpha and mucosal T helper-1 cytokines in the pathogenesis of Crohn's disease., Journal of immunology (Baltimore, Md. : 1950) 159, 6276-82 (1997).
42. F. Hiepe, T. Dörner, A. E. Hauser, B. F. Hoyer, H. Mei, A. Radbruch, Long-lived autoreactive plasma cells drive persistent autoimmune inflammation., Nature reviews. Rheumatology 7, 170-8 (2011).
43. D. P. B. McGovern, A. Gardet, L. Törkvist, P. Goyette, J. Essers, K. D. Taylor, B. M. Neale, R. T. H. Ong, C. Lagacé, C. Li, T. Green, C. R. Stevens, C. Beauchamp, P. R. Fleshner, M. Carlson, M. D'Amato, J. Halfvarson, M. L. Hibberd, M. Lördal, L. Padyukov, A. Andriulli, E. Colombo, A. Latiano, O. Palmieri, E.-J. Bernard, C. Deslandres, D. W. Hommes, D. J. de Jong, P. C. Stokkers, R. K. Weersma, Y. Sharma, M. S. Silverberg, J. H. Cho, J. Wu, K. Roeder, S. R. Brant, L. P. Schumm, R. H. Duerr, M. C. Dubinsky, N. L. Glazer, T. Haritunians, A. Ippoliti, G. Y. Melmed, D. S. Siscovick, E. A. Vasiliauskas, S. R. Targan, V. Annese, C. Wijmenga, S. Pettersson, J. I. Rotter, R. J. Xavier, M. J. Daly, J. D. Rioux, M. Seielstad, Genome-wide association identifies multiple ulcerative colitis susceptibility loci., Nature genetics 42, 332-7 (2010).
44. K. Asano, T. Matsushita, J. Umeno, N. Hosono, A. Takahashi, T. Kawaguchi, T. Matsumoto, T. Matsui, Y. Kakuta, Y. Kinouchi, T. Shimosegawa, M. Hosokawa, Y. Arimura, Y. Shinomura, Y. Kiyohara, T. Tsunoda, N. Kamatani, M. Iida, Y. Nakamura, M. Kubo, A genome-wide association study identifies three new susceptibility loci for ulcerative colitis in the Japanese population., Nature genetics 41, 1325-9 (2009).
45. P. Minar, Y. Haberman, I. Jurickova, T. Wen, M. E. Rothenberg, M.-O. Kim, S. A. Saeed, R. N. Baldassano, M. Stephens, J. Markowitz, J. Rosh, W. V. Crandall, M. B. Heyman, D. R. Mack, A. M. Griffiths, S. S. Baker, J. S. Hyams, S. Kugathasan, L. A. Denson, Utility of neutrophil Fcγ receptor I (CD64) index as a biomarker for mucosal inflammation in pediatric Crohn's disease., Inflammatory bowel diseases 20, 1037-48 (2014).
46. M. Uo, T. Hisamatsu, J. Miyoshi, D. Kaito, K. Yoneno, M. T. Kitazume, M. Mori, A. Sugita, K. Koganei, K. Matsuoka, T. Kanai, T. Hibi, Mucosal CXCR4+ IgG plasma cells contribute to the pathogenesis of human ulcerative colitis through FcYR-mediated CD14 macrophage activation., Gut 62, 1734-44 (2013).

## Claims

1. A method of predicting responsiveness of a subject having an inflammatory bowel disease (IBD) to a tumor necrosis factor (TNF)-alpha inhibitor, comprising:
analyzing a frequency of at least one subpopulation of immune cells in a colon and/or ileum tissue biopsy of the subject,
wherein a frequency above a predetermined threshold of immune cells of a subpopulation plasma cells is indicative of the subject being non-responder to the TNF-alpha inhibitor, wherein said plasma cells are **characterized by** positive expression of CD138, and
wherein said predetermined threshold is determined by the frequency of the subpopulation of said immune cells in the same tissue biopsy of a responder subject to said TNF-alpha inhibitor treatment,
thereby predicting the responsiveness of the subject having the inflammatory bowel disease (IBD) to the TNF-alpha inhibitor.

2. A method of selecting treatment to inflammatory bowel disease (IBD) in a subject in need thereof, the method comprising:
(a) determining responsiveness to a TNF-alpha inhibitor according to the method of claim 1; and
(b) selecting treatment based on said responsiveness.

3. The method of claim 1 or 2, wherein said tissue biopsy of the subject comprises an inflamed tissue.

4. The method of any one of claims 1-3, wherein said subject is a naive subject who hasn't been treated with said TNF-alpha inhibitor.

5. The method of any one of claims 1-4, wherein said analyzing said frequency of said at least one subpopulation of immune cells is performed by a morphometric analysis and/or using at least one histological stain.

6. The method of any one of claims 1-4, wherein said analyzing said frequency of said at least one subpopulation of immune cells is performed using at least one antibody.

7. The method of any one of claims 1-4, wherein said analyzing said frequency of said at least one subpopulation of immune cells is performed by a method selected from the group consisting of: an RNA in-situ hybridization assay, a single cell RNA sequencing (RNA SEQ) analysis, exome sequencing, reverse-transcriptase polymerase chain reaction (RT-PCR) followed by deconvolution, and micro array followed by deconvolution.

8. The method of any one of claims 1-7, wherein said tissue biopsy is a colon tissue biopsy.

## Patentansprüche

1. Verfahren zur Vorhersage der Reaktionsfähigkeit eines Patienten mit einer entzündlichen Darmerkrankung (IBD) auf einen Tumornekrosefaktor (TNF)-alpha-Inhibitor, umfassend:
Analysieren der Häufigkeit mindestens einer Subpopulation von Immunzellen in einer Gewebebiopsie des Kolons und/oder des Ileums des Patienten,
wobei eine Häufigkeit oberhalb eines vorgegebenen Schwellenwerts von Immunzellen einer Subpopulation von Plasmazellen darauf schließen lässt, dass der Patient ein Non-Responder auf den TNF-alpha-Inhibitor ist, wobei diese Plasmazellen **dadurch gekennzeichnet sind, dass** sie eine positive Expression von CD138 aufweisen, und
wobei dieser vorbestimmte Schwellenwert durch die Häufigkeit der Subpopulation dieser Immunzellen in derselben Gewebebiopsie eines Responders bestimmt wird, der einer Behandlung mit dem TNF-alpha-Inhibitor unterzogen wurde,
wodurch die Reaktionsfähigkeit des Patienten mit der entzündlichen Darmerkrankung (IBD) auf den TNF-alpha-Inhibitor vorhergesagt wird.

2. Verfahren zur Auswahl einer Behandlung für eine entzündliche Darmerkrankung (IBD) bei einem Patienten, der dieser bedarf, wobei das Verfahren umfasst:
(a) Bestimmen der Reaktionsfähigkeit auf einen TNF-alpha-Inhibitor nach dem Verfahren von Anspruch 1; und
(b) Auswählen einer Behandlung basierend auf dieser Reaktionsfähigkeit.

3. Verfahren nach Anspruch 1 oder 2, wobei diese Gewebebiopsie des Patienten ein entzündetes Gewebe umfasst.

4. Verfahren nach einem der Ansprüche 1 bis 3, wobei der Patient ein naiver Patient ist, der noch nicht mit diesem TNF-alpha-Inhibitor behandelt wurde.

5. Verfahren nach einem der Ansprüche 1 bis 4, wobei dieses Analysieren dieser Häufigkeit dieser mindestens einen Subpopulation von Immunzellen durch eine morphometrische Analyse und/oder unter Verwendung mindestens einer histologischen Färbung durchgeführt wird.

6. Verfahren nach einem der Ansprüche 1 bis 4, wobei dieses Analysieren dieser Häufigkeit dieser mindestens einen Subpopulation von Immunzellen unter Verwendung mindestens eines Antikörpers durchgeführt wird.

7. Verfahren nach einem der Ansprüche 1 bis 4, wobei dieses Analysieren dieser Häufigkeit dieser mindestens einen Subpopulation von Immunzellen durch ein Verfahren durchgeführt wird, das ausgewählt ist aus der Gruppe bestehend aus: einem RNA-In-situ-Hybridisierungsassay, einer Einzelzell-RNA-Sequenzierungsanalyse (RNA-SEQ), Exomsequenzierung, reverser Transkriptase-Polymerase-Kettenreaktion (RT-PCR) gefolgt von Dekonvolution und Microarray gefolgt von Dekonvolution.

8. Verfahren nach einem der Ansprüche 1 bis 7, wobei es sich bei dieser Gewebebiopsie um eine Kolongewebebiopsie handelt.

## Revendications

1. Procédé de prédiction de la réactivité chez un sujet ayant une maladie inflammatoire de l'intestin (IBD) à un inhibiteur du facteur de nécrose tumorale (TNF)-alpha, comprenant :
l'analyse d'une fréquence d'au moins une sous-population de cellules immunitaires dans un colon et/ou une biopsie de tissu de l'iléon du sujet,
dans lequel une fréquence au-dessus d'un seuil prédéterminé de cellules immunitaires d'une sous-population de plasmocytes est indicative du fait que le sujet est non répondant à l'inhibiteur du TNF-alpha, dans lequel lesdits plasmocytes sont **caractérisés par** une expression positive de CD138, et
dans lequel ledit seuil prédéterminé est déterminé par la fréquence de la sous-population desdites cellules immunitaires dans la même biopsie de tissu d'un sujet répondant audit traitement d'inhibiteur du TNF-alpha,
prédisant de ce fait la réactivité du sujet ayant la maladie inflammatoire de l'intestin (IBD) à l'inhibiteur du TNF-alpha.

2. Procédé de sélection d'un traitement pour une maladie inflammatoire de l'intestin (IBD) chez un sujet en ayant besoin, le procédé comprenant :
(a) la détermination d'une réactivité à un inhibiteur du TNF-alpha selon le procédé de la revendication 1 ; et
(b) la sélection d'un traitement sur la base de ladite réactivité.

3. Procédé selon la revendication 1 ou 2, dans lequel ladite biopsie de tissu du sujet comprend un tissu enflammé.

4. Procédé selon l'une quelconque des revendications 1 à 3, dans lequel ledit sujet est un sujet vierge qui n'a pas été traité avec ledit inhibiteur du TNF-alpha.

5. Procédé selon l'une quelconque des revendications 1 à 4, dans lequel ladite analyse de ladite fréquence de ladite au moins une sous-population de cellules immunitaires est réalisée par une analyse morphométrique et/ou en utilisant au moins une coloration histologique.

6. Procédé selon l'une quelconque des revendications 1 à 4, dans lequel ladite analyse de ladite fréquence de ladite au moins une sous-population de cellules immunitaires est réalisée en utilisant au moins un anticorps.

7. Procédé selon l'une quelconque des revendications 1 à 4, dans lequel ladite analyse de ladite fréquence de ladite au moins une sous-population de cellules immunitaires est réalisée par un procédé choisi dans le groupe constitué par : un essai d'hybridation in-situ d'ARN, une analyse de séquençage d'ARN de cellule unique (RNA SEQ), un séquençage de l'exome, une transcription inverse couplée à une réaction d'amplification en chaîne par polymérase (RT-PCR) suivie d'une déconvolution, un microréseau suivi d'une déconvolution.

8. Procédé selon l'une quelconque des revendications 1 à 7, dans lequel ladite biopsie de tissu est une biopsie de tissu du colon.
